(19) **Europäisches Patentamt / European Patent Office / Office européen des brevets**

(11) **EP 4 129 471 A1**

(12) **EUROPEAN PATENT APPLICATION**
published in accordance with Art. 153(4) EPC

(43) Date of publication:
08.02.2023 Bulletin 2023/06

(21) Application number: 21782118.0

(22) Date of filing: 31.03.2021

(51) International Patent Classification (IPC):
*B01J 23/78* (1974.07)  *B01J 21/10* (1974.07)
*B01J 23/02* (1974.07)  *B01J 23/04* (1974.07)
*B01J 23/06* (1974.07)  *B01J 23/10* (1974.07)
*B01J 29/70* (1995.01)  *C07C 45/29* (1980.01)
*C07C 47/02* (1968.09)  *C07C 47/22* (1968.09)
*C07B 61/00* (1985.01)

(52) Cooperative Patent Classification (CPC):
**B01J 21/10; B01J 23/02; B01J 23/04; B01J 23/06;**
**B01J 23/10; B01J 23/78; B01J 29/70; C07C 45/29;**
**C07C 47/02; C07C 47/22;** C07B 61/00

(86) International application number:
**PCT/JP2021/013989**

(87) International publication number:
**WO 2021/201155 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 31.03.2020  JP 2020065205
08.09.2020  JP 2020150757

(71) Applicants:
• **Mitsubishi Chemical Corporation**
**Chiyoda-ku**
**Tokyo 100-8251 (JP)**
• **National University Corporation**
**Hokkaido University**
**Sapporo-shi, Hokkaido 060-0808 (JP)**

(72) Inventors:
• **SUZUKI, Tatsuya**
**Tokyo 100-8251 (JP)**
• **KATOU, Yuuki**
**Tokyo 100-8251 (JP)**
• **NINOMIYA, Wataru**
**Tokyo 100-8251 (JP)**
• **MASUDA, Takao**
**Sapporo-shi, Hokkaido 060-0808 (JP)**
• **NAKASAKA, Yuta**
**Sapporo-shi, Hokkaido 060-0808 (JP)**
• **YOSHIKAWA, Takuya**
**Sapporo-shi, Hokkaido 060-0808 (JP)**
• **YAMASHITA, Shohei**
**Sapporo-shi, Hokkaido 060-0808 (JP)**

(74) Representative: **Hoffmann Eitle**
**Patent- und Rechtsanwälte PartmbB**
**Arabellastraße 30**
**81925 München (DE)**

(54) **CATALYST, METHOD FOR PRODUCING ISOBUTYL ALDEHYDE AND METHACROLEIN, METHOD FOR PRODUCING METHACRYLIC ACID, AND METHOD FOR PRODUCING METHACRYLIC ACID ESTER**

(57) There is provided a catalyst having an average electronegativity of 2.1 or more and 2.8 or less.

EP 4 129 471 A1

Processed by Luminess, 75001 PARIS (FR)

## Description

[Technical Field]

The present invention relates to a catalyst, a method for producing isobutyl aldehyde and methacrolein, a method for producing methacrylic acid, and a method for producing a methacrylic acid ester.

Priority is claimed on Japanese Patent Application No. 2020-65205 filed in Japan on March 31, 2020, and Japanese Patent Application No. 2020-150757 filed in Japan on September 8, 2020, the contents of which are incorporated herein by reference.

[Background Art]

Isobutyl aldehyde can be induced to methacrolein and methacrylic acid by an oxidation reaction using a heteropoly acid and is a useful intermediate of a methacrylic acid ester (Non-Patent Documents 1 and 2).

An industrial method for producing isobutyl aldehyde is a hydroformylation reaction between propylene and carbon monoxide. However, according to this method, isobutyl aldehyde is produced as a by-product at the time of producing n-butyraldehyde, and thus isobutyl aldehyde cannot be selectively produced in any amount.

As a method for producing isobutyl aldehyde, there is also known a method of carrying out conversion to isobutyl aldehyde by a dehydrogenation reaction of isobutanol (hereinafter, also referred to as "isobutanol"). Non-Patent Document 3 describes that isobutyl aldehyde can be synthesized by a dehydrogenation reaction of isobutanol using Zn/bentonite as a catalyst. In addition, isobutanol as a raw material can be produced from biomass (Patent Document 1), and the synthesis of a methacrylic acid ester from a recyclable raw material can be expected.

Further, Patent Documents 2 and 3 each propose that a copper catalyst and a copper magnesia catalyst are used as dehydrogenation catalysts. However, these catalysts had to be subjected to a reduction treatment with hydrogen before the start of the reaction. In addition, it is known that hydrogen has a wide explosive range (4% to 75% by volume) and embrittles a metal such as stainless steel. As a result, not only the handling of hydrogen is difficult, but also an explosion-proof structure, a concentration control of a hydrogen-containing gas, a safety mechanism for preparing for abnormal situations, and the like are required.

[Citation List]

[Patent Documents]

[Patent Document 1]
Published Japanese Patent Publication No. 2011-505490 of the PCT International Publication
[Patent Document 2]
Japanese Unexamined Patent Application, First Publication No. 2010-104938
[Patent Document 3]
Japanese Unexamined Patent Application, First Publication No. 2016-79143

[Non Patent Documents]

[Non Patent Document 1]
Journal of Catalysis 195 (2000) 360-375
[Non Patent Document 2]
Journal of Molecular Catalysis A: Chemical 152 (2000) 141-155
[Non Patent Document 3]
Journal of the Serbian Chemical Society 73, 997-1009 (2008)

[Summary of Invention]

[Technical Problem]

However, there are few studies on the method of synthesizing isobutyl aldehyde by the dehydrogenation reaction of isobutanol, and the selectivity of isobutyl aldehyde has been not sufficient. In addition, complicated treatment such as the reduction treatment with hydrogen has been required.

An object of the present invention is to provide a catalyst for dehydration, by which a reduction treatment with hydrogen is not required, and isobutyl aldehyde and methacrolein can be produced from isobutanol with a high selectivity while suppressing the oxidation and dimerization of isobutyl aldehyde and to provide a method for producing a dehydrogenated compound using a catalyst for dehydration.

[Solution to Problem]

The present invention has the following configurations.

[1] A catalyst having an average electronegativity of 2.1 or more and 2.8 or less.

[2] The catalyst according to [1], in which the catalyst contains a metal oxide having an average electronegativity of 2.1 or more and 2.8 or less.

[3] The catalyst according to [2], in which the metal oxide includes an oxide of one or more metals selected from the group consisting of Al, Si, Zn, Ag, Ce, La, and Mg.

[4] The catalyst according to [2], in which the metal oxide includes an oxide of one or more metals selected from the group consisting of Zn, Al, Fe, Si, Zr, and Ti.

[5] The catalyst according to any one of [1] to [4], in which the catalyst contains one or more alkali metals.

[6] The catalyst according to any one of [1] to [5], in which an acid amount with respect to 1 g of the catalyst is 0.3 mmol/g or less.

[7] The catalyst according to any one of [1] to [6], in which the catalyst is a catalyst that is used for producing, from isobutanol, at least one dehydrogenated compound selected from the group consisting of isobutyl aldehyde and methacrolein.

[8] The catalyst according to any one of [1] to [7], in which the average electronegativity of the catalyst is preferably 2.10 or more and 2.75 or less, more preferably 2.10 or more and 2.70 or less, still more preferably 2.10 or more and 2.65 or less, even still more preferably 2.10 or more and 2.60 or less, particularly preferably 2.10 or more and 2.55 or less, and most preferably 2.10 or more and 2.50 or less.

[9] The catalyst according to any one of [1] to [8], in which the catalyst contains a metal oxide, and a content of the metal oxide is preferably 80% by mass or more and 100% by mass or less, more preferably 85% by mass or more and 100% by mass or less still more preferably 90% by mass or more and 100% by mass or less, even still more preferably 95% by mass or more and 100% by mass or less, particularly preferably 97.5% by mass or more and 100% by mass or less, and most preferably 99% by mass or more and 100% by mass or less, with respect to a total mass of the catalyst.

[10] The catalyst according to any one of [1] to [9], in which the catalyst contains a metal oxide and an alkali metal, and a content of the alkali metal is preferably 1.0% by mole or more and 9.0% by mole or less, more preferably 2.0% by mole or more and 8.5% by mole or less, still more preferably 3.0% by mole or more and 8.0% by mole or less, particularly preferably 4.0% by mole or more and 7.5% by mole or less, and most preferably 5.0% by mole or more and 7.0% by mole or less, with respect to a total mass of metals in the catalyst.

[11] The catalyst according to any one of [1] to [10], in which an acid amount in the catalyst is preferably 0 mmol/g or more and 0.25 mmol/g or less, more preferably 0 mmol/g or more and 0.20 mmol/g or less, still more preferably 0 mmol/g or more and 0.15 mmol/g or less, particularly preferably 0 mmol/g or more and 0.10 mmol/g or less, and most preferably 0 mmol/g or more and 0.08 mmol/g or less.

[12] The catalyst according to any one of [1] to [11], in which a base amount in the catalyst is preferably 0 mmol/g or more and 4.0 mmol/g or less, more preferably 0.001 mmol/g or more and 3.0 mmol/g or less, still more preferably 0.01 mmol/g or more and 2.0 mmol/g or less, particularly preferably 0.1 mmol/g or more and 1.0 mmol/g or less, and most preferably 0.15 mmol/g or more and 0.5 mmol/g or less.

[13] The catalyst according to any one of [1] to [12], in which a BET specific surface area of the catalyst is preferably 3 $m^2$/g or more and 230 $m^2$/g or less, more preferably 5 $m^2$/g or more and 200 $m^2$/g or less, still more preferably 10 $m^2$/g or more and 180 $m^2$/g or less, and particularly preferably 30 $m^2$/g or more and 170 $m^2$/g or less.

[14] A method for producing the catalyst according to any one of [1] to [13], the method including:
Calcining a metal oxide.

[15] The method for producing a catalyst according to [14], in which a calcining temperature is preferably 200°C or higher and 1,000°C or lower, more preferably 250°C or higher and 950°C or lower, still more preferably 300°C or higher and 900°C or lower, even still more preferably 350°C or higher and 850°C or lower, particularly preferably 400°C or higher and 800°C or lower, and most preferably 450°C or higher and 750°C or lower.

[16] A method for producing a dehydrogenated compound, which is a method for producing a dehydrogenated compound from isobutanol, the method including:

producing, from isobutanol, at least one dehydrogenated compound selected from the group consisting of isobutyl aldehyde and methacrolein by using the catalyst according to any one of [1] to [13].

[17] The method for producing a dehydrogenated compound according to [16], in which the producing of the dehydrogenated compound is carried out in a liquid phase or a gas phase.

[18] The method for producing a dehydrogenated compound according to [16] or [17], in which the producing the dehydrogenated compound is carried out in a gas phase and includes vaporizing a raw material gas containing isobutanol at a temperature of 108°C or higher and 600°C or lower and a pressure of 0.05 MPa or higher and 1 MPa or lower.

[19] The method for producing a dehydrogenated compound according to [17], in which an isobutanol concentration in the raw material gas is preferably 0.1% by volume or more and 100% by volume or less, more preferably 1% by volume or more and 50% by volume or less, and still more preferably 10% by volume or more and 30% by volume or less is, with respect to a total volume of the raw material gas.

[20] The method for producing a dehydrogenated compound according to [18] or [19], in which the raw material gas contains water vapor, and a concentration of the water vapor in the raw material gas is preferably 2.5% by volume or more and 80.0% by volume or less, more preferably 5.0% by volume or more and 75.0% by volume or less, still more preferably 10.0% by volume or more and 70.0% by volume or less, particularly preferably 15.0% by volume or more and 65.0% by volume or less and most preferably 20.0% by volume or more and 60.0% by volume or less, with respect to a total volume of the raw material gas.

[21] The method for producing a dehydrogenated compound according to any one of [16] to [20], in which a reaction temperature in the producing the dehydrogenated compound is preferably 320°C or higher and 600°C or lower, more preferably 340°C or higher and 580°C or lower, still more preferably 360°C or higher and 560°C or lower, and particularly preferably 380°C or higher and 540°C or lower.

[22] The method for producing a dehydrogenated compound according to any one of [16] to [21], in which W/F, which is calculated by "feeding amount of isobutanol supplied to a reactor (unit: g/h)/mass of catalyst with which reaction tube is filled" (unit: g), is preferably 0.30 or more and 8.0 or less, more preferably 0.50 or more and 4.5 or less, still more preferably 0.6 or more and 4.5 or less, particularly preferably 0.65 or more and 2.0 or less, and most preferably 0.68 or more and 1.0 or less.

[23] A method for producing methacrylic acid, the method including:
producing methacrylic acid from at least one selected from the group consisting of the isobutyl aldehyde and methacrolein obtained according to the method for producing a dehydrogenated compound according to [16] to [22].

[24] The method for producing methacrylic acid according to [23], in which the producing methacrylic acid is carried out in a gas phase.

[25] A method for producing a methacrylic acid ester, the method including:
producing methacrylic acid according to the method for producing methacrylic acid according to [23] and producing a methacrylic acid ester from the obtained methacrylic acid and an alcohol having 1 to 20 carbon atoms.

[26] The method for producing a methacrylic acid ester according to [25], in which the alcohol having 1 to 20 carbon atoms is at least one selected from the group consisting of an aliphatic alcohol and an aromatic alcohol.

[27] The method for producing a methacrylic acid ester according to [26], in which the alcohol having 1 to 20 carbon atoms is an aliphatic alcohol, and the aliphatic alcohol is at least one selected from methanol, ethanol, propanol, butanol, pentanol, hexanol, heptanol, octanol, nonanol, decanol, and eicosanol.

[28] The method for producing a methacrylic acid ester according to any one of [25] to [27], in which the producing the methacrylic acid ester is carried out in a presence of a catalyst, and the catalyst is sulfuric acid or an ion exchange resin.

[29] The method for producing a methacrylic acid ester according to [28], in which the catalyst is an ion exchange resin, and a flow-through amount of the methacrylic acid and the alcohol having 1 to 20 carbon atoms is preferably 0.10 times or more and 10.0 times or less and more preferably 0.20 times or more and 5.0 times or less in terms of mass ratio with respect to an amount of the ion exchange resin.

[30] The method for producing a methacrylic acid ester according to any one of [25] to [29], in which a reaction temperature in the producing the methacrylic acid ester is 40°C or higher and 130°C or lower.

[Advantageous Effects of Invention]

According to the present invention, it is possible to provide a catalyst for dehydration, by which a reduction treatment with hydrogen is not required, and isobutyl aldehyde and methacrolein can be produced from isobutanol with a high selectivity while suppressing the oxidation and dimerization of isobutyl aldehyde and to provide a method for producing a dehydrogenated compound using a catalyst for dehydration.

[Brief Description of Drawings]

Fig. 1 is a graph showing isobutanol (iBuOH) conversion rates and selectivities and yields of isobutyl aldehyde (iBAld) and methacrolein (MAL) in Examples 1 to 3.

Fig. 2 is a graph showing isobutanol (iBuOH) conversion rates and selectivities and yields of isobutyl aldehyde (iBAld) and methacrolein (MAL) in Examples 5 to 7.

Fig. 3 is a graph showing isobutanol (iBuOH) conversion rates and selectivities and yields of isobutyl aldehyde (iBAld) and methacrolein (MAL) in Examples 8 to 10.

Fig. 4 is a graph showing isobutanol (iBuOH) conversion rates and selectivities and yields of isobutyl aldehyde (iBAld) and methacrolein (MAL) in Examples 11 to 13.

Fig. 5 is a graph showing isobutanol (iBuOH) conversion rates and selectivities and yields of isobutyl aldehyde (iBAld) and methacrolein (MAL) in Examples 14 to 16.

Fig. 6 is a graph showing isobutanol (iBuOH) conversion rates and selectivities and yields of isobutyl aldehyde (iBAld) and methacrolein (MAL) in Examples 8 and 17 to 19.

Fig. 7 is a graph showing isobutanol (iBuOH) conversion rates and selectivities and yields of isobutyl aldehyde (iBAld) and methacrolein (MAL) in Examples 20 to 24.

Fig. 8A is a graph showing isobutanol (iBuOH) conversion rates and selectivities and yields of isobutyl aldehyde (iBAld) and methacrolein (MAL) in Examples 25 to 31 and Comparative Example 1.

Fig. 8B is a graph showing selectivities and average electronegativities of isobutyl aldehyde (iBAld) and methacrolein (MAL) in Examples 25 to 31 and Comparative Example 1.

Fig. 8C is a graph showing isobutanol (iBuOH) conversion rates and selectivities of isobutyl aldehyde (iBAld) and methacrolein (MAL) in Examples 25 to 31 and Comparative Example 1.

Fig. 9 is a graph showing isobutanol (iBuOH) conversion rates and selectivities and yields of isobutyl aldehyde (iBAld) and methacrolein (MAL) in Examples 33 to 36.

Fig. 10 is a graph showing isobutanol (iBuOH) conversion rates and selectivities and yields of isobutyl aldehyde (iBAld) and methacrolein (MAL) in Examples 37 to 40.

Fig. 11 is a graph showing isobutanol (iBuOH) conversion rates and selectivities and yields of isobutyl aldehyde (iBAld) and methacrolein (MAL) in Examples 25, 41, and 42.

Fig. 12 is a graph showing isobutanol (iBuOH) conversion rates and selectivities and yields of isobutyl aldehyde (iBAld) and methacrolein (MAL) in Examples 1, 43, and 44.

Fig. 13 is a graph showing isobutanol (iBuOH) conversion rates and selectivities and yields of isobutyl aldehyde (iBAld) and methacrolein (MAL) in Examples 3, 13, and 45.

Fig. 14 is a graph showing isobutanol (iBuOH) conversion rates and selectivities and yields of isobutyl aldehyde (iBAld) and methacrolein (MAL) in Examples 46 to 50.

Fig. 15 is a graph showing isobutanol (iBuOH) conversion rates and selectivities and yields of isobutyl aldehyde (iBAld) and methacrolein (MAL) in Examples 51 to 53 and 28.

Fig. 16 is a graph showing isobutanol (iBuOH) conversion rates and selectivities and yields of isobutyl aldehyde (iBAld) and methacrolein (MAL) in Examples 5, 54 to 56, and 8.

Fig. 17 is a graph showing isobutanol (iBuOH) conversion rates and selectivities and yields of isobutyl aldehyde (iBAld) and methacrolein (MAL) in Examples 57 and 2.

Fig. 18 is a graph showing isobutanol (iBuOH) conversion rates and selectivities and yields of isobutyl aldehyde (iBAld) and methacrolein (MAL) in Examples 58, 22, and 59.

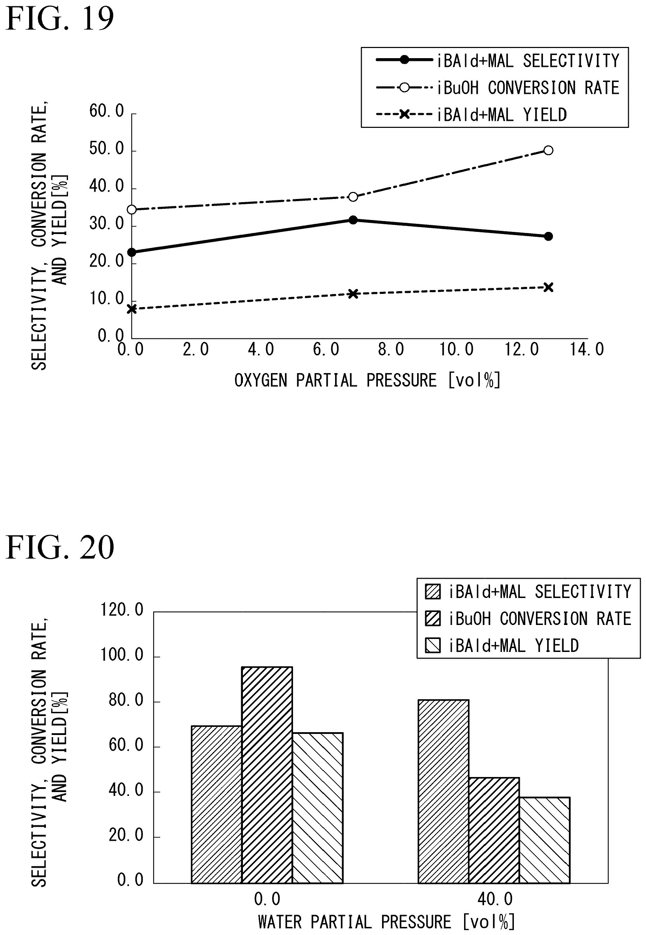

Fig. 19 is a graph showing isobutanol (iBuOH) conversion rates and selectivities and yields of isobutyl aldehyde (iBAld) and methacrolein (MAL) in Examples 61 to 63.

Fig. 20 is a graph showing isobutanol (iBuOH) conversion rates and selectivities and yields of isobutyl aldehyde (iBAld) and methacrolein (MAL) in Examples 53 and 64.

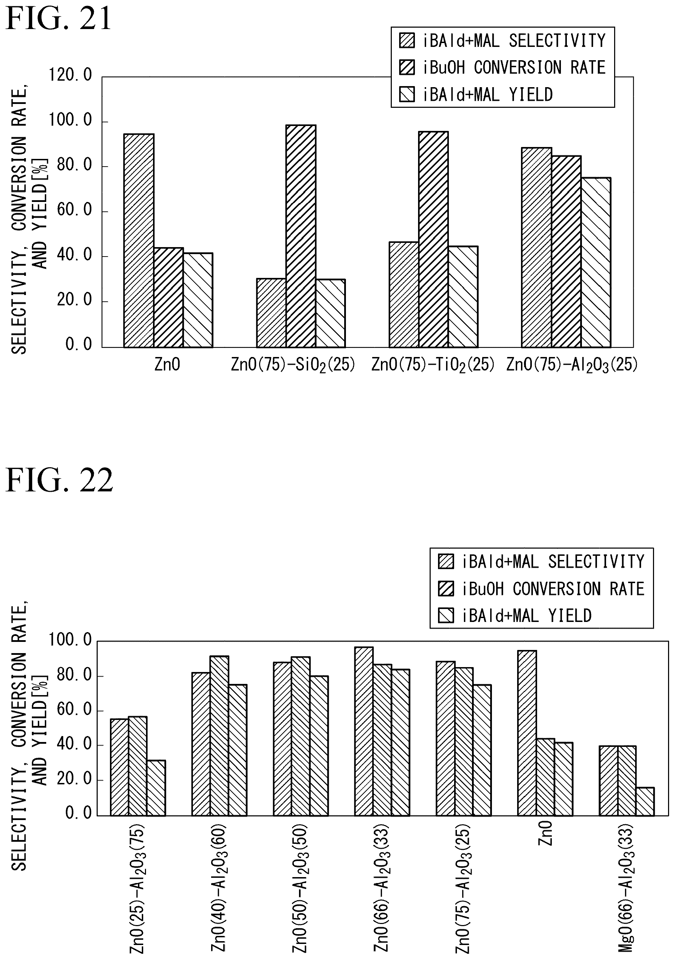

Fig. 21 is a graph showing isobutanol (iBuOH) conversion rates and selectivities and yields of isobutyl aldehyde (iBAld) and methacrolein (MAL) in Examples 52 and 65 to 67.

Fig. 22 is a graph showing isobutanol (iBuOH) conversion rates and selectivities and yields of isobutyl aldehyde (iBAld) and methacrolein (MAL) in Examples 67 to 71, 52, and 79.

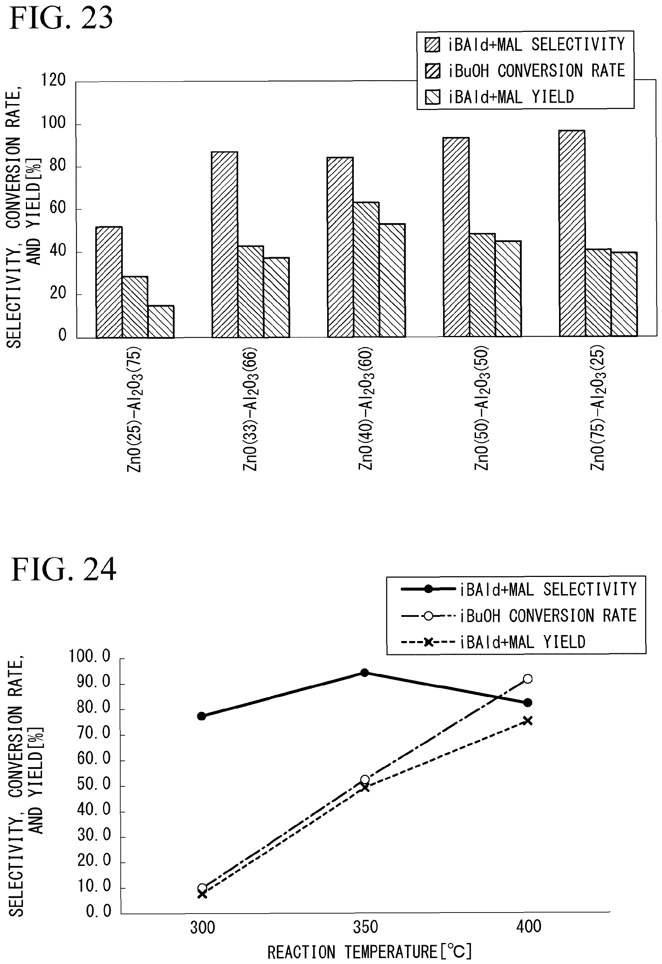

Fig. 23 is a graph showing isobutanol (iBuOH) conversion rates and selectivities and yields of isobutyl aldehyde (iBAld) and methacrolein (MAL) in Examples 72 to 76.

Fig. 24 is a graph showing isobutanol (iBuOH) conversion rates and selectivities and yields of isobutyl aldehyde (iBAld) and methacrolein (MAL) in Examples 77, 74, and 69.

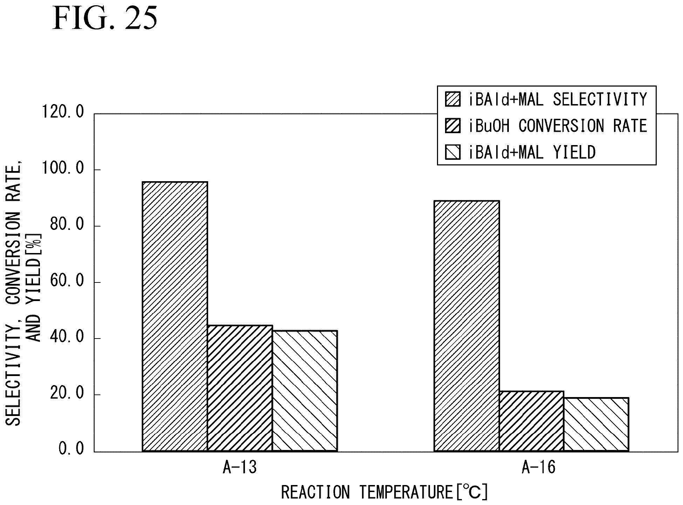

Fig. 25 is a graph showing isobutanol (iBuOH) conversion rates and selectivities and yields of isobutyl aldehyde (iBAld) and methacrolein (MAL) in Examples 75 and 78.

[Description of Embodiments]

[First embodiment]

(Catalyst)

A catalyst of the first embodiment (hereinafter, also referred to as a "catalyst (A)") is a catalyst that enables the production of isobutyl aldehyde (isobutyl aldehyde) and/or methacrolein by a dehydrogenation reaction of isobutanol (isobutanol), and It is a catalyst that does not require reduction with hydrogen.

The catalyst (A) has an average electronegativity of 2.10 or more and 2.80 or less. The average electronegativity of the catalyst is preferably 2.10 or more and 2.75 or less, more preferably 2.10 or more and 2.70 or less, still more preferably 2.10 or more and 2.65 or less, even still more preferably 2.10 or more and 2.60 or less, particularly preferably 2.10 or more and 2.55 or less, and most preferably 2.10 or more and 2.50 or less. When the average electronegativity is within the above range, it is possible to obtain a catalyst that suppresses the dehydration reaction of isobutanol and suppresses the dimerization of the generated isobutyl aldehyde as well, thereby efficiently producing isobutyl aldehyde. In the present invention, the average electronegativity of a compound $P_pQ_qR_r$ is defined according to the Sanderson's expression below.

Average electronegativity = $(S_P{}^p \cdot S_Q{}^q \cdot S_R{}^r)^{1/(p + q + r)}$
$S_P$: Pauling's electronegativity of element P
$S_Q$: Pauling's electronegativity of element Q
$S_R$: Pauling's electronegativity of element R
$p$: Number of elements of element P
$q$: Number of elements of element Q
$r$: Number of elements of element R

Regarding the constitutional element of the catalyst (A), the catalyst is preferably a catalyst containing one or more metal oxides selected from the group consisting of Al, Si, Zn, Ti, Ag, Ce, La, and Mg, more preferably a catalyst containing one or more metal oxides selected from the group consisting of Al, Zn, Ti, Ag, Ce, La, and Mg, still more preferably a catalyst containing one or more metal oxides selected from the group consisting of Al, Zn, Ti, Ag, Ce, and Mg, even still more preferably a catalyst containing one or more metal oxides selected from the group consisting of Al, Zn, Ag, Ce, and Mg, particularly preferably a catalyst containing one or more metal oxides selected from the group consisting of Al, Zn, Ag, and Mg, and most preferably a catalyst containing one or more metal oxides selected from the group consisting of Al, Zn, and Ag, from the viewpoint of increasing the isobutyl aldehyde selectivity. It is noted that one kind of the metal may be used, or two or more kinds thereof may be used.

Further, with respect to the sum of the masses of all the metal oxides contained in the catalyst, the content of the metal oxide contained in the catalyst is preferably 80% by mass or more, more preferably 85% by mass or more, still more preferably 90% by mass or more, even still more preferably 95% by mass or more, particularly preferably 97.5% by mass or more, and most preferably 99% by mass or more, from the viewpoint of improving the isobutyl aldehyde selectivity. In a case where the catalyst (A) is used, the reduction with hydrogen is not required, and isobutyl aldehyde and methacrolein can be produced from isobutanol with a high selectivity while suppressing the dehydration reaction and the oxidation and dimerization of isobutyl aldehyde.

The catalyst (A) is not particularly limited as long as it can contain one or more metal oxides selected from the group consisting of Al, Si, Zn, Ag, Ce, La, and Mg, and a known method can be used. For example, the catalyst (A) can be easily produced by a coprecipitation method, a deposition method, an impregnation method, a kneading method, or a method in which these are used in combination. The raw material for the catalyst (A) is not particularly limited, and examples thereof include a hydroxide, a nitrate, an acetate, and a sulfate.

The catalyst (A) may be calcined before use. From the viewpoint of improving the selectivities of isobutyl aldehyde and/or methacrolein, the calcining temperature is preferably 200°C or higher and 1,000°C or lower, more preferably 250°C or higher and 950°C or lower, still more preferably 300°C or higher and 900°C or lower, even still more preferably 350°C or higher and 850°C or lower, particularly preferably 400°C or higher and 800°C or lower, and most preferably 450°C or higher and 750°C or lower. The calcining is preferably carried out in an environment of air or oxygen from the viewpoint of removing sulfate ions, acetate ions, and the like in the catalyst. Air or oxygen may be diluted with an inert gas in order to suppress the heat generation due to the catalyst during calcining. Examples of the inert gas include helium, neon, krypton, xenon, and radon.

[Second embodiment]

(Catalyst)

A catalyst of a second embodiment (hereinafter, also referred to as a "catalyst (B)") is a catalyst for producing isobutyl aldehyde (isobutyl aldehyde) and/or methacrolein by a dehydrogenation reaction of isobutanol (isobutanol), and it is a catalyst that does not require reduction with hydrogen.

The catalyst (B) has an average electronegativity of 2.10 or more and 2.80 or less.

The catalyst (B) is a catalyst containing an oxide of one or more metals selected from the group consisting of Al, Si, Fe, Zr, and Ti, and one or more alkali metals. In a case where the catalyst (B) is used, the reduction with hydrogen is not required, and isobutyl aldehyde and methacrolein can be produced from isobutanol with a high selectivity while suppressing the dehydration reaction and the oxidation and dimerization of isobutyl aldehyde.

Examples of the alkali metal include Na, K, Rb, and Cs.

The lower limit of the content of the alkali metal in the catalyst (B) is preferably 1.0% by mole or more, more preferably 2.0% by mole or more, still more preferably 3.0% by mole or more, particularly preferably 4.0% by mole or more, and most preferably 5.0% by mole or more, with respect to the total amount of metal atoms in the catalyst. In a case where the content of the alkali metal is equal to or larger than the above-described lower limit value, the dehydration reaction can be suppressed. In addition, the upper limit of the content of the alkali metal in the catalyst (B) is preferably 9.0% by mole or less, more preferably 8.5% by mole or less, still more preferably 8.0% by mole or less, particularly preferably 7.5% by mole or less, and most preferably 7.0% by mole or less, with respect to the total amount of metal atoms in the catalyst. In a case where the content of the alkali metal is equal to or smaller than the above-described upper limit value, the oxidation and dimerization of isobutyl aldehyde can be suppressed.

When the catalyst contains a metal oxide and an alkali metal, the alkali metal is preferably supported on the metal oxide.

The mass ratio represented by [mass of alkali metal]/[mass of metal oxide] is preferably 1 to 10, more preferably 2 to 9, and still more preferably 2.3 to 8.

The acid amount in the catalyst (B) is preferably 0.25 mmol/g or less, more preferably 0.20 mmol/g or less, still more preferably 0.15 mmol/g or less, particularly preferably 0.10 mmol/g or less, and most preferably 0.08 mmol/g or less, from the viewpoint of improving the selectivities of isobutyl aldehyde and methacrolein. It is noted that the acid amount is measured according to temperature programed desorption (TPD) using $NH_3$ as a probe molecule.

In addition, the base amount in the catalyst (2) for dehydrogenation is preferably 4.0 mmol/g or less, more preferably 3.0 mmol/g or less, still more preferably 2.0 mmol/g or less, particularly preferably 1.0 mmol/g or less, and most preferably 0.5 mmol/g or less, from the viewpoint of improving the selectivities of isobutyl aldehyde and methacrolein. It is noted that the base amount is measured according to temperature programed desorption (TPD) using $CO_2$ as a probe molecule.

Examples of the method for reducing the acid amount of the catalyst (B) include a method of supporting an alkali metal on a metal oxide. In a case where the amount of alkali metal supported is increased, the acid amount tends to decrease.

In addition, examples of the method for reducing the base amount of the catalyst (B) include a method of reducing the amount of alkali metal supported when supporting an alkali metal on a metal oxide. In a case where the amount of alkali metal supported is reduced, the base amount tends to decrease. It is noted that one kind of alkali metal may be used, or two or more kinds thereof may be used.

The method for producing the catalyst (B) is not particularly limited as long as at least one or more metals selected from alkali metals can be contained in a metal oxide containing one or more metals selected from the group consisting of Al, Si, Fe, Zr, and Ti, and a known method can be used. For example, the catalyst (B) can be easily produced by a coprecipitation method, a deposition method, an impregnation method, a kneading method, or a method in which these are used in combination. The raw material for the catalyst (B) is not particularly limited, and examples thereof include a hydroxide, a nitrate, an acetate, and a sulfate.

The catalyst (B) may be calcined before use. From the viewpoint of improving the selectivities of isobutyl aldehyde and/or methacrolein, the calcining temperature is preferably 200°C or higher and 1,000°C or lower, more preferably 250°C or higher and 950°C or lower, still more preferably 300°C or higher and 900°C or lower, even still more preferably 350°C or higher and 850°C or lower, particularly preferably 400°C or higher and 800°C or lower, and most preferably 450°C or higher and 750°C or lower. The calcining is preferably carried out in an environment of air or oxygen from the viewpoint of removing sulfate ions, acetate ions, and the like in the catalyst. Air or oxygen may be diluted with an inert gas in order to suppress the heat generation due to the catalyst during calcining. Examples of the inert gas include helium, neon, krypton, xenon, and radon.

(Catalyst shape)

The catalysts (A) and (B) may be molded as necessary. For example, in a case of a gas phase fixed bed reaction, it is preferable to determine the shapes of the catalysts (A) and (B) in consideration of the pressure loss in the reactor and the diffusion of a gas. Further, in both the gas phase fluidized bed reaction and the liquid phase reaction, it is preferable to determine the shapes of the catalyst (A) and (B) in consideration of the reaction conditions and material transfer.

Examples of the method for molding the catalysts (A) and (B) according to the present invention include a method using a powder molding machine such as a tableting molding machine, an extrusion molding machine, or a rolling granulator.

As the shape when molding the catalysts (A) and (B), any shape such as a spherical shape, a ring shape, a columnar shape, or a star shape can be adopted.

It is noted that the molded catalysts (A) and (B) may be ground and used as a powder. As necessary, an additive may be mixed with the catalysts (A) and (B) at the time of molding.

(Method for producing isobutyl aldehyde and/or methacrolein)

A method for producing isobutyl aldehyde and/or methacrolein according to a third embodiment is a method for subjecting isobutanol to a dehydrogenation reaction to produce isobutyl aldehyde and/or methacrolein, where isobutanol is subjected to a dehydrogenation reaction to produce isobutyl aldehyde and/or methacrolein in the presence of any one or both of the catalysts (A) and (B). In the method for producing isobutyl aldehyde and/or methacrolein of the third embodiment, a target product may be both isobutyl aldehyde and methacrolein, may be isobutyl aldehyde alone, or may be methacrolein alone. Among the above, the method for producing isobutyl aldehyde and/or methacrolein according to the third embodiment is preferably a method in which isobutyl aldehyde and methacrolein are produced with a total selectivity of 5% or more in a steady operation after the start of the reaction. It is still more preferably a method in which isobutyl aldehyde and methacrolein are produced with a total selectivity of 10% or more. Further, since it is preferable that the amount of isobutylene generated is small in the method for producing a dehydrogenated compound of the sixth embodiment, the isobutylene selectivity in the method for producing a dehydrogenated compound of the sixth embodiment in a steady operation is preferably 60% or less and more preferably 50% or less.

The isobutanol that is used as a starting raw material is not particularly limited, and it is preferably biomass-derived isobutanol from the viewpoint of environmental protection. As the starting raw material for isobutanol, fossil-derived isobutanol and biomass-derived isobutanol can be mixed and used.

The "biomass-derived isobutanol" is either isobutanol purified from an organic compound obtained by using fermentable sugar of biomass and subjecting it to a fermentation process or isobutanol obtained by a process including any one or more processes of catalytic chemical conversion and thermochemical conversion of biomass. The biomass can be broadly divided into one derived from resource crops and one derived from waste. Examples of the biomass derived from resource crops include food crops, wood, and flowers, and unused portions of these crops can also be used. Examples of the biomass derived from waste include food waste, sludge such as sewage, livestock excreta, and waste paper.

The dehydrogenation reaction of isobutanol may be carried out in the liquid phase or in the gas phase. When the reaction is carried out in the gas phase, a type of a fixed bed, a fluidized bed, or the like can be adopted. Hereinafter, when the reaction is carried out in the gas phase will be described; however, the present invention is not limited thereto.

For example, by vaporizing a raw material with a vaporizer, it can be supplied to a reactor as a raw material gas. The vaporizer is not particularly limited, and examples thereof include a jacket type, a horizontal tube type with a natural circulation system, an immersion tube type with a natural circulation system, a vertical short tube type with a natural circulation system, a rising membrane type having a vertical long tube, a descending membrane type having a horizontal tube, a horizontal tube type with a forced circulation system, a vertical tube type with a forced circulation system, and a coil type. It is also possible to use a method in which a heating coil is simply wound around a raw material supply pipe, and a raw material moving in the raw material supply pipe is vaporized in the raw material supply pipe before entering a reactor and supplied into the reactor in a gas state. The vaporizer is not particularly limited as well, similarly when a component other than the raw material, such as a diluent gas, is vaporized and supplied into the reactor.

The conditions for vaporizing the raw material are not particularly limited, and for example, the temperature can be 108°C or higher and 600°C or lower, and the pressure can be 0.05 MPa or higher and 1 MPa or lower in terms of absolute pressure.

In the raw material gas, the isobutanol concentration can be adjusted by diluting isobutanol with a diluent gas. The raw material gas may be a gas consisting only of isobutanol.

The diluent gas may be any gas that does not affect the dehydrogenation reaction of isobutanol, and examples thereof include air, nitrogen, helium, neon, krypton, xenon, radon, argon, methane, ethane, propane, butane, isobutane, carbon monoxide, carbon dioxide, nitric oxide, nitrogen dioxide, nitrous oxide, dinitrogen trioxide, dinitrogen tetroxide, dinitrogen pentoxide, and water vapor. Hydrogen may be used as a diluent gas as long as a side reaction is not promoted. The diluent gas included in the raw material gas may be one kind or two or more kinds. Moisture may be included in the raw material gas.

The lower limit of the isobutanol concentration in the raw material gas is not particularly limited; however, it is preferably 0.1% by volume or more with respect to the total volume of the raw material gas. The upper limit is not particularly limited, and it is 100% by volume or less.

When supplying water vapor to a reactor, the lower limit of the water vapor concentration in the raw material gas is preferably 2.5% by volume or more, more preferably 5.0% by volume or more, still more preferably 10.0% by volume or more, particularly preferably 15.0% by volume or more, and most preferably 20.0% by volume or more, with respect to the total volume of the raw material gas. The upper limit of the water vapor concentration in the raw material gas is preferably 80.0% by volume or less, more preferably 75.0% by volume or less, still more preferably 70.0% by volume or less, particularly preferably 65.0% by volume or less, and most preferably 60.0% by volume or less.

The lower limit of the reaction temperature (the temperature in the catalyst layer during the reaction) of the dehydrogenation reaction of isobutanol is preferably 320°C or higher, more preferably 340°C or higher, still more preferably 360°C or higher, and particularly preferably 380°C or higher. The upper limit of the reaction temperature is preferably 600°C or lower, more preferably 580°C or lower, still more preferably 560°C or lower, even still preferably 540°C or lower, particularly preferably 520°C or lower, and most preferably 500°C or lower. In a case where the reaction temperature is moderately high, the sequential dehydrogenation reaction from isobutyl aldehyde to methacrolein is promoted. In addition, the need to increase the amount of the catalyst and the need to reduce the supply rate of raw material gas are reduced, which is advantageous in terms of cost and productivity.

The reaction temperature is the lowest temperature among the temperatures of the catalyst layer in the reactor, which can be confirmed after the reaction reached a steady state shall be the reaction temperature. Therefore, when the catalyst layer has a temperature distribution, it is preferable to increase the number of measurement points or measure the temperature continuously in the catalyst filling direction. The method for controlling the reaction temperature is not particularly limited, and a known method can be adopted.

The reaction pressure in the dehydrogenation reaction of isobutanol is not particularly limited; however, it is preferably 0.050 MPa or more in terms of absolute pressure.

The upper limit of the reaction pressure is not particularly limited; however, it is preferably 10.0 MPa or less.

The reaction pressure is a value measured by a pressure sensor installed at a position where the influence of the pressure loss can be ignored with respect to the pressure at the inlet of the reactor.

[Method for producing methacrylic acid from isobutyl aldehyde and/or methacrolein]

The method for producing methacrylic acid according to the present invention is a method for producing methacrylic acid using isobutyl aldehyde produced according to the above-described method for producing isobutyl aldehyde, and methacrylic acid can be produced from isobutyl aldehyde with a high selectivity.

As the catalyst that is used in the production of methacrylic acid from isobutyl aldehyde and/or methacrolein, a known catalyst can be used, and examples thereof include a catalyst containing a heteropoly acid consisting of molybdenum and phosphorus, or iron phosphate.

The reaction of producing methacrylic acid can be carried out on a fixed bed. The catalyst layer for the second stage oxidation reaction is not particularly limited, and it may be an undiluted layer containing only the catalyst for the second stage oxidation reaction or may be a diluted layer further containing an inert carrier. In addition, the catalyst layer for the second stage oxidation reaction may be a single layer or may be a mixed layer composed of a plurality of layers.

Although it is economical to use air as the molecular oxygen source that is used in the reaction for producing methacrylic acid from isobutyl aldehyde and/or methacrolein, it is also possible to use air enriched with pure oxygen, as necessary.

As reaction gas of the reaction for producing methacrylic acid from isobutyl aldehyde and/or methacrolein, water (water vapor) may be added in addition to isobutyl aldehyde and/or methacrolein as well as molecular oxygen.

Although the reaction gas of the reaction for producing methacrylic acid from isobutyl aldehyde and/or methacrolein may contain a small amount of impurities such as lower saturated aldehyde, the amount thereof is preferably as small as possible. Further, the reaction gas of the reaction for producing methacrylic acid from isobutyl aldehyde and/or methacrolein may contain an inert gas such as nitrogen or carbon dioxide gas.

[Method for producing methacrylic acid ester]

The method for producing a methacrylic acid ester according to the present invention is a method of producing a methacrylic acid ester using the methacrylic acid produced according to the above-described method for producing methacrylic acid.

The method for producing a methacrylic acid ester according to the present invention includes a step of producing methacrylic acid according to the above-mentioned method for producing methacrylic acid, and a step of subjecting methacrylic acid to an esterification reaction with an alcohol having 1 to 20 carbon atoms to produce a methacrylic acid ester. According to such a method, a methacrylic acid ester can be produced from isobutyl aldehyde and/or methacrolein with a high selectivity.

For example, methacrylic acid produced according to the above-described production method is recovered by extraction, distillation operation, or the like, and it is subjected to an esterification reaction with methanol in the presence of an acid catalyst.

Examples of the alcohol having 1 to 20 carbon atoms include aliphatic alcohols such as methanol, ethanol, propanol, butanol, pentanol, hexanol, heptanol, octanol, nonanol, decanol, and eicosanol; and aromatic alcohols such as benzyl alcohol.

It is preferable to use a catalyst for the esterification reaction. The catalyst to be used is preferably acid catalysts, among which sulfuric acid or an ion exchange resin can be used. The ion exchange resin is preferably a strongly acidic cation exchange resin. Specific examples of the strongly acidic cation exchange resin include DIAION (registered trade name), PK216, RCP12H (manufactured by Mitsubishi Chemical Corporation), LEWATIT (registered trade name), K2431 (manufactured by Bayer AG), and Amberlyst (registered trade name) 15WET (manufactured by Rohm and Haas Company, Japan). These may be used singly, or two or more kinds thereof may be used in combination.

The flow direction of the reaction fluid in the esterification reaction may be either vertically upward or vertically downward, and it can be appropriately selected. When the swelling of the ion exchange resin that is used as the acid catalyst for the esterification reaction is large, the flow direction of the reaction fluid is preferably vertically upward. When the reaction fluid forms a non-uniform phase, the flow direction of the reaction fluid is preferably vertically downward.

When carrying out the esterification reaction by filling the fixed bed type reactor with an ion exchange resin, the flow-through amount of the raw material including methacrylic acid and an alcohol having 1 to 20 carbon atoms is preferably 0.10 times or more and more preferably 0.20 times or more in terms of the mass ratio to the amount of the ion exchange resin. In addition, the flow-through amount of the raw material is preferably 10.0 times or less and more preferably 5.0 times or less in terms of the mass ratio with respect to the amount of the ion exchange resin.

When a strongly acidic cation exchange resin is used as the acid catalyst, the reaction temperature of the esterification reaction is preferably 40°C or higher and 130°C or lower. The higher the reaction temperature is, the faster the reaction rate is, and thus the more efficiently the reaction can be carried out. The lower the reaction temperature is, the lower the deterioration rate of the ion exchange resin is, and thus the esterification reaction can be continuously carried out for a long time. The reaction temperature of the esterification can be appropriately determined to be the optimum temperature from the viewpoint of chemical equilibrium.

Regarding the raw material composition, from the viewpoint of chemical equilibrium, the process of the recovery step and the purification step can be simplified by increasing the concentration of any one of methacrylic acid or an alcohol having 1 to 20 carbon atoms and increasing the conversion rate of the raw material thereof having the lower concentration.

As described above, in the present invention, in a case where the catalysts (A) and (B) are used, a reduction treatment with hydrogen is not required, and isobutyl aldehyde and methacrolein can be efficiently produced from isobutanol while suppressing the oxidation and dimerization of isobutyl aldehyde.

[Examples]

Hereinafter, the present invention will be specifically described with reference to Examples; however, the present invention is not limited to the following description.

[Conversion rate and selectivity]

The analysis of the raw material gas and the product was carried out using gas chromatography. The isobutanol conversion rate and the selectivity for each product are defined as follows.

$$\text{Isobutanol conversion rate } (\%) = (b/a) \times 100$$

$$\text{W/F} = \text{feeding amount of isobutanol supplied to reactor (unit: g/h)/mass of}$$

catalyst with which reaction tube is filled" (unit: g)

$$\text{Isobutyl aldehyde selectivity (\%)} = (c/b) \times 100$$

$$\text{Methacrolein selectivity (\%)} = (d/b) \times 100$$

$$\text{Isobutyric acid selectivity (\%)} = (e/b) \times 100$$

$$\text{C4 butene selectivity (\%)} = (f/b) \times 100$$

$$\text{Propylene selectivity (\%)} = (g/b) \times 100$$

$$\text{Propionaldehyde selectivity (\%)} = (h/b) \times 100$$

$$\text{Ketone selectivity (\%)} = [(i/b) + (j/b)] \times 100$$

$$\text{Cyclic compound selectivity (\%)} = [(k/b) + (l/b) + (m/b)] \times 100$$

$$\text{COx (CO and CO}_2\text{) selectivity} = (n/b) \times 100$$

$$\text{Coke selectivity (\%)} = (o/b) \times 100$$

Unknown material selectivity (%) = 100 - isobutyl aldehyde selectivity - methacrolein selectivity - isobutyric acid selectivity - C4 butene selectivity - propylene selectivity - propionaldehyde selectivity - ketone selectivity - cyclic compound selectivity - COx selectivity- coke selectivity

However, the symbols in the above expressions have the following meanings.

a: Number of moles of isobutanol supplied/4
b: Number of moles of isobutanol reacted/4
c: Number of moles of isobutyl aldehyde generated/4
d: Number of moles of methacrolein generated/4
e: Number of moles of isobutyric acid generated/4
f: Number of moles of C4 butenes generated (isobutene, 1-butene, trans-2-butene, and cis-2-butene)/4
g: Number of moles of propylene generated/3
h: Number of moles of propionaldehyde generated/3
i: Number of moles of (2-methyl-3-pentanone generated/6 + number of moles of 3-methyl-2-butanone generated/5
j: Number of moles of ketones 2 (3-pentanone) generated/5
k: Number of moles of cyclic compounds 1 generated (methyl cyclopentenone)/6
l: Number of moles of cyclic compounds 2 generated (dimethyl cyclopentanone and dimethyl cyclopentenone)17
m: Number of moles of cyclic compound 3 generated (trimethyl cyclopentanone and trimethyl cyclopentenone)/8
n: Number of moles of carbon monoxide and carbon dioxide generated/1
o: Number of moles of coke generated/1

$$\text{Isobutyl aldehyde + methacrolein yield = (isobutyl aldehyde selectivity +}$$

$$\text{methacrolein selectivity)} \times \text{isobutanol conversion rate/100}$$

[Analysis method for acid amount in catalyst]

The acid amount of the catalyst was measured by BELCAT (manufactured by MicrotracBEL).

After setting the catalyst in a measurement cell, pretreatment was carried out at 550°C for 1 hour while allowing He to flow at 50 cc/min. After the pretreatment, the cell was cooled to 100°C, and a mixed gas of 1.0% by volume $NH_3$ and 99.0% by volume He was allowed to flow at 50 cc/min for 1 hour, thereby adsorbing $NH_3$ on the catalyst. Then, stabilization was carried out while allowing He as a carrier to flow at 30 cc/min, the temperature was raised to 800°C at 10°C/min, the desorption amount of $NH_3$ was measured, and the acid amount was calculated according to the following expression.

$$\text{Acid amount on catalyst (mmol/g) = amount of desorption of } NH_3$$

$$\text{(mmol)/weight of catalyst set in measurement cell (g)}$$

[Analysis method for base amount in catalyst]

The base amount of the catalyst was measured by BELCATII (manufactured by MicrotracBEL). After setting the catalyst in a measurement cell, pretreatment was carried out at 550°C for 1 hour while allowing He to flow at 50 cc/min. After the pretreatment, the cell was cooled to 50°C, and a mixed gas of 2.9% by volume $CO_2$ and 97.1% by volume Ar was allowed to flow at 50 cc/min for 1 hour, thereby adsorbing $CO_2$ on the catalyst. Then, stabilization was carried out while allowing He as a carrier to flow at 30 cc/min, the temperature was raised to 800°C at 10°C/min, the desorption amount of $CO_2$ was measured, and the base amount was calculated according to the following expression.

$$\text{Base amount on catalyst (mmol/g) = amount of desorption of } CO_2$$

$$\text{(mmol)/weight of catalyst set in measurement cell (g)}$$

[Analysis method for BET specific surface area of catalyst]

The catalyst set in the cell for measurement was vacuum-heated at 300°C overnight, and then the adsorption isotherm of the nitrogen gas at a liquid nitrogen temperature (77K) was measured with BELSORP-mini (manufactured by MicrotracBEL). The obtained adsorption isotherm of the nitrogen gas was analyzed by the BET method, and the BET specific surface area ($m^2$/g) was calculated.

[Evaluation method for coke precipitation amount after reaction evaluation]

The catalyst after the reaction was withdrawn from the reaction tube and pulverized until a uniform fine powder was obtained. After setting this in a measurement cell, the carbon precipitation amount was measured with an organic trace element analyzer (Micro corder JM10 manufactured by J-SCIENCE LAB Co., Ltd.), and the coke precipitation amount was calculated according to the following expression.

$$\text{Coke precipitation amount (C-mol) = catalyst filling amount} \times \text{carbon}$$

$$\text{concentration (\% by mass) obtained by analysis/100/12.01 (g/mol)}$$

[Example 1]

(Preparation of catalyst for dehydrogenation)

1.9 g of zirconyl nitrate dihydrate (manufactured by FUJIFILM Wako Pure Chemical Corporation) was added to 70 mL of distilled water (manufactured by FUJIFILM Wako Pure Chemical Corporation), and stirring was carried out until it was completely dissolved to obtain a solution A.

45 g of iron (III) nitrate nonahydrate (manufactured by FUJIFILM Wako Pure Chemical Corporation) was added to 600 mL of distilled water, and stirring was carried out until it was completely dissolved to obtain a solution B. 60 mL of distilled water was added to 40 mL of ammonia water (manufactured by FUJIFILM Wako Pure Chemical Corporation) to obtain a solution C.

The solution A was added to the solution B, and stirring was carried out for 30 minutes. Then, the solution C was added dropwise thereto until the pH reached about 7, and stirring was carried out for 1 hour. The obtained precipitate was separated by suction filtration for 24 hours and dried at 110°C overnight. The obtained solid was pulverized in a mortar and calcined in the air at 500°C for 2 hours.

After the calcining, it was further pulverized finely again in a mortar to obtain $ZrO_2$ (7)-FeOx, which is a composite oxide. Here, the number in parentheses indicates the content (% by mole) of each component. The content of FeOx was 93% by mole.

0.60 g of potassium nitrate (manufactured by FUJIFILM Wako Pure Chemical Corporation) was dissolved in 3.0 mL of distilled water (manufactured by FUJIFILM Wako Pure Chemical Corporation) to prepare an aqueous potassium nitrate solution. The aqueous potassium nitrate solution was uniformly added dropwise onto 6.0 g of $ZrO_2$ (7)-FeOx aliquoted into a mortar, and stirring was carried out for 15 minutes. After drying at 25°C for 24 hours, drying was carried out at 40°C for 2 hours and 60°C for 2 hours using a vacuum dryer, and drying was further carried out at 110°C for 24 hours using a dryer. After the drying treatment, grinding was carried out in a mortar, followed by calcining in the air in a calcining furnace at 550°C for 12 hours. After the calcining, grinding was carried out again in a mortar, and pressing was carried out at 2.0 tons for 15 minutes using a tablet molder to form a tablet, thereby obtaining K(5)/$ZrO_2$ (6.7)-FeOx (88.3) (catalyst (abbreviation): B-1, average electronegativity: 2.55) having a particle diameter of 300 to 850 $\mu$m by using a sieve.

Here, the number in parentheses indicates the content (% by mole) of each component. In addition, the acid amount of the catalyst B-1 was 0.004 mmol/g, the base amount was 0.187 mmol/g, and the BET specific surface area was 30 $m^2$/g.

(Catalyst performance evaluation)

The inside of a vertical-type tubular-shaped reaction tube was filled with 1.1 g of the catalyst B-1 to form a catalyst layer. In the reactor, the temperature of the catalyst layer was adjusted by using an electric furnace so that the temperature of the catalyst layer became a predetermined temperature. Isobutanol (manufactured by FUJIFILM Wako Pure Chemical Corporation, specific gravity: 0.80 g/mL) and pure water were each adjusted to a flow rate of 1.9 mL/hour and 2.0 mL/hour using a syringe pump and supplied into a vaporizer set at 150°C and vaporized. Nitrogen gas as a diluent gas was supplied into the vaporizer at a flow rate of 1,140 mL (standard state)/hour using a mass flow meter, where it was supplied into a reactor together with the vaporized isobutanol. The isobutanol concentration in the raw material gas supplied to the catalyst layer was 11.4% by volume, the W/F was 0.70 $h^{-1}$, and the temperature (the reaction temperature) of the catalyst layer during the reaction was 450°C. A pressure gauge for measuring the reaction pressure was installed between the vaporizer and the reactor inlet. It is noted that the reaction pressure was the atmospheric pressure.

After the reaction reached a steady state, the gas on the outlet side of the reactor was sampled and gas chromatography (manufactured by Shimadzu Corporation, GC-8A) was used to quantify propylene, isobutylene, 1-butene, cis-2-butene, trans-2-butene, CO, and $CO_2$. In addition, the reaction gas discharged from the reactor outlet side was collected by being allowed to pass through an ice-cooled trap, and Gas chromatography (manufactured by Shimadzu Corporation, GC-2014, internal standard: isopropanol) was used to quantify isobutanol, isobutyl aldehyde, isobutyric acid, 2,4-dimethyl-3-pentanone, methacrolein, propionaldehyde, 2-methyl-3-pentanone, 3-methyl-2-butanone, 3-pentanone, dimethyl cyclopentanone, dimethyl cyclopentenone, methyl cyclopentenone, trimethyl cyclopentanone, and trimethyl cyclopentane.

[Example 2]

$Al_2O_3$ (7)-FeOx, which is a composite oxide, was obtained in the same manner as in Example 1 except that 4.9 g of aluminum nitrate (manufactured by FUJIFILM Wako Pure Chemical Corporation) was added to 170 mL of distilled water (manufactured by FUJIFILM Wako Pure Chemical Corporation), and stirring was carried out until it was completely dissolved to obtain a solution as the solution A. Further, K (5)/$Al_2O_3$ (6.7)-FeOx (88. 3) (catalyst (abbreviation): B-2, average electronegativity: 2.56) was obtained in the same manner as Example 1 except that $Al_2O_3$ (7)-FeOx was used. Here, the number in parentheses indicates the content (% by mole) of each component. In addition, the acid amount of the catalyst B-2 was 0.000 mmol/g, and the base amount was 0.268 mmol/g.

The catalyst performance was evaluated in the same manner as in Example 1 except that 1.1 g of the catalyst B-2 was used instead of the catalyst B-1.

[Example 3]

γ-alumina (manufactured by Strem Chemicals, Inc.) was calcined in the air at 550°C for 12 hours using an electric furnace as a pretreatment. 0.60 g of potassium nitrate (manufactured by FUJIFILM Wako Pure Chemical Corporation) was dissolved in 4.0 mL of distilled water (manufactured by FUJIFILM Wako Pure Chemical Corporation) to prepare an aqueous potassium nitrate solution. The aqueous potassium nitrate solution was uniformly added dropwise onto 4.0 g of the pretreated alumina aliquoted into a mortar, and stirring was carried out for 15 minutes. After drying at 25°C for 24 hours, drying was carried out at 40°C for 2 hours and 60°C for 2 hours using a vacuum dryer, and drying was further carried out at 110°C for 24 hours using a dryer. After the drying treatment, grinding was carried out in a mortar, followed by calcining in the air in a calcining furnace at 550°C for 12 hours. After the calcining, grinding was carried out again in a mortar, and pressing was carried out at 2 tons for 15 minutes using a tablet molder to form a tablet, thereby preparing K (5)/Al$_2$O$_3$ (catalyst (abbreviation): B-3, average electronegativity: 2.48) having a particle diameter of 300 to 850 μm by using a sieve. Here, the number in parentheses indicates the ratio (% by mole) of the supported metal element to the Al atomic weight in the catalyst. In addition, the acid amount of the catalyst B-3 was 0.079 mmol/g, the base amount was 0.439 mmol/g, and the BET specific surface area was 173 m$^2$/g. The content of Al$_2$O$_3$ was 95% by mole.

The catalyst performance was evaluated in the same manner as in Example 1 except that 1.1 g of the catalyst B-3 was used instead of the catalyst B-1.

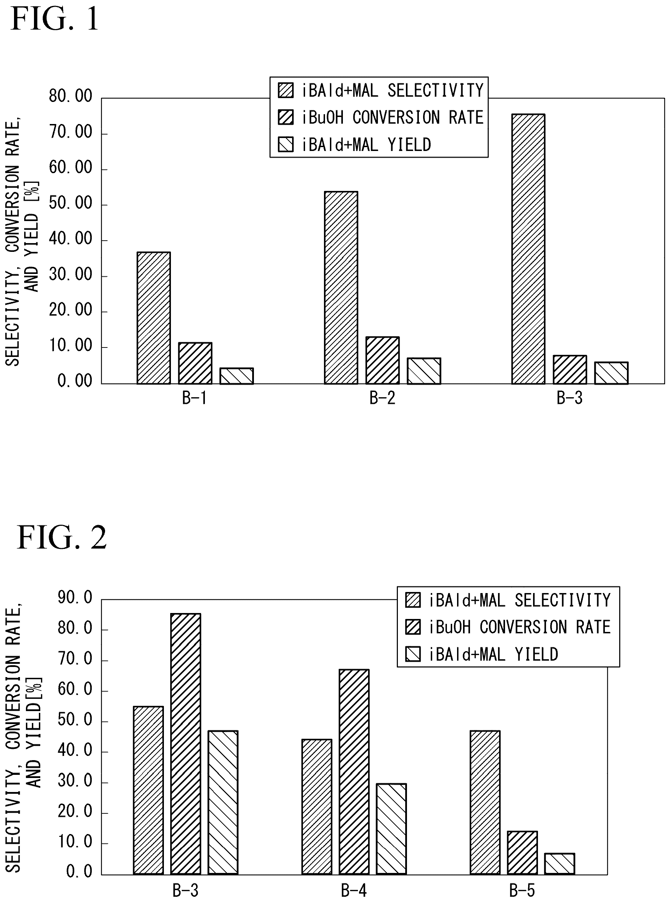

Table 1 shows the reaction conditions and evaluation results in Examples 1 to 3. In addition, Fig. 1 shows the isobutanol conversion rate, and the selectivities and yields of isobutyl aldehyde and methacrolein (MAL) by the catalyst of each example.

[Table 1]

| | | Example 1 | Example 2 | Example 3 |
|---|---|---|---|---|
| Catalyst name | | K (5)/ZrO$_2$ (6.7)-FeOx (88.3) | K (5)/Al$_2$O$_3$ (6.7)-FeOx (88.3) | K (5) /Al$_2$O$_3$ |
| Catalyst (abbreviation) | | B-1 | B-2 | B-3 |
| Potassium supporting rate [% by mole] | | 5 | 5 | 5 |
| Acid amount [mmol/g] | | 0.004 | 0.000 | 0.079 |
| Base amount [mmol/g] | | 0.187 | 0.268 | 0.439 |
| Average electronegativity | | 2.55 | 2.56 | 2.48 |
| Reaction temperature [°C] | | 450 | 450 | 450 |
| Catalyst mass [g] | | 1.1 | 1.1 | 1.1 |
| W/F [h$^{-1}$] | | 0.70 | 0.70 | 0.70 |
| Conditions for supply amount of raw material | | a | a | a |
| Supply amount of raw material | Isobutanol [mL/hour] | 1.9 | 1.9 | 1.9 |
| | Water [mL/hour] | 2.0 | 2.0 | 2.0 |
| | Nitrogen [mL (standard state)/hour] | 1140 | 1140 | 1140 |
| Composition of raw material gas [% by volume] | Isobutanol | 11.4 | 11.4 | 11.4 |
| | Water | 60.2 | 60.2 | 60.2 |
| | Nitrogen | 28.4 | 28.4 | 28.4 |
| Conversion rate [%] | | 11.3 | 12.9 | 7.7 |

14

(continued)

| | | Example 1 | Example 2 | Example 3 |
|---|---|---|---|---|
| Selectivity [%] | Isobutyl aldehyde + methacrolein | 36.69 | 53.76 | 75.46 |
| | Isobutyl aldehyde | 36.52 | 53.76 | 75.44 |
| | Methacrolein | 0.18 | 0.00 | 0.02 |
| | Propionaldehyde | 0.25 | 0.00 | 0.04 |
| | Propylene | 7.79 | 0.00 | 0.60 |
| | C4 butenes | 0.91 | 0.00 | 14.54 |
| | COx | 25.98 | 26.44 | 0.20 |
| | Isobutyric acid | 2.71 | 0.00 | 0.22 |
| | Ketones | 5.93 | 0.00 | 0.02 |
| | Cyclic compounds | 1.59 | 0.00 | 0.55 |
| | Coke | 11.32 | 0.00 | 5.52 |
| | Unknown material | 6.83 | 19.80 | 2.86 |
| Yield [%] | Isobutyl aldehyde + methacrolein | 4.1 | 6.9 | 5.8 |

As shown in Table 1 and Fig. 1, catalysts having an average electronegativity in a range of 2.1 to 2.8 generated isobutyl aldehyde and methacrolein without carrying out a hydrogen reduction.

[Example 5]

The catalyst performance was evaluated in the same manner as in Example 1 except for the following points. 1.1 g of the catalyst B-3 was used instead of the catalyst B-1. The flow rate of isobutanol into a reactor was adjusted to 1.9 mL/hour without supplying water to the reactor, and the reaction temperature was set to 500°C. The flow rate of nitrogen gas was set to 3,660 mL (standard state)/hour. The isobutanol concentration in the raw material gas supplied to the catalyst layer was 11.4% by volume, the W/F was 0.70 $h^{-1}$, and the temperature (the reaction temperature) of the catalyst layer during the reaction was 500°C.

[Example 6]

Potassium was supported by the same method as in Example 1 except that 6.0 g of titanium oxide (manufactured by FUJIFILM Wako Pure Chemical Corporation) was used instead of ZrOz(7)-FeOx, and K (5)/$TiO_2$ (catalyst (abbreviation): B-4, average electronegativity: 2.59) was prepared. Here, the number in parentheses indicates the ratio (% by mole) of the supported metal element to the Ti atomic weight in the catalyst. In addition, the acid amount of the catalyst B-4 was 0.002 mmol/g, the base amount was 0.022 mmol/g, and the BET specific surface area was 13 $m^2$/g. The content of $TiO_2$ was 95% by mole.
The catalyst performance was evaluated in the same manner as in Example 5 except that 1.1 g of the catalyst B-4 was used instead of the catalyst B-3.

[Example 7]

A BEA type zeolite (manufactured by Tosoh Corporation, $SiO_2$/$Al_2O_3$ = 500, HSZ-980HOA) was calcined in the air at 550°C for 12 hours using an electric furnace as a pretreatment. 6.1 g of potassium nitrate (manufactured by FUJIFILM Wako Pure Chemical Corporation) was dissolved in 100 mL of distilled water (manufactured by FUJIFILM Wako Pure Chemical Corporation) to prepare an aqueous potassium nitrate solution. A pretreated BEA type zeolite was added thereto, stirring was carried out with a stirrer at 70°C for 3 hours, and the solid content was recovered by centrifugation. This operation was repeated 3 times. Next, the recovered solid content was washed with distilled water, and the solid content was recovered by centrifugation. This washing operation was repeated twice. Further, the solid component was washed with 2-propanol (manufactured by FUJIFILM Wako Pure Chemical Corporation), and the solid component was recovered by centrifugation.

The recovered solid content was calcined in the air at 550°C for 12 hours using an electric furnace to prepare K-BEA in which potassium was supported on zeolite.

0.65 g of potassium nitrate (manufactured by FUJIFILM Wako Pure Chemical Corporation) was dissolved in 3.0 mL of distilled water (manufactured by FUJIFILM Wako Pure Chemical Corporation) to prepare an aqueous potassium nitrate solution. The aqueous potassium nitrate solution was uniformly added dropwise onto 5.0 g of K-BEA aliquoted into a mortar, and stirring was carried out for 15 minutes. After drying at 25°C for 24 hours, drying was carried out at 40°C for 2 hours and 60°C for 2 hours using a vacuum dryer, and drying was further carried out at 110°C for 24 hours using a dryer. This operation was repeated twice. Next, grinding was carried out in a mortar, followed by calcining in the air in a calcining furnace at 550°C for 12 hours. After the calcining, grinding was carried out again in a mortar, and pressing was carried out at 2 tons for 15 minutes using a tablet molder to form a tablet, thereby preparing K (10)/K-BEA (catalyst (abbreviation): B-5, average electronegativity: 2.78) having a particle diameter of 300 to 850 $\mu$m by using a sieve. Here, the number in parentheses indicates the ratio (% by mole) of the supported metal element to the Si atomic weight in the catalyst. In addition, the acid amount in the catalyst B-5 was 0.000 mmol/g, and the base amount was 0.005 mmol/g.

The catalyst performance was evaluated in the same manner as in Example 5 except that 1.1 g of the catalyst B-5 was used instead of the catalyst B-3.

Table 2 shows the reaction conditions and evaluation results in Examples 5 to 7. In addition, Fig. 2 shows the isobutanol conversion rate, and the selectivities and yields of isobutyl aldehyde and methacrolein (MAL) by the catalyst of each example.

[Table 2]

| | | Example 5 | Example 6 | Example 7 |
|---|---|---|---|---|
| Catalyst name | | K (5)/Al$_2$O$_3$ | K (5)/TiO$_2$ | K (10)/K-BEA |
| Catalyst (abbreviation) | | B-3 | B-4 | B-5 |
| Potassium supporting rate [% by mole] | | 5 | 5 | 10 |
| Acid amount [mmol/g] | | 0.079 | 0.002 | 0.000 |
| Base amount [mmol/g] | | 0.439 | 0.022 | 0.005 |
| Average electronegativity | | 2.48 | 2.59 | 2.78 |
| Reaction temperature [°C] | | 500 | 500 | 500 |
| Catalyst mass [g] | | 1.1 | 1.1 | 1.1 |
| W/F [h$^{-1}$] | | 0.70 | 0.69 | 0.69 |
| Conditions for supply amount of raw material | | b | b | b |
| Supply amount of raw material | Isobutanol [mL/hour] | 1.9 | 1.9 | 1.9 |
| | Water [mL/hour] | 0.0 | 0.0 | 0.0 |
| | Nitrogen [mL (standard state)/hour] | 3660 | 3660 | 3660 |
| Composition of raw material gas [% by volume] | Isobutanol | 11.4 | 11.4 | 11.4 |
| | Water | 0.0 | 0.0 | 0.0 |
| | Nitrogen | 88.6 | 88.6 | 88.6 |
| Conversion rate [%] | | 85.4 | 67.1 | 14.1 |

(continued)

| | | Example 5 | Example 6 | Example 7 |
|---|---|---|---|---|
| Selectivity [%] | Isobutyl aldehyde + methacrolein | 55.0 | 44.2 | 47.0 |
| | Isobutyl aldehyde | 54.9 | 43.8 | 46.8 |
| | Methacrolein | 0.0 | 0.4 | 0.2 |
| | Propionaldehyde | 0.0 | 0.1 | 0.1 |
| | Propylene | 3.7 | 13.9 | 1.2 |
| | C4 butenes | 29.3 | 11.6 | 48.1 |
| | COx | 1.1 | 1.4 | 0.2 |
| | Isobutyric acid | 0.6 | 0.1 | 0.0 |
| | Ketones | 2.4 | 0.5 | 0.0 |
| | Cyclic compounds | 0.8 | 0.6 | 0.1 |
| | Coke | 0.7 | 0.0 | 0.0 |
| | Unknown material | 6.2 | 27.8 | 3.3 |
| Yield [%] | Isobutyl aldehyde + methacrolein | 46.9 | 29.6 | 6.6 |

As shown in Table 2 and Fig. 2, the selectivities of isobutyl aldehyde and methacrolein were also high in Examples 5 to 7 using the catalysts B-3 to 5 in which an alkali metal was supported on alumina, titanium oxide, and zeolite.

[Example 8]

The catalyst performance was evaluated in the same manner as in Example 1 except for the following points. 1.1 g of the catalyst B-3 was used instead of the catalyst B-1. The flow rates of isobutanol and water into a reactor were each set to 1.9 mL/hour and 1.0 mL/hour, the flow rate of nitrogen gas was set to 2,400 mL (standard state)/hour, and the reaction temperature was set to 500°C. The isobutanol concentration in the raw material gas supplied to the catalyst layer was 11.4% by volume, the water concentration was 29.6% by volume, the W/F was 0.70 h$^{-1}$, and the temperature (the reaction temperature) of the catalyst layer during the reaction was 500°C.

[Example 9]

After calcining the catalyst A-1 in an electric furnace at 700°C for 12 hours, potassium was supported by the same method as in Example 1 to obtain K (5)/Al$_2$O$_3$-700°C (catalyst (abbreviation): B-6, average electronegativity: 2.48) was prepared as a catalyst for dehydrogenation. Here, the number in parentheses indicates the ratio (% by mole) of the supported metal element to the Al atomic weight in the catalyst. The content of Al$_2$O$_3$ was 95% by mole.
The catalyst performance was evaluated in the same manner as in Example 7 except that 1.1 g of the catalyst B-6 was used instead of the catalyst B-3.

[Example 10]

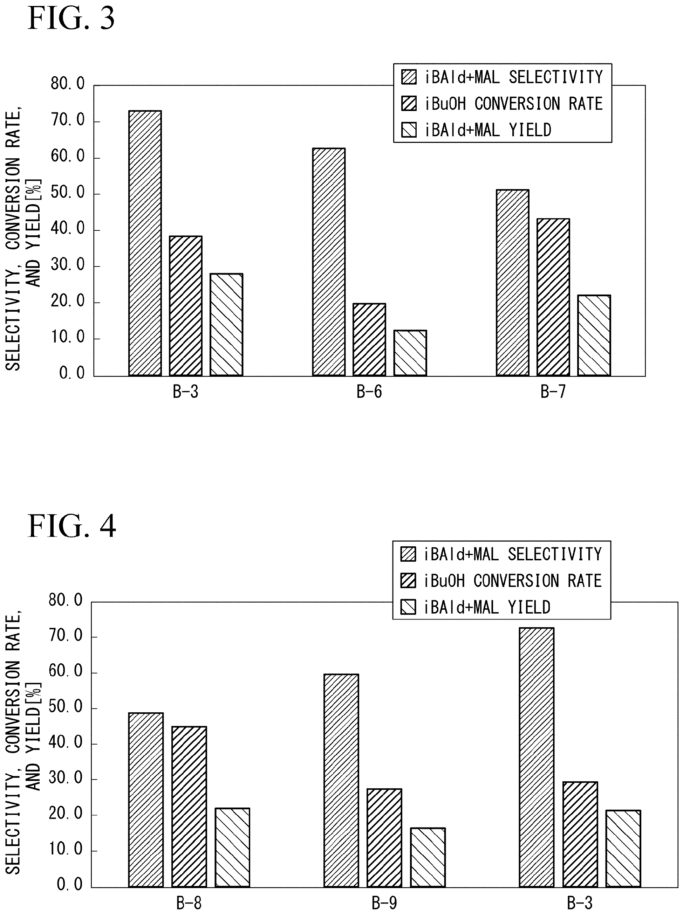

Potassium was supported by the same method as in Example 1 except that γ-alumina (SA63158 manufactured by Saint-Gobain SA) was used, and K (5)/SA63158 (catalyst (abbreviation): B-7, average electronegativity: 2.48) was prepared as a catalyst for dehydrogenation. Here, the number in parentheses indicates the ratio (% by mole) of the supported metal element to the Al atomic weight in the catalyst. The content of γ-alumina was 95% by mole.
The catalyst performance was evaluated in the same manner as in Example 7 except that 1.0 g of the catalyst B-7 was used instead of the catalyst B-3.
Table 3 shows the reaction conditions and evaluation results in Examples 8 to 10. In addition, Fig. 3 shows the isobutanol conversion rate, and the selectivities and yields of isobutyl aldehyde and methacrolein (MAL) by the catalyst of each example.

[Table 3]

| | | Example 8 | Example 9 | Example 10 |
|---|---|---|---|---|
| Catalyst name | | K (5)/Al$_2$O$_3$ | K (5) /Al$_2$O$_3$-700°C | K (5)/SA63158 |
| Catalyst (abbreviation) | | B-3 | B-6 | B-7 |
| Potassium supporting rate [% by mole] | | 5.0 | 5.0 | 5.0 |
| Average electronegativity | | 2.48 | 2.48 | 2.48 |
| Reaction temperature [°C] | | 500 | 500 | 500 |
| Catalyst mass [g] | | 1.1 | 1.1 | 1.0 |
| W/F [h$^{-1}$] | | 0.70 | 0.70 | 0.67 |
| Conditions for supply amount of raw material | | c | c | c |
| Supply amount of raw material | Isobutanol [ml/hour] | 1.9 | 1.9 | 1.9 |
| | Water [ml/hour] | 1.0 | 1.0 | 1.0 |
| | Nitrogen [ml (standard state)/hour] | 2400 | 2400 | 2400 |
| Composition of raw material gas [% by volume] | Isobutanol | 11.4 | 11.4 | 11.4 |
| | Water | 29.6 | 29.6 | 29.6 |
| | Nitrogen | 59.0 | 59.0 | 59.0 |
| Conversion rate [%] | | 38.4 | 19.7 | 43.2 |
| Selectivity [%] | Isobutyl aldehyde + methacrolein | 73.0 | 62.7 | 51.1 |
| | Isobutyl aldehyde | 73.0 | 62.7 | 50.9 |
| | Methacrolein | 0.0 | 0.0 | 0.2 |
| | Propionaldehyde | 0.0 | 0.0 | 0.6 |
| | Propylene | 2.6 | 14.5 | 6.1 |
| | C4 butenes | 15.9 | 4.8 | 27.0 |
| | COx | 0.8 | 5.5 | 2.1 |
| | Isobutyric acid | 1.2 | 1.4 | 0.6 |
| | Ketones | 1.3 | 4.8 | 1.5 |
| | Cyclic compounds | 0.4 | 0.8 | 0.9 |
| | Coke | 1.5 | 2.5 | 0.0 |
| | Unknown material | 2.7 | 2.6 | 10.1 |
| Yield [%] | Isobutyl aldehyde + methacrolein | 28.0 | 12.4 | 22.1 |

As shown in Table 3 and Fig. 3, the selectivities of isobutyl aldehyde and methacrolein were also high in Examples 8 to 10 using the catalysts B-3, 6, and 7 in which an alkali metal was supported on various alumina.

[Example 11]

Potassium was supported by the same method as in Example 1 except that the reference catalyst (JRC-ALOS) according to the Catalysis Society of Japan was used as an alumina carrier, and K (5)/AL05 (catalyst (abbreviation)):

B-8, average electronegativity: 2.48) was prepared as a catalyst for dehydrogenation. Here, the number in parentheses indicates the ratio (% by mole) of the supported metal element to the Al atomic weight in the catalyst. The BET specific surface area was 197 $m^2/g$. The content of JRC-ALO5 was 95% by mole.

The catalyst performance was evaluated in the same manner as in Example 1 except that 1.0 g of the catalyst B-8 was used instead of the catalyst B-1 and the reaction temperature was set to 500°C.

[Example 12]

Potassium was supported by the same method as in Example 1 except that the reference catalyst (JRC-ALO8) according to the Catalysis Society of Japan was used as an alumina carrier, and K (5)/AL08 (catalyst (abbreviation)): B-9, average electronegativity: 2.48) was prepared as a catalyst for dehydrogenation. Here, the number in parentheses indicates the ratio (% by mole) of the supported metal element to the Al atomic weight in the catalyst. The BET specific surface area was 158 $m^2/g$. The content of JRC-ALO8 was 95% by mole.

The catalyst performance was evaluated in the same manner as in Example 1 except that 1.0 g of the catalyst B-9 was used instead of the catalyst B-1.

[Example 13]

The catalyst performance was evaluated in the same manner as in Example 11 except that 1.1 g of the catalyst B-3 was used instead of the catalyst B-9.

Table 4 shows the reaction conditions and evaluation results in Examples 11 to 13. In addition, Fig. 4 shows the isobutanol conversion rate, and the selectivities and yields of isobutyl aldehyde and methacrolein (MAL) by the catalyst of each example.

[Table 4]

| | | Example 11 | Example 12 | Example 13 |
|---|---|---|---|---|
| Catalyst name | | K (5)/AL05 | K (5)/AL08 | K (5)/Al$_2$O$_3$ |
| Catalyst (abbreviation) | | B-8 | B-9 | B-3 |
| Potassium supporting rate [% by mole] | | 5.0 | 5.0 | 5.0 |
| Average electronegativity | | 2.48 | 2.48 | 2.48 |
| Reaction temperature [°C] | | 500 | 500 | 500 |
| Catalyst mass [g] | | 1.0 | 1.0 | 1.1 |
| W/F [h$^{-1}$] | | 0.67 | 0.67 | 0.70 |
| Conditions for supply amount of raw material | | a | a | a |
| Supply amount of raw material | Isobutanol [mL/hour] | 1.9 | 1.9 | 1.9 |
| | Water [mL/hour] | 2.0 | 2.0 | 2.0 |
| | Nitrogen [mL (standard state)/hour] | 1140 | 1140 | 1140 |
| Composition of raw material gas [% by volume] | Isobutanol | 11.4 | 11.4 | 11.4 |
| | Water | 60.2 | 60.2 | 60.2 |
| | Nitrogen | 28.4 | 28.4 | 28.4 |
| Conversion rate [%] | | 44.9 | 27.4 | 29.4 |

(continued)

|  |  | Example 11 | Example 12 | Example 13 |
|---|---|---|---|---|
| Selectivity [%] | Isobutyl aldehyde + methacrolein | 48.8 | 59.7 | 72.7 |
|  | Isobutyl aldehyde | 48.6 | 59.6 | 72.5 |
|  | Methacrolein | 0.2 | 0.1 | 0.2 |
|  | Propionaldehyde | 0.4 | 1.4 | 0.4 |
|  | Propylene | 4.7 | 16.9 | 2.3 |
|  | C4 butenes | 23.4 | 5.4 | 17.4 |
|  | COx | 1.5 | 5.6 | 0.7 |
|  | Isobutyric acid | 1.0 | 0.9 | 1.2 |
|  | Ketones | 0.7 | 2.3 | 0.8 |
|  | Cyclic compounds | 1.8 | 0.4 | 0.3 |
|  | Coke | 1.0 | 1.8 | 1.5 |
|  | Unknown material | 16.7 | 5.6 | 2.6 |
| Yield [%] | Isobutyl aldehyde + methacrolein | 21.9 | 16.4 | 21.3 |

As shown in Table 4 and Fig. 4, the selectivities of isobutyl aldehyde and methacrolein were also high in Examples 11 to 13 using the catalysts B-3, 8, and 9 in which an alkali metal was supported on various alumina.

[Example 14]

$\gamma$-alumina (manufactured by Strem Chemicals, Inc.) was calcined in the air at 550°C for 12 hours using an electric furnace as a pretreatment. 0.89 g of potassium nitrate was dissolved in 4.0 mL of distilled water (manufactured by FUJIFILM Wako Pure Chemical Corporation) to prepare an aqueous potassium nitrate solution. The aqueous potassium nitrate solution was uniformly added dropwise to 6.0 g of the pretreated alumina aliquoted into a mortar, and stirring was carried out for 15 minutes. After drying at 25°C for 24 hours, drying was carried out at 40°C for 2 hours and 60°C for 2 hours using a vacuum dryer, and drying was further carried out at 110°C for 24 hours using a dryer. After the drying treatment, grinding was carried out in a mortar, followed by calcining in the air in a calcining furnace at 550°C for 12 hours. After the calcining, grinding was carried out again in a mortar, and pressing was carried out at 2 tons for 15 minutes using a tablet molder to form a tablet, thereby preparing K (7.5)/$Al_2O_3$ (a catalyst B-10) having a particle diameter of 300 to 850 $\mu$m by using a sieve. Here, the number in parentheses indicates the ratio (% by mole) of the supported metal element to the Al atomic weight in the catalyst. In addition, the acid amount of the catalyst L was 0.040 mmol/g, the base amount was 0.590 mmol/g, and the BET specific surface area was 159 m$^2$/g. The content of $Al_2O_3$ was 92.5% by mole.
1.1 g of the catalyst B-10 was used instead of the catalyst B-3, the flow rate of isobutanol into a reactor was adjusted to 1.9 mL/hour, and the reaction temperature was set to 500°C. The catalyst performance was evaluated in the same manner as in Example 1 except that the flow rate of nitrogen gas was set to 1,140 mL (standard state)/hour, the isobutanol concentration in the raw material gas supplied to the catalyst layer was set to 28.8% by volume, and the W/F was set to 0.70 h$^{-1}$.

[Example 15]

$\gamma$-alumina (manufactured by Strem Chemicals, Inc.) was calcined in the air at 550°C for 12 hours using an electric furnace as a pretreatment. 0.89 g of potassium nitrate was dissolved in 4.0 mL of distilled water (manufactured by FUJIFILM Wako Pure Chemical Corporation) to prepare an aqueous potassium nitrate solution. The aqueous potassium nitrate solution was uniformly added dropwise to 6.0 g of the pretreated alumina aliquoted into a mortar, and stirring was carried out for 15 minutes. After drying at 25°C for 24 hours, drying was carried out at 40°C for 2 hours and 60°C for 2 hours using a vacuum dryer, and drying was further carried out at 110°C for 24 hours using a dryer. After the drying treatment, grinding was carried out in a mortar, followed by calcining in the air in a calcining furnace at 850°C for 12 hours. After the calcining, grinding was carried out again in a mortar, and pressing was carried out at 2 tons for 15 minutes using a tablet molder to form a tablet, thereby preparing K (7.5)/$Al_2O_3$-850°C (a catalyst B-11) having a particle diameter of 300 to 850 μm by using a sieve. Here, the number in parentheses indicates the ratio (% by mole) of the supported metal element to the Al atomic weight in the catalyst. The content of $Al_2O_3$ was 92.5% by mole.

The catalyst performance was evaluated in the same manner as in Example 14 except that 1.1 g of the catalyst B-11 was used instead of the catalyst B-10.


[Example 16]

K (7.5)/$Al_2O_3$ (a catalyst B-12) was prepared as a catalyst for dehydrogenation by the same method as in Example 14 except that 0.58 g of potassium acetate (manufactured by FUJIFILM Wako Pure Chemical Corporation) was used as a potassium raw material. Here, the number in parentheses indicates the ratio (% by mole) of the supported metal element to the Al atomic weight in the catalyst. In addition, the acid amount of the catalyst N was 0.030 mmol/g, the base amount was 0.650 mmol/g, and the BET specific surface area was 177 $m^2$/g. The content of $Al_2O_3$ was 92.5% by mole.

The catalyst performance was evaluated in the same manner as in Example 14 except that 1.1 g of the catalyst B-12 was used instead of the catalyst B-10.

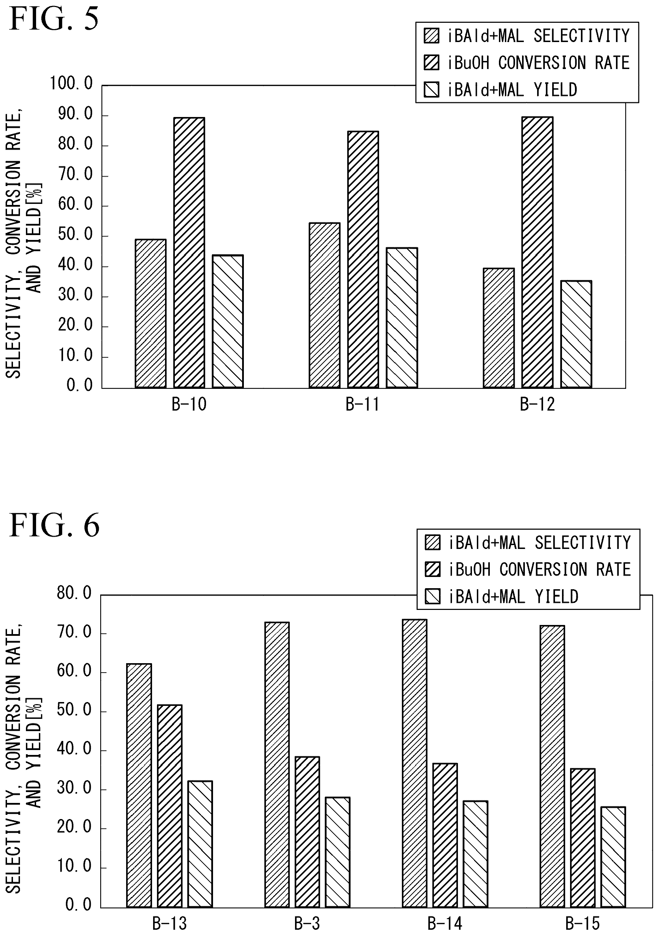

Table 5 shows the reaction conditions and evaluation results in Examples 14 to 16. In addition, Fig. 5 shows the isobutanol conversion rate, and the selectivities and yields of isobutyl aldehyde and methacrolein (MAL) by the catalyst of each example.

[Table 5]

| | | Example 14 | Example 15 | Example 16 |
|---|---|---|---|---|
| Catalyst name | | K (7.5)/$Al_2O_3$-550°C | K (7.5)/$Al_2O_3$-850°C | K (7.5)/$Al_2O_3$-K acetate |
| Catalyst (abbreviation) | | B-10 | B-11 | B-12 |
| Potassium supporting rate [% by mole] | | 7.5 | 7.5 | 7.5 |
| Average electronegativity | | 2.47 | 2.47 | 2.47 |
| Reaction temperature [°C] | | 500 | 500 | 500 |
| Catalyst mass [g] | | 1.1 | 1.1 | 1.1 |
| W/F [$h^{-1}$] | | 0.70 | 0.71 | 0.70 |
| Conditions for supply amount of raw material | | d | d | d |
| Supply amount of raw material | Isobutanol [mL/hour] | 1.9 | 1.9 | 1.9 |
| | Water [mL/hour] | 0.0 | 0.0 | 0.0 |
| | Nitrogen [mL (standard state) /hour] | 1140 | 1140 | 1140 |
| Composition of raw material gas [% by volume] | Isobutanol | 28.8 | 28.8 | 28.8 |
| | Water | 0.0 | 0.0 | 0.0 |
| | Nitrogen | 71.2 | 71.2 | 71.2 |
| Conversion rate [%] | | 89.4 | 84.8 | 89.6 |

(continued)

|  |  | Example 14 | Example 15 | Example 16 |
|---|---|---|---|---|
| Selectivity [%] | Isobutyl aldehyde + methacrolein | 48.9 | 543 | 39.4 |
|  | Isobutyl aldehyde | 48.5 | 53.8 | 39.0 |
|  | Methacrolein | 0.4 | 0.6 | 0.4 |
|  | Propionaldehyde | 0.5 | 0.8 | 0.5 |
|  | Propylene | 3.3 | 3.7 | 3.6 |
|  | C4 butenes | 8.1 | 13.0 | 6.8 |
|  | COx | 5.5 | 4.1 | 5.6 |
|  | Isobutyric acid | 0.2 | 0.3 | 0.2 |
|  | Ketones | 16.8 | 103 | 17.7 |
|  | Cyclic compounds | 1.1 | 1.0 | 1.3 |
|  | Unknown material + coke | 15.6 | 12.6 | 24.8 |
| Yield [%] | Isobutyl aldehyde + methacrolein | 43.7 | 46.1 | 35.3 |

As shown in Table 5 and Fig. 5, the selectivities of isobutyl aldehyde and methacrolein were also high in Examples 14 to 16 in which potassium was supported and then calcined or the potassium precursor was changed in the catalyst preparation.

[Example 17]

Na (5)/$Al_2O_3$ (catalyst (abbreviation): B-13, average electronegativity: 2.49) was prepared as a catalyst for dehydrogenation by the same method as in Example 3 except that 0.50 g of sodium nitrate (manufactured by FUJIFILM Wako Pure Chemical Corporation) was used as a raw material for the alkali metal. Here, the number in parentheses indicates the ratio (% by mole) of the supported metal element to the Al atomic weight in the catalyst. In addition, the acid amount of the catalyst B-13 was 0.111 mmol/g, and the base amount was 0.373 mmol/g. The BET specific surface area was 170 $m^2$/g. The content of $Al_2O_3$ was 95% by mole.
The catalyst performance was evaluated in the same manner as in Example 8 except that 1.1 g of the catalyst B-13 was used instead of the catalyst B-3.

[Example 18]

Rb (5)/$Al_2O_3$ (catalyst (abbreviation): B-14, average electronegativity: 2.48) was prepared as a catalyst for dehydrogenation by the same method as in Example 3 except that 0.89 g of Rubidium nitrate (manufactured by FUJIFILM Wako Pure Chemical Corporation) was used as a raw material for the alkali metal. Here, the number in parentheses indicates the ratio (% by mole) of the supported metal element to the Al atomic weight in the catalyst. In addition, the acid amount in the catalyst B-14 was 0.056 mmol/g, and the base amount was 0.431 mmol/g. The BET specific surface area was 162 $m^2$/g. The content of $Al_2O_3$ was 95% by mole.
The catalyst performance was evaluated in the same manner as in Example 8 except that 1.1 g of the catalyst B-14 was used instead of the catalyst B-3.

[Example 19]

Cs (5)/$Al_2O_3$ (catalyst (abbreviation): B-15, average electronegativity: 2.48) was prepared as a catalyst for dehydrogenation by the same method as in Example 7 except that 1.1 g of Cesium nitrate (manufactured by FUJIFILM Wako Pure Chemical Corporation) was used as a raw material for the alkali metal. Here, the number in parentheses indicates the ratio (% by mole) of the supported metal element to the Al atomic weight in the catalyst. In addition, the acid amount in the catalyst B-15 was 0.046 mmol/g, and the base amount was 0.437 mmol/g. The BET specific surface area was 157 $m^2$/g. The content of $Al_2O_3$ was 95% by mole.

The catalyst performance was evaluated in the same manner as in Example 8 except that 1.1 g of the catalyst B-15 was used instead of the catalyst B-3.

Table 6 shows the reaction conditions and evaluation results in Examples 8 and 17 to 19. In addition, Fig. 6 shows the isobutanol conversion rate, and the selectivities and yields of isobutyl aldehyde and methacrolein (MAL) by the catalyst of each example.

[Table 6]

| | | Example 17 | Example 8 | Example 18 | Example 19 |
|---|---|---|---|---|---|
| Catalyst name | | Na (5)/$Al_2O_3$ | K (5)/$Al_2O_3$ | Rb (5)/$Al_2O_3$ | Cs (5)/$Al_2O_3$ |
| Catalyst (abbreviation) | | B-13 | B-3 | B-14 | B-15 |
| Supported metal | | Na | K | Rb | Cs |
| Acid amount [mmol/g] | | 0.111 | 0.079 | 0.056 | 0.046 |
| Base amount [mmol/g] | | 0.373 | 0.439 | 0.431 | 0.437 |
| Average electronegativity | | 2.49 | 2.48 | 2.48 | 2.48 |
| Reaction temperature [°C] | | 500 | 500 | 500 | 500 |
| Catalyst mass [g] | | 1.1 | 1.1 | 1.1 | 1.1 |
| W/F [$h^{-1}$] | | 0.69 | 0.70 | 0.69 | 0.69 |
| Conditions for supply amount of raw material | | c | c | c | c |
| Supply amount of raw material | Isobutanol [mL/hour] | 1.9 | 1.9 | 1.9 | 1.9 |
| | Water [mL/hour] | 1.0 | 1.0 | 1.0 | 1.0 |
| | Nitrogen [mL (standard state)/hour] | 2400 | 2400 | 2400 | 2400 |
| Composition of raw material gas [% by volume] | Isobutanol | 11.4 | 11.4 | 11.4 | 11.4 |
| | Water | 29.6 | 29.6 | 29.6 | 29.6 |
| | Nitrogen | 59.0 | 59.0 | 59.0 | 59.0 |
| Conversion rate [%] | | 51.7 | 38.4 | 36.7 | 35.4 |
| Selectivity [%] | Isobutyl aldehyde + methacrolein | 62.3 | 73.0 | 73.8 | 72.2 |
| | Isobutyl aldehyde | 62.0 | 73.0 | 73.6 | 72.0 |
| | Methacrolein | 0.2 | 0.0 | 0.2 | 0.2 |
| | Propionaldehyde | 0.3 | 0.0 | 0.4 | 0.4 |
| | Propylene | 2.7 | 2.6 | 3.2 | 3.7 |
| | C4 butenes | 23.7 | 15.9 | 8.9 | 7.1 |
| | COx | 1.3 | 0.8 | 1.6 | 2.2 |
| | Isobutyric acid | 2.2 | 1.2 | 3.2 | 3.9 |
| | Ketones | 2.6 | 1.3 | 3.7 | 4.5 |
| | Cyclic compounds | 0.6 | 0.4 | 0.5 | 0.4 |
| | Unknown material + coke | 4.2 | 4.2 | 4.7 | 5.6 |
| Yield [%] | Isobutyl aldehyde + methacrolein | 32.2 | 28.0 | 27.1 | 25.6 |

As shown in Table 6 and Fig. 6, the selectivities of isobutyl aldehyde and methacrolein were also high in Examples 8 and 17 to 19 using the catalysts B-3 and 13 to 15 in which an alkali metal other than potassium was supported.

[Example 20]

The catalyst performance was evaluated in the same manner as in Example 5 except that 1.1 g of magnesium oxide (manufactured by FUJIFILM Wako Pure Chemical Corporation, acid amount: 0.000 mmol/g, base amount: 0.030 mmol/g, BET specific surface area: 8.0 $m^2$/g, average electronegativity: 2.12) was used as a catalyst (abbreviation) A-2.

[Example 21]

The catalyst performance was evaluated in the same manner as in Example 5 except that 0.5 g of the catalyst B-13 (acid amount: 0.111 mmol/g, base amount: 0.373 mmol/g, BET specific surface area: 170 $m^2$/g, average electronegativity: 2.49) was used, the flow rate of isobutanol into a reactor was set to 0.95 mL/hour, and the flow rate of nitrogen gas was set to 1,800 mL (standard state)/hour.

[Example 22]

The catalyst performance was evaluated in the same manner as in Example 5 except that 1.1 g of the catalyst B-3 (acid amount: 0.079 mmol/g, base amount: 0.439 mmol/g, BET specific surface area: 173 $m^2$/g, average electronegativity: 2.48) was used.

[Example 23]

The catalyst performance was evaluated in the same manner as in Example 5 except that 1.1 g of the catalyst B-14 (acid amount: 0.056 mmol/g, base amount: 0.431 mmol/g, BET specific surface area: 162 $m^2$/g, average electronegativity: 2.48) was used.

[Example 24]

The catalyst performance was evaluated in the same manner as in Example 5 except that 1.1 g of the catalyst B-15 (acid amount: 0.046 mmol/g, base amount: 0.437 mmol/g, BET specific surface area: 157 $m^2$/g, average electronegativity: 2.48) was used.

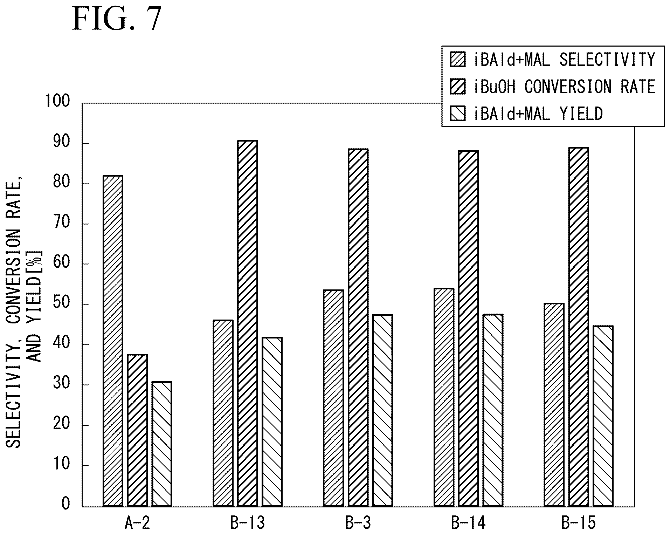

Tables 7A and 7B show reaction conditions and evaluation results in Examples 20 to 24. In addition, Fig. 7 shows the isobutanol conversion rate, and the selectivities and yields of isobutyl aldehyde and methacrolein (MAL) by the catalyst of each example.

[Table 7A]

| | | Example 20 | Example 21 | Example 22 |
|---|---|---|---|---|
| Catalyst name | | MgO | Na (5)/Al$_2$O$_3$ | K (5)/Al$_2$O$_3$ |
| Catalyst (abbreviation) | | A-2 | B-13 | B-3 |
| Acid amount [mmol/g] | | 0.000 | 0.111 | 0.079 |
| Base amount [mmol/g] | | 0.030 | 0.373 | 0.439 |
| Average electronegativity | | 2.12 | 2.49 | 2.48 |
| Reaction temperature [°C] | | 500 | 500 | 500 |
| Catalyst mass [g] | | 1.1 | 0.5 | 1.1 |
| W/F [$h^{-1}$] | | 0.69 | 0.68 | 0.67 |
| Conditions for supply amount of raw material | | b | b | b |
| Supply amount of raw material | Isobutanol [mL/hour] | 1.9 | 0.95 | 1.9 |
| | Water [mL/hour] | 0.0 | 0.0 | 0.0 |
| | Nitrogen [mL (standard state)/hour] | 3660 | 1800 | 3660 |

(continued)

|  | | Example 20 | Example 21 | Example 22 |
|---|---|---|---|---|
| Composition of raw material gas [% by volume] | Isobutanol | 11.4 | 11.4 | 11.4 |
| | Water | 0.0 | 0.0 | 0.0 |
| | Nitrogen | 88.6 | 88.6 | 88.6 |
| Conversion rate [%] | | 37.5 | 90.7 | 88.6 |
| Selectivity [%] | Isobutyl aldehyde + methacrolein | 81.9 | 46.1 | 53.5 |
| | Isobutyl aldehyde | 81.8 | 45.6 | 53.1 |
| | Methacrolein | 0.1 | 0.5 | 0.4 |
| | Propionaldehyde | 0.1 | 0.5 | 0.8 |
| | Propylene | 1.7 | 2.8 | 2.7 |
| | C4 butenes | 9.5 | 24.8 | 15.2 |
| | COx | 0.1 | 1.3 | 2.1 |
| | Isobutyric acid | 0.0 | 0.3 | 0.3 |
| | Ketones | 0.3 | 3.2 | 4.3 |
| | Cyclic compounds | 1.5 | 0.6 | 0.6 |
| | Unknown material + coke | 4.9 | 20.5 | 20.4 |
| Yield [%] | Isobutyl aldehyde + methacrolein | 30.7 | 41.8 | 47.4 |

[Table 7B]

|  | | Example 23 | Example 24 |
|---|---|---|---|
| Catalyst name | | Rb (5)/Al$_2$O$_3$ | Cs (5)/Al$_2$O$_3$ |
| Catalyst (abbreviation) | | B-14 | B-15 |
| Acid amount [mmol/g] | | 0.056 | 0.046 |
| Base amount [mmol/g] | | 0.431 | 0.437 |
| Average electronegativity | | 2.48 | 2.48 |
| Reaction temperature [°C] | | 500 | 500 |
| Catalyst mass [g] | | 1.1 | 1.1 |
| W/F [h$^{-1}$] | | 0.68 | 0.68 |
| Conditions for supply amount of raw material | | b | b |
| Supply amount of raw material | Isobutanol [mL/hour] | 1.9 | 1.9 |
| | Water [mL/hour] | 0.0 | 0.0 |
| | Nitrogen [mL (standard state)/hour] | 3660 | 3660 |
| Composition of raw material gas [% by volume] | Isobutanol | 11.4 | 11.4 |
| | Water | 0.0 | 0.0 |
| | Nitrogen | 88.6 | 88.6 |
| Conversion rate [%] | | 88.1 | 88.9 |

(continued)

|  |  | Example 23 | Example 24 |
|---|---|---|---|
| Selectivity [%] | Isobutyl aldehyde + methacrolein | 53.9 | 50.2 |
|  | Isobutyl aldehyde | 53.6 | 49.7 |
|  | Methacrolein | 0.4 | 0.5 |
|  | Propionaldehyde | 0.7 | 0.7 |
|  | Propylene | 2.6 | 2.8 |
|  | C4 butenes | 13.5 | 10.8 |
|  | COx | 2.7 | 3.3 |
|  | Isobutyric acid | 0.3 | 0.2 |
|  | Ketones | 5.5 | 6.6 |
|  | Cyclic compounds | 0.6 | 0.6 |
|  | Unknown material + coke | 20.2 | 24.8 |
| Yield [%] | Isobutyl aldehyde + methacrolein | 47.5 | 44.6 |

As shown in Table 7A, Table 7B, and Fig. 7, in a case where catalysts A-2, B-3, 14, and 15 having an average electronegativity within a range of 2.1 to 2.8 are used, the selectivities of isobutyl aldehyde and methacrolein are high without carrying out a hydrogen reduction.

[Example 25]

The catalyst performance was evaluated in the same manner as in Example 1 except for the following points. The inside of a reaction tube was filled with 1.1 g of the catalyst A-2 (acid amount: 0.000 mmol/g, base amount: 0.030 mmol/g, BET specific surface area: 8.0 $m^2$/g, average electronegativity: 2.12) to form a catalyst layer. Without supplying water to the reaction tube, the flow rate of isobutanol (manufactured by FUJIFILM Wako Pure Chemical Corporation, specific gravity: 0.80 g/mL) was set to 1.9 mL/hour, and isobutanol was supplied into a vaporizer set at 150°C and vaporized. The flow rate of nitrogen gas was 1,080 mL (standard state)/hour. The isobutanol concentration in the raw material gas supplied to the catalyst layer was 29.6% by volume, and the temperature (the reaction temperature) of the catalyst layer during the reaction was 500°C.

[Example 26]

The catalyst performance was evaluated in the same manner as in Example 25 except that 1.1 g of cerium oxide (manufactured by FUJIFILM Wako Pure Chemical Corporation, acid amount: 0.004 mmol/g, base amount: 0.004 mmol/g, BET specific surface area: 5.0 $m^2$/g, average electronegativity: 2.37) was used as a catalyst A-3.

[Example 27]

The catalyst performance was evaluated in the same manner as in Example 25 except that 1.1 g of the catalyst B-3 (acid amount: 0.079 mmol/g, base amount: 0.439 mmol/g, average electronegativity: 2.48) was used.

[Example 28]

The catalyst performance was evaluated in the same manner as in Example 25 except that 1.1 g of zinc oxide (manufactured by FUJIFILM Wako Pure Chemical Corporation, acid amount: 0.004 mmol/g, base amount: 0.003 mmol/g, BET specific surface area: 7.0 $m^2$/g, average electronegativity: 2.38) was used as a catalyst A-4.

[Example 29]

The catalyst performance was evaluated in the same manner as in Example 25 except that 1.1 g of silver oxide (manufactured by FUJIFILM Wako Pure Chemical Corporation, average electronegativity: 2.34) was used as a catalyst A-5.

[Example 30]

The catalyst performance was evaluated in the same manner as in Example 25 except that 1.1 g of hydrotalcite (manufactured by FUJIFILM Wako Pure Chemical Corporation, acid amount: 0.242 mmol/g, base amount: 0.172 mmol/g, BET specific surface area: 197 $m^2$/g, average electronegativity: 2.49) was used as a catalyst A-6.

[Example 31]

The catalyst performance was evaluated in the same manner as in Example 25 except that 1.1 g of lanthanum oxide (manufactured by FUJIFILM Wako Pure Chemical Corporation, acid amount: 0.000 mmol/g, base amount: 0.006 mmol/g, average electronegativity: 2.18) was used as a catalyst A-7.

[Comparative Example 1]

The catalyst performance was evaluated in the same manner as in Example 25 except that 1.1 g of silicon oxide (manufactured by FUJI SILYSIA CHEMICAL LTD., product name: CARiACT Q-10, acid amount: 0.008 mmol/g, average electronegativity: 2.82) was used.

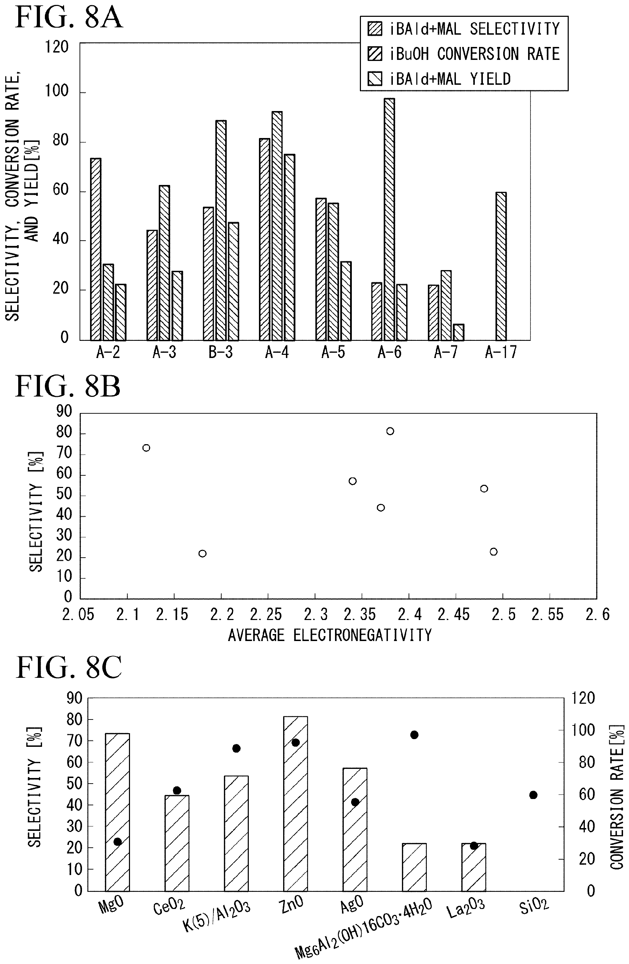

Tables 8A to 8C show reaction conditions and evaluation results in Examples 25 to 31 and Comparative Example 1. In addition, Fig. 8 shows the isobutanol conversion rate, and the selectivities and yields of isobutyl aldehyde and methacrolein (MAL) by the catalyst of each example.

[Table 8A]

| | | Example 25 | Example 26 | Example 27 |
|---|---|---|---|---|
| Catalyst name | | MgO | CeO₂ | K (5)/Al₂O₃ |
| Catalyst (abbreviation) | | A-2 | A-3 | B-3 |
| Acid amount [mmol/g] | | 0.000 | 0.004 | 0.079 |
| Average electronegativity | | 2.12 | 2.37 | 2.48 |
| Reaction temperature [°C] | | 500 | 500 | 500 |
| Catalyst mass [g] | | 1.1 | 1.1 | 1.1 |
| W/F [$h^{-1}$] | | 0.70 | 0.69 | 0.67 |
| Conditions for supply amount of raw material | | d | d | d |
| Supply amount of raw material | Isobutanol [mL/hour] | 1.9 | 1.9 | 1.9 |
| | Water [mL/hour] | 0.0 | 0.0 | 0.0 |
| | Nitrogen [mL (standard state)/hour] | 1080 | 1080 | 1080 |
| Composition of raw material gas [% by volume] | Isobutanol | 29.6 | 29.6 | 29.6 |
| | Water | 0.0 | 0.0 | 0.0 |
| | Nitrogen | 70.4 | 70.4 | 70.4 |
| Conversion rate [%] | | 30.4 | 62.3 | 88.6 |

(continued)

| | | Example 25 | Example 26 | Example 27 |
|---|---|---|---|---|
| Selectivity [%] | Isobutyl aldehyde + methacrolein | 73.4 | 44.3 | 53.5 |
| | Isobutyl aldehyde | 73.3 | 44.1 | 53.1 |
| | Methacrolein | 0.1 | 0.2 | 0.4 |
| | Propionaldehyde | 0.1 | 0.0 | 0.8 |
| | Propylene | 0.6 | 4.8 | 2.7 |
| | C4 butenes | 3.3 | 3.8 | 15.2 |
| | COx | 0.0 | 4.1 | 2.1 |
| | Isobutyric acid | 0.0 | 0.3 | 0.3 |
| | Ketones | 0.1 | 28.1 | 4.3 |
| | Cyclic compounds | 1.0 | 0.8 | 0.6 |
| | Unknown material + coke | 21.5 | 13.7 | 20.4 |
| Yield [%] | Isobutyl aldehyde + methacrolein | 22.3 | 27.6 | 47.4 |

[Table 8B]

| | | Example 28 | Example 29 | Example 30 |
|---|---|---|---|---|
| Catalyst name | | ZnO | AgO | $Mg_6Al_2(OH)16CO_3 \cdot 4H_2O$ |
| Catalyst (abbreviation) | | A-4 | A-5 | A-6 |
| Acid amount [mmol/g] | | 0.004 | 0.000 | 0.242 |
| Average electronegativity | | 2.38 | 2.34 | 0.00 |
| Reaction temperature [°C] | | 500 | 500 | 500 |
| Catalyst mass [g] | | 1.1 | 1.1 | 1.1 |
| W/F [$h^{-1}$] | | 0.70 | 0.71 | 0.71 |
| Conditions for supply amount of raw material | | d | d | d |
| Supply amount of raw material | Isobutanol [mL/hour] | 1.9 | 1.9 | 1.9 |
| | Water [mL/hour] | 0.0 | 0.0 | 0.0 |
| | Nitrogen [mL (standard state)/hour] | 1080 | 18 | 1080 |
| Composition of raw material gas [% by volume] | Isobutanol | 29.6 | 29.6 | 29.6 |
| | Water | 0.0 | 0.0 | 0.0 |
| | Nitrogen | 70.4 | 70.4 | 70.4 |
| Conversion rate [%] | | 92.2 | 55.1 | 97.1 |

(continued)

|  |  | Example 28 | Example 29 | Example 30 |
|---|---|---|---|---|
| Selectivity [%] | Isobutyl aldehyde + methacrolein | 81.3 | 57.1 | 22.8 |
|  | Isobutyl aldehyde | 80.1 | 56.6 | 22.6 |
|  | Methacrolein | 1.2 | 0.5 | 0.2 |
|  | Propionaldehyde | 0.1 | 0.0 | 0.3 |
|  | Propylene | 0.3 | 0.5 | 2.9 |
|  | C4 butenes | 0.8 | 0.4 | 35.0 |
|  | COx | 0.4 | 0.0 | 2.0 |
|  | Isobutyric acid | 0.8 | 0.0 | 0.4 |
|  | Ketones | 0.2 | 0.0 | 2.3 |
|  | Cyclic compounds | 0.4 | 1.2 | 0.3 |
|  | Unknown material + coke | 15.7 | 40.8 | 34.0 |
| Yield [%] | Isobutyl aldehyde + methacrolein | 75.0 | 31.5 | 22.1 |

[Table 8C]

|  |  | Example 31 | Comparative Example 1 |
|---|---|---|---|
| Catalyst name | | $La_2O_3$ | $SiO_2$ |
| Catalyst (abbreviation) | | A-7 | A-17 |
| Acid amount [mmol/g] | | 0.000 | 0.008 |
| Average electronegativity | | 2.18 | 2.82 |
| Reaction temperature [°C] | | 500 | 500 |
| Catalyst mass [g] | | 1.1 | 1.1 |
| W/F [$h^{-1}$] | | 0.71 | 0.70 |
| Conditions for supply amount of raw material | | d | d |
| Supply amount of raw material | Isobutanol [mL/hour] | 1.9 | 1.9 |
|  | Water [mL/hour] | 0.0 | 0.0 |
|  | Nitrogen [mL (standard state)/ hour] | 18 | 1080 |
| Composition of raw material gas [% by volume] | Isobutanol | 29.6 | 29.6 |
|  | Water | 0.0 | 0.0 |
|  | Nitrogen | 70.4 | 70.4 |
| Conversion rate [%] | | 27.8 | 59.6 |

(continued)

|  |  | Example 31 | Comparative Example 1 |
|---|---|---|---|
| Selectivity [%] | Isobutyl aldehyde + methacrolein | 22.0 | 0.0 |
|  | Isobutyl aldehyde | 21.9 | 0.0 |
|  | Methacrolein | 0.1 | 0.0 |
|  | Propionaldehyde | 0.0 | 0.0 |
|  | Propylene | 0.2 | 6.1 |
|  | C4 butenes | 14.6 | 45.8 |
|  | COx | 3.0 | 0.1 |
|  | Isobutyric acid | 0.1 | 0.0 |
|  | Ketones | 19.8 | 0.0 |
|  | Cyclic compounds | 0.3 | 0.0 |
|  | Unknown material + coke | 40.0 | 47.9 |
| Yield [%] | Isobutyl aldehyde + methacrolein | 6.1 | 0.0 |

As shown in Tables 8A to 8C and Fig. 8, in Examples 25 to 31 using catalysts having an average electronegativity in a range of 2.1 to 2.8, the selectivities of isobutyl aldehyde and methacrolein were high as compared with the catalysts having the average electronegativity outside the above range.

[Examples 33 to 36]

The catalyst performance was evaluated in the same manner as in Example 8 except that the catalyst B-1 was used and the reaction temperature was changed as shown in Table 9.

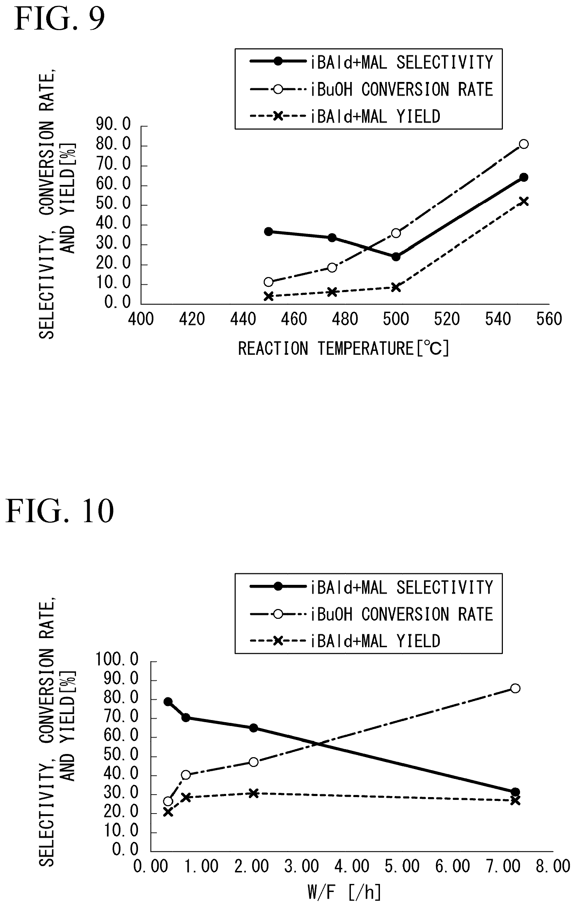

Table 9 shows the reaction conditions and evaluation results in Examples 33 to 36. In addition, Fig. 9 shows the isobutanol conversion rate, and the selectivities and yields of isobutyl aldehyde and methacrolein (MAL) by the catalyst of each example.

[Table 9]

|  | Example 33 | Example 34 | Example 35 | Example 36 |
|---|---|---|---|---|
| Catalyst name | K (5)/ZrO$_2$ (6.7)-FeOx (88.3) | K (5)/ZrO$_2$ (6.7)-FeOx (88.3) | K (5)/ZrO$_2$ (6.7)-FeOx (88.3) | K (5)/ZrO$_2$ (6.7)-FeOx (88.3) |
| Catalyst (abbreviation) | B-1 | B-1 | B-1 | B-1 |
| Average electronegativity | 2.55 | 2.55 | 2.55 | 2.55 |
| Reaction temperature [°C] | 450 | 475 | 500 | 550 |
| Catalyst mass [g] | 1.1 | 1.1 | 1.1 | 1.1 |
| W/F [h$^{-1}$] | 0.70 | 0.70 | 0.70 | 0.70 |
| Conditions for supply amount of raw material | c | c | c | c |

(continued)

|  |  | Example 33 | Example 34 | Example 35 | Example 36 |
|---|---|---|---|---|---|
| Supply amount of raw material | Isobutanol [mL/hour] | 1.90 | 1.90 | 1.90 | 1.90 |
|  | Water [mL/hour] | 1.00 | 1.00 | 1.00 | 1.00 |
|  | Nitrogen [mL (standard state)/hour] | 2400 | 2400 | 2400 | 2400 |
| Composition of raw material gas [% by volume] | Isobutanol | 11.4 | 11.4 | 11.4 | 11.4 |
|  | Water | 30.6 | 30.6 | 30.6 | 30.6 |
|  | Nitrogen | 58.0 | 58.0 | 58.0 | 58.0 |
| Conversion rate [%] |  | 11.3 | 18.6 | 35.8 | 81.0 |
| Selectivity [%] | Isobutyl aldehyde + methacrolein | 36.7 | 33.6 | 24.1 | 64.3 |
|  | Isobutyl aldehyde | 36.5 | 33.3 | 23.7 | 55.6 |
|  | Methacrolein | 0.18 | 0.26 | 0.40 | 8.70 |
|  | Propionaldehyde | 0.3 | 0.2 | 0.2 | 1.2 |
|  | Propylene | 7.8 | 7.7 | 9.1 | 5.8 |
|  | C4 butenes | 0.9 | 1.7 | 1.2 | 0.8 |
|  | COx | 26.0 | 24.2 | 27.1 | 13.4 |
|  | Isobutyric acid | 2.7 | 1.3 | 1.0 | 0.3 |
|  | Ketones | 5.9 | 4.7 | 3.8 | 0.1 |
|  | Cyclic compounds | 1.6 | 1.1 | 2.1 | 1.9 |
|  | Coke | 11.3 | 10.4 | 9.1 | 5.6 |
|  | Unknown material | 6.8 | 15.2 | 22.4 | 6.6 |
| Yield [%] | Isobutyl aldehyde + methacrolein | 4.1 | 6.2 | 8.6 | 52.1 |

As shown in Table 9 and Fig. 9, in Examples 33 to 36 using the catalyst B-1, the selectivities of isobutyl aldehyde and methacrolein were high at any reaction temperature.

[Examples 37 to 39]

The catalyst performance was evaluated in the same manner as in Example 8 except that the using amount of the catalyst B-3 was changed as shown in Table 10.

[Example 40]

The catalyst performance was evaluated in the same manner as in Example 37 except that 3.2 g of the catalyst B-3 was used, the flow rates of isobutanol and water were each set to 0.55 mL/hour and 0.30 mL/hour, supplied into a vaporizer set at 150°C, and vaporized, the flow rate of nitrogen gas was set to 720 mL (standard state)/hour, and the isobutanol concentration and the concentration of water in the raw material gas supplied to the catalyst layer were each set to 10.9% by volume and 30.4% by volume.

Table 10 shows the reaction conditions and evaluation results in Examples 37 to 40. In addition, Fig. 10 shows the isobutanol conversion rate, and the selectivities and yields of isobutyl aldehyde and methacrolein (MAL) by the

31

catalyst of each example.

[Table 10]

| | | Example 37 | Example 38 | Example 39 | Example 40 |
|---|---|---|---|---|---|
| Catalyst name | | K (5)/$Al_2O_3$ | K (5)/$Al_2O_3$ | K (5)/$Al_2O_3$ | K (5)/$Al_2O_3$ |
| Catalyst (abbreviation) | | B-3 | B-3 | B-3 | B-3 |
| Average electronegativity | | 2.48 | 2.48 | 2.48 | 2.48 |
| Reaction temperature [°C] | | 500 | 500 | 500 | 500 |
| Catalyst mass [g] | | 0.5 | 1.1 | 3.1 | 3.2 |
| W/F [$h^{-1}$] | | 0.34 | 0.69 | 2.04 | 7.25 |
| Conditions for supply amount of raw material | | c | c | c | c |
| Supply amount of raw material | Isobutanol [mL/hour] | 1.90 | 1.90 | 1.90 | 0.55 |
| | Water [mL/hour] | 1.00 | 1.00 | 1.00 | 0.30 |
| | Nitrogen [mL (standard state)/hour] | 2400 | 2400 | 2400 | 720 |
| Composition of raw material gas [% by volume] | Isobutanol | 11.4 | 11.4 | 11.4 | 10.9 |
| | Water | 30.6 | 30.6 | 30.6 | 30.4 |
| | Nitrogen | 58.0 | 58.0 | 58.0 | 58.7 |
| Conversion rate [%] | | 26.5 | 40.4 | 47.2 | 85.9 |
| Selectivity [%] | Isobutyl aldehyde + methacrolein | 78.8 | 70.5 | 65.0 | 31.3 |
| | Isobutyl aldehyde | 78.6 | 70.2 | 64.8 | 31.2 |
| | Methacrolein | 0.20 | 0.24 | 0.20 | 0.12 |
| | Propionaldehyde | 0.3 | 0.3 | 0.4 | 0.2 |
| | Propylene | 1.7 | 3.1 | 3.8 | 8.0 |
| | C4 butenes | 7.4 | 9.1 | 10.2 | 12.4 |
| | COx | 0.9 | 2.1 | 2.7 | 7.6 |
| | Isobutyric acid | 2.8 | 2.8 | 2.7 | 3.3 |
| | Ketones | 2.3 | 4.8 | 4.9 | 9.5 |
| | Cyclic compounds | 0.6 | 0.6 | 0.6 | 0.4 |
| | Unknown material + coke | 5.1 | 6.6 | 9.7 | 27.2 |
| Yield [%] | Isobutyl aldehyde + methacrolein | 20.9 | 28.5 | 30.6 | 26.9 |

As shown in Table 10 and Fig. 10, in Examples 37 to 40 using the catalyst B-3, the selectivities of isobutyl aldehyde and methacrolein were also high in any W/F conditions.

[Example 41]

The catalyst performance was evaluated in the same manner as in Example 25 except that 2.2 g of the catalyst A-2 was used.

[Example 42]

The catalyst performance was evaluated in the same manner as in Example 1 except for the following points. The inside of a reaction tube was filled with 3.3 g of the catalyst A-2 to form a catalyst layer. Without supplying water to the reaction tube, the flow rate of isobutanol was set to 0.92 mL/hour, and isobutanol was supplied into a vaporizer set at 150°C and vaporized. The flow rate of nitrogen gas was 540 mL (standard state)/hour. The isobutanol concentration in the raw material gas supplied to the catalyst layer was 29.6% by volume, and the temperature (the reaction temperature) of the catalyst layer during the reaction was 500°C.

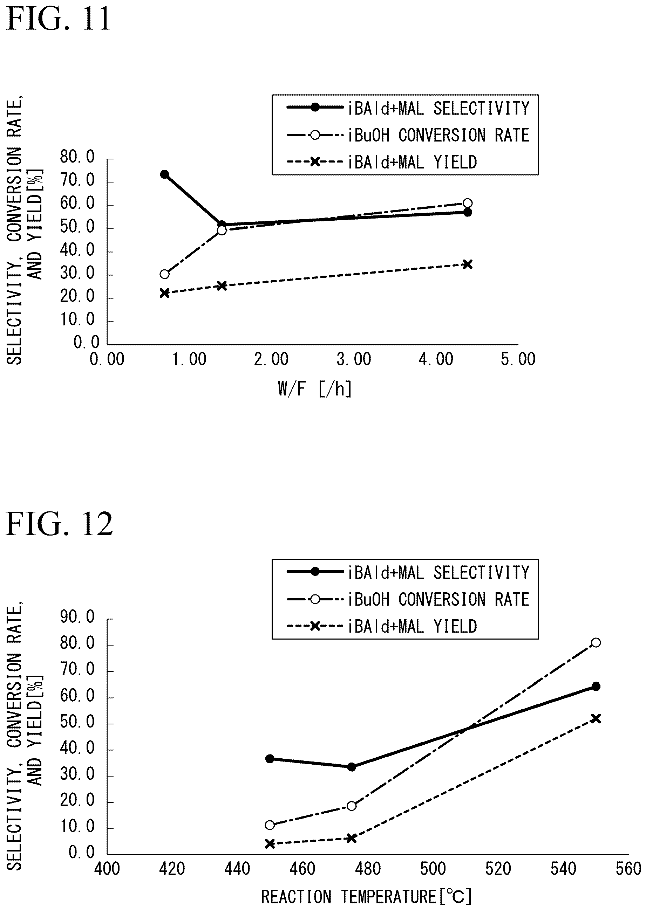

Table 11 shows the reaction conditions and evaluation results in Examples 25, 41, and 42. In addition, Fig. 11 shows the isobutanol conversion rate, and the selectivities and yields of isobutyl aldehyde and methacrolein (MAL) by the catalyst of each example.

[Table 11]

| | | Example 25 | Example 41 | Example 42 |
|---|---|---|---|---|
| Catalyst name | | MgO | MgO | MgO |
| Catalyst (abbreviation) | | A-2 | A-2 | A-2 |
| Average electronegativity | | 2.12 | 2.12 | 2.12 |
| Reaction temperature [°C] | | 500 | 500 | 500 |
| Catalyst mass [g] | | 1.1 | 2.2 | 3.3 |
| W/F [$h^{-1}$] | | 0.70 | 1.40 | 4.39 |
| Conditions for supply amount of raw material | | d | d | d |
| Supply amount of raw material | Isobutanol [mL/hour] | 1.90 | 1.90 | 0.92 |
| | Water [mL/hour] | 0.0 | 0.0 | 0.0 |
| | Nitrogen [mL (standard state)/hour] | 1080 | 1080 | 540 |
| Composition of raw material gas [% by volume] | Isobutanol | 29.6 | 29.6 | 29.6 |
| | Water | 0.0 | 0.0 | 0.0 |
| | Nitrogen | 70.4 | 70.4 | 70.4 |
| Conversion rate [%] | | 30.4 | 49.3 | 61.0 |
| Selectivity [%] | Isobutyl aldehyde + methacrolein | 73.4 | 51.6 | 57.0 |
| | Isobutyl aldehyde | 73.3 | 51.5 | 56.9 |
| | Methacrolein | 0.05 | 0.05 | 0.09 |
| | Propionaldehyde | 0.1 | 0.1 | 0.2 |
| | Propylene | 0.6 | 0.5 | 1.1 |
| | C4 butenes | 3.3 | 3.0 | 5.4 |
| | COx | 0.0 | 0.0 | 0.3 |
| | Isobutyric acid | 0.0 | 0.0 | 0.0 |
| | Ketones | 0.1 | 0.1 | 0.9 |
| | Cyclic compounds | 1.0 | 0.9 | 0.9 |
| | Unknown material + coke | 21.5 | 43.7 | 34.2 |
| Yield [%] | Isobutyl aldehyde + methacrolein | 22.3 | 25.4 | 34.8 |

As shown in Table 11 and Fig. 11, in Examples 25, 41, and 42 using the catalyst A-2, the selectivities of isobutyl aldehyde and methacrolein were also high in any W/F conditions.

[Examples 43 and 44]

The catalyst performance was evaluated in the same manner as in Example 1 except that the reaction temperature was changed as shown in Table 12.

Table 12 shows the reaction conditions and evaluation results in Examples 1, 43, and 44. In addition, Fig. 12 shows the isobutanol conversion rate, and the selectivities and yields of isobutyl aldehyde and methacrolein (MAL) by the catalyst of each example.

[Table 12]

| | | Example 1 | Example 43 | Example 44 |
|---|---|---|---|---|
| Catalyst name | | K (5)/ZrO$_2$ (6.7)-FeOx (88.3) | K (5)/ZrO$_2$ (6.7)-FeOx (88.3) | K (5)/ZrO$_2$ (6.7)-FeOx (88.3) |
| Catalyst (abbreviation) | | B-1 | B-1 | B-1 |
| Average electronegativity | | 2.55 | 2.55 | 2.55 |
| Reaction temperature [°C] | | 450 | 475 | 550 |
| Catalyst mass [g] | | 1.1 | 1.1 | 1.1 |
| W/F [h$^{-1}$] | | 0.70 | 0.70 | 0.70 |
| Conditions for supply amount of raw material | | a | a | a |
| Supply amount of raw material | Isobutanol [mL/hour] | 1.9 | 1.9 | 1.9 |
| | Water [mL/hour] | 2.0 | 2.0 | 2.0 |
| | Nitrogen [mL (standard state)/ hour] | 1140 | 1140 | 1140 |
| Composition of raw material gas [% by volume] | Isobutanol | 11.4 | 11.4 | 11.4 |
| | Water | 60.2 | 60.2 | 60.2 |
| | Nitrogen | 28.4 | 28.4 | 28.4 |
| Conversion rate [%] | | 11.3 | 18.6 | 81.0 |
| Selectivity [%] | Isobutyl aldehyde + methacrolein | 36.7 | 33.6 | 64.3 |
| | Isobutyl aldehyde | 36.5 | 33.3 | 55.6 |
| | Methacrolein | 0.2 | 0.3 | 8.7 |
| | Propionaldehyde | 0.3 | 0.2 | 1.2 |
| | Propylene | 7.8 | 7.7 | 5.8 |
| | C4 butenes | 0.9 | 1.7 | 0.8 |
| | COx | 26.0 | 24.2 | 13.4 |
| | Isobutyric acid | 2.7 | 1.3 | 0.3 |
| | Ketones | 5.9 | 4.7 | 0.1 |
| | Cyclic compounds | 1.6 | 1.1 | 1.9 |
| | Coke | 11.3 | 10.4 | 5.6 |
| | Unknown material | 6.8 | 15.2 | 6.6 |
| Yield [%] | Isobutyl aldehyde + methacrolein | 4.1 | 6.2 | 52.1 |

As shown in Table 12 and Fig. 12, in Examples 1, 43, and 44 using the catalyst B-1, the selectivities of isobutyl aldehyde and methacrolein were high at any reaction temperature.

[Example 45]

The catalyst performance was evaluated in the same manner as in Example 3 except that the reaction temperature was changed as shown in Table 13.

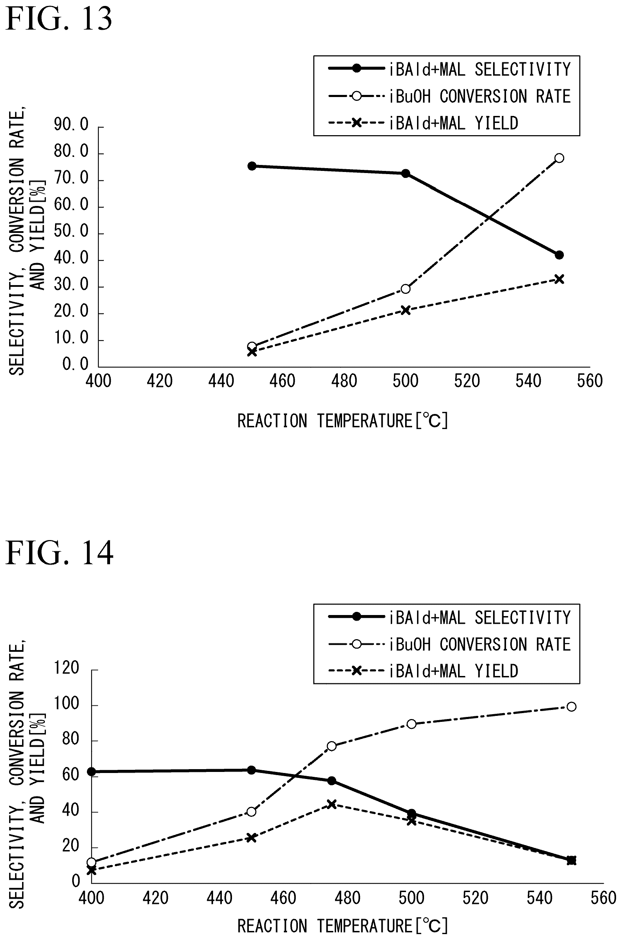

Table 13 shows reaction conditions and evaluation results in Examples 3, 13, and 45. In addition, Fig. 13 shows the isobutanol conversion rate, and the selectivities and yields of isobutyl aldehyde and methacrolein (MAL) by the catalyst of each example.

[Table 13]

| | | Example 3 | Example 13 | Example 45 |
|---|---|---|---|---|
| Catalyst name | | K (5)/Al$_2$O$_3$ | K (5)/Al$_2$O$_3$ | K (5)/Al$_2$O$_3$ |
| Catalyst (abbreviation) | | B-3 | B-3 | B-3 |
| Average electronegativity | | 2.48 | 2.48 | 2.48 |
| Reaction temperature [°C] | | 450 | 500 | 550 |
| Catalyst mass [g] | | 1.1 | 1.1 | 1.1 |
| W/F [h$^{-1}$] | | 0.70 | 0.70 | 0.70 |
| Conditions for supply amount of raw material | | a | a | a |
| Supply amount of raw material | Isobutanol [mL/hour] | 1.9 | 1.9 | 1.9 |
| | Water [mL/hour] | 2.0 | 2.0 | 2.0 |
| | Nitrogen [mL (standard state)/hour] | 1140 | 1140 | 1140 |
| Composition of raw material gas [% by volume] | Isobutanol | 11.4 | 11.4 | 11.4 |
| | Water | 60.2 | 60.2 | 60.2 |
| | Nitrogen | 28.4 | 28.4 | 28.4 |
| Conversion rate [%] | | 7.7 | 29.4 | 78.4 |
| Selectivity [%] | Isobutyl aldehyde + methacrolein | 75.5 | 72.7 | 42.1 |
| | Isobutyl aldehyde | 75.4 | 72.5 | 41.8 |
| | Methacrolein | 0.0 | 0.2 | 0.3 |
| | Propionaldehyde | 0.0 | 0.4 | 0.5 |
| | Propylene | 0.6 | 2.3 | 9.6 |
| | C4 butenes | 14.5 | 17.4 | 17.5 |
| | COx | 0.2 | 0.7 | 5.2 |
| | Isobutyric acid | 0.2 | 1.2 | 1.0 |
| | Ketones | 0.0 | 0.8 | 3.8 |
| | Cyclic compounds | 0.5 | 0.3 | 0.8 |
| | Coke | 5.5 | 1.5 | 0.3 |
| | Unknown material | 2.9 | 2.6 | 19.3 |
| Yield [%] | Isobutyl aldehyde + methacrolein | 5.8 | 21.3 | 33.0 |

As shown in Table 13 and Fig. 13, in Examples 3, 13, and 45 using the catalyst C, the selectivities of isobutyl aldehyde and methacrolein were also high at any reaction temperature.

[Examples 46 to 50]

The catalyst performance was evaluated in the same manner as in Example 25 except that 1.1 g of the catalyst B-10 was used instead of the catalyst A-2 and the reaction temperature was changed as shown in Table 14.

Tables 14A and 14B show reaction conditions and evaluation results in Examples 46 to 50. In addition, Fig. 14 shows the isobutanol conversion rate, and the selectivities and yields of isobutyl aldehyde and methacrolein (MAL) by the catalyst of each example.

[Table 14A]

| | | Example 46 | Example 47 | Example 48 |
|---|---|---|---|---|
| Catalyst name | | K (7.5)/Al$_2$O$_3$ | K (7.5)/Al$_2$O$_3$ | K (7.5)/Al$_2$O$_3$ |
| Catalyst (abbreviation) | | B-10 | B-10 | B-10 |
| Average electronegativity | | 2.47 | 2.47 | 2.47 |
| Reaction temperature [°C] | | 400 | 450 | 475 |
| Catalyst mass [g] | | 1.1 | 1.1 | 1.1 |
| W/F [h$^{-1}$] | | 0.71 | 0.70 | 0.69 |
| Conditions for supply amount of raw material | | d | a | d |
| Supply amount of raw material | Isobutanol [mL/hour] | 1.9 | 1.9 | 1.9 |
| | Water [mL/hour] | 0.0 | 0.0 | 0.0 |
| | Nitrogen [mL (standard state)/hour] | 1080 | 1080 | 1080 |
| Composition of raw material gas [% by volume] | Isobutanol | 29.6 | 29.6 | 29.6 |
| | Water | 0.0 | 0.0 | 0.0 |
| | Nitrogen | 70.4 | 70.4 | 70.4 |
| Conversion rate [%] | | 11.8 | 40.3 | 77.1 |
| Selectivity [%] | Isobutyl aldehyde + methacrolein | 62.9 | 63.8 | 57.8 |
| | Isobutyl aldehyde | 62.9 | 63.7 | 57.5 |
| | Methacrolein | 0.0 | 0.1 | 0.3 |
| | Propionaldehyde | 0.0 | 0.5 | 0.7 |
| | Propylene | 0.4 | 1.1 | 2.1 |
| | C4 butenes | 10.0 | 6.1 | 5.1 |
| | COx | 0.2 | 0.4 | 1.9 |
| | Isobutyric acid | 0.0 | 0.1 | 0.7 |
| | Ketones | 0.2 | 1.8 | 7.7 |
| | Cyclic compounds | 0.7 | 1.9 | 0.8 |
| | Unknown material + coke | 25.6 | 24.6 | 23.1 |
| Yield [%] | Isobutyl aldehyde + methacrolein | 7.4 | 25.7 | 44.6 |

[Table 14B]

| | | Example 49 | Example 50 |
|---|---|---|---|
| Catalyst name | | K (7.5)/Al$_2$O$_3$ | K (7.5)/Al$_2$O$_3$ |
| Catalyst (abbreviation) | | B-10 | B-10 |
| Average electronegativity | | 2.47 | 2.47 |
| Reaction temperature [°C] | | 500 | 550 |
| Catalyst mass [g] | | 1.1 | 1.1 |
| W/F [h$^{-1}$] | | 0.70 | 0.69 |
| Conditions for supply amount of raw material | | d | d |
| Supply amount of raw material | Isobutanol [mL/hour] | 1.9 | 1.9 |
| | Water [mL/hour] | 0.0 | 0.0 |
| | Nitrogen [mL (standard state)/hour] | 1080 | 1080 |
| Composition of raw material gas [% by volume] | Isobutanol | 29.6 | 29.6 |
| | Water | 0.0 | 0.0 |
| | Nitrogen | 70.4 | 70.4 |
| Conversion rate [%] | | 89.6 | 99.3 |
| Selectivity [%] | Isobutyl aldehyde + methacrolein | 39.4 | 13.0 |
| | Isobutyl aldehyde | 39.0 | 11.9 |
| | Methacrolein | 0.4 | 1.1 |
| | Propionaldehyde | 0.5 | 0.7 |
| | Propylene | 3.6 | 8.8 |
| | C4 butenes | 6.8 | 10.5 |
| | COx | 5.6 | 11.4 |
| | Isobutyric acid | 0.2 | 0.2 |
| | Ketones | 17.7 | 4.2 |
| | Cyclic compounds | 1.3 | 2.0 |
| | Unknown material + coke | 24.8 | 49.1 |
| Yield [%] | Isobutyl aldehyde + methacrolein | 35.3 | 12.9 |

As shown in Tables 14A and 14B and Fig. 14, in Examples 46 to 50 using the catalyst N, the selectivities of isobutyl aldehyde and methacrolein were also high at any reaction temperature.

[Examples 51 to 53]

The catalyst performance was evaluated in the same manner as in Example 28 except that the reaction temperature was changed as shown in Table 15.

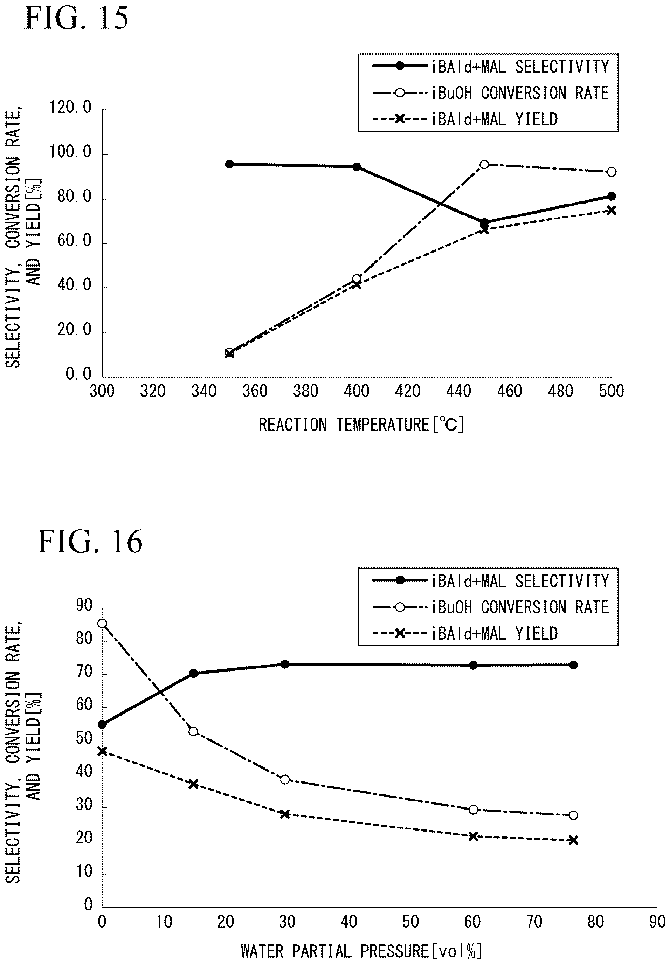

Table 15 shows the reaction conditions and evaluation results in Examples 28 and 51 to 53. In addition, Fig. 15 shows the isobutanol conversion rate, and the selectivities and yields of isobutyl aldehyde and methacrolein (MAL) by the catalyst of each example.

[Table 15]

| | Example 51 | Example 52 | Example 53 | Example 28 |
|---|---|---|---|---|
| Catalyst name | ZnO | ZnO | ZnO | ZnO |
| Catalyst (abbreviation) | A-4 | A-4 | A-4 | A-4 |

(continued)

|  |  | Example 51 | Example 52 | Example 53 | Example 28 |
|---|---|---|---|---|---|
| Average electronegativity | | 2.38 | 2.38 | 2.38 | 2.38 |
| Reaction temperature [°C] | | 350 | 400 | 450 | 500 |
| Catalyst mass [g] | | 1.1 | 1.1 | 1.1 | 1.1 |
| W/F [h$^{-1}$] | | 0.68 | 0.70 | 0.69 | 0.70 |
| Conditions for supply amount of raw material | | d | d | a | d |
| Supply amount of raw material | Isobutanol [mL/hour] | 1.9 | 1.9 | 1.9 | 1.9 |
| | Water [mL/hour] | 0.0 | 0.0 | 0.0 | 0.0 |
| | Nitrogen [mL (standard state)/ hour] | 1080 | 1080 | 1080 | 1080 |
| Composition of raw material gas [% by volume] | Isobutanol | 29.6 | 29.6 | 29.6 | 29.6 |
| | Water | 0.0 | 0.0 | 0.0 | 0.0 |
| | Nitrogen | 70.4 | 70.4 | 70.4 | 70.4 |
| Conversion rate [%] | | 10.9 | 43.9 | 95.6 | 92.2 |
| Selectivity [%] | Isobutyl aldehyde + methacrolein | 95.7 | 94.6 | 69.3 | 81.3 |
| | Isobutyl aldehyde | 95.7 | 94.5 | 68.1 | 80.1 |
| | Methacrolein | 0.1 | 0.1 | 1.2 | 1.2 |
| | Propionaldehyde | 0.0 | 0.0 | 0.0 | 0.1 |
| | Propylene | 0.0 | 0.0 | 0.1 | 0.3 |
| | C4 butenes | 0.4 | 1.5 | 1.0 | 0.8 |
| | COx | 0.0 | 0.0 | 0.2 | 0.4 |
| | Isobutyric acid | 0.3 | 1.2 | 0.5 | 0.8 |
| | Ketones | 0.2 | 0.2 | 0.6 | 0.2 |
| | Cyclic compounds | 1.1 | 0.3 | 0.4 | 0.4 |
| | Unknown material + coke | 2.2 | 2.2 | 27.8 | 15.7 |
| Yield [%] | Isobutyl aldehyde + methacrolein | 10.5 | 41.5 | 66.3 | 75.0 |

As shown in Table 15 and Fig. 15, in Examples 28 and 51 to 53 using the catalyst A-4, the selectivities of isobutyl aldehyde and methacrolein were also high at any reaction temperature.

[Example 54]

The catalyst performance was evaluated in the same manner as in Example 5 except for the following points. The inside of a reaction tube was filled with 1.1 g of the catalyst B-3 to form a catalyst layer. The flow rates of isobutanol and water were each set to 1.9 mL/hour and 0.50 mL/hour, and isobutanol and water were supplied into a vaporizer set at 150°C and vaporized. The flow rate of nitrogen gas was 3,000 mL (standard state)/hour. The concentrations of the isobutanol and the water in the raw material gas supplied to the catalyst layer were each 11.4% by volume and 14.8% by volume, and the temperature (the reaction temperature) of the catalyst layer during the reaction was 500°C.

[Example 55]

The catalyst performance was evaluated in the same manner as in Example 8 except for the following points. The inside of a reaction tube was filled with 1.1 g of the catalyst B-3 to form a catalyst layer. The flow rates of isobutanol and water were each set to 1.9 mL/hour and 2.0 mL/hour, and isobutanol and water were supplied into a vaporizer set at 150°C and vaporized. The flow rate of nitrogen gas was 1,140 mL (standard state)/hour. The concentrations of the isobutanol and the water in the raw material gas supplied to the catalyst layer were each 11.4% by volume and 60.2% by volume, and the temperature (the reaction temperature) of the catalyst layer during the reaction was 500°C.

[Example 56]

The catalyst performance was evaluated in the same manner as in Example 8 except for the following points. The inside of a reaction tube was filled with 1.1 g of the catalyst B-3 to form a catalyst layer. The flow rates of isobutanol and water were each set to 1.9 mL/hour and 2.6 mL/hour, and isobutanol and water were supplied into a vaporizer set at 150°C and vaporized. The flow rate of nitrogen gas was 540 mL (standard state)/hour. The concentrations of the isobutanol and the water in the raw material gas supplied to the catalyst layer were each 10.9% by volume and 76.4% by volume, and the temperature (the reaction temperature) of the catalyst layer during the reaction was 500°C.
Tables 16A and 16B show the reaction conditions and evaluation results in Examples 5, 8, and 54 to 56. In addition, Fig. 16 shows the isobutanol conversion rate, and the selectivities and yields of isobutyl aldehyde and methacrolein (MAL) by the catalyst of each example.

[Table 16A]

| | | Example 5 | Example 54 | Example 8 |
|---|---|---|---|---|
| Catalyst name | | K (5)/Al$_2$O$_3$ | K (5)/Al$_2$O$_3$ | K (5)/Al$_2$O$_3$ |
| Catalyst (abbreviation) | | B-3 | B-3 | B-3 |
| Average electronegativity | | 2.48 | 2.48 | 2.48 |
| Reaction temperature [°C] | | 500 | 500 | 500 |
| Catalyst mass [g] | | 1.1 | 1.1 | 1.1 |
| W/F [h$^{-1}$] | | 0.70 | 0.70 | 0.70 |
| Conditions for supply amount of raw material | | b | e | c |
| Supply amount of raw material | Isobutanol [mL/hour] | 1.9 | 1.9 | 1.9 |
| | Water [mL/hour] | 0.0 | 0.5 | 1.0 |
| | Nitrogen [mL (standard state)/hour] | 3660 | 3000 | 2460 |
| Composition of raw material gas [% by volume] | Isobutanol | 11.4 | 11.4 | 11.4 |
| | Water | 0.0 | 14.8 | 29.6 |
| | Nitrogen | 88.6 | 73.8 | 59.0 |
| Conversion rate [%] | | 85.4 | 52.8 | 38.4 |

(continued)

|  |  | Example 5 | Example 54 | Example 8 |
|---|---|---|---|---|
| Selectivity [%] | Isobutyl aldehyde + methacrolein | 55.0 | 70.3 | 73.0 |
|  | Isobutyl aldehyde | 54.9 | 70.0 | 73.0 |
|  | Methacrolein | 0.0 | 0.2 | 0.0 |
|  | Propionaldehyde | 0.0 | 0.2 | 0.0 |
|  | Propylene | 3.7 | 2.7 | 2.6 |
|  | C4 butenes | 29.3 | 19.1 | 15.9 |
|  | COx | 1.1 | 0.8 | 0.8 |
|  | Isobutyric acid | 0.6 | 0.9 | 1.2 |
|  | Ketones | 2.4 | 1.8 | 1.3 |
|  | Cyclic compounds | 0.8 | 0.6 | 0.4 |
|  | Unknown material + coke | 6.9 | 3.6 | 4.2 |
| Yield [%] | Isobutyl aldehyde + methacrolein | 46.9 | 37.1 | 28.0 |

[Table 16B]

|  |  | Example 55 | Example 56 |
|---|---|---|---|
| Catalyst name | | K (5)/Al$_2$O$_3$ | K (5)/Al$_2$O$_3$ |
| Catalyst (abbreviation) | | B-3 | B-3 |
| Average electronegativity | | 2.48 | 2.48 |
| Reaction temperature [°C] | | 500 | 500 |
| Catalyst mass [g] | | 1.1 | 1.1 |
| W/F [h$^{-1}$] | | 0.7 | 0.7 |
| Conditions for supply amount of raw material | | a | d |
| Supply amount of raw material | Isobutanol [mL/hour] | 1.9 | 1.9 |
|  | Water [mL/hour] | 2.0 | 2.6 |
|  | Nitrogen [mL (standard state)/hour] | 1140 | 540 |
| Composition of raw material gas [% by volume] | Isobutanol | 11.4 | 10.9 |
|  | Water | 60.2 | 76.4 |
|  | Nitrogen | 28.4 | 12.8 |
| Conversion rate [%] | | 29.4 | 27.7 |

(continued)

| Selectivity [%] | | Example 55 | Example 56 |
|---|---|---|---|
| | Isobutyl aldehyde + methacrolein | 72.7 | 72.8 |
| | Isobutyl aldehyde | 72.5 | 72.6 |
| | Methacrolein | 0.2 | 0.2 |
| | Propionaldehyde | 0.4 | 0.6 |
| | Propylene | 2.3 | 3.9 |
| | C4 butenes | 17.4 | 6.8 |
| | COx | 0.7 | 2.5 |
| | Isobutyric acid | 1.2 | 4.9 |
| | Ketones | 0.8 | 3.3 |
| | Cyclic compounds | 0.3 | 0.8 |
| | Unknown material + coke | 2.6 | 4.4 |
| Yield [%] | Isobutyl aldehyde + methacrolein | 21.3 | 20.2 |

As shown in Table 16, Table 16B, and Fig. 16, in Examples 5, 8, and 54 to 56 using the catalyst B-3, the selectivities of isobutyl aldehyde and methacrolein were high at any water concentration.

[Example 57]

The catalyst performance was evaluated in the same manner as in Example 2 except for the following points. The inside of a reaction tube was filled with 1.1 g of the catalyst B-2 to form a catalyst layer. The flow rate of isobutanol was set to 1.9 mL/hour, and isobutanol was supplied into a vaporizer set at 150°C and vaporized. The flow rate of nitrogen gas was set to 3,660 mL (standard state)/hour. In addition, the reaction temperature was set to 450°C. The isobutanol concentration in the raw material gas supplied to the catalyst layer was 11.4% by volume.

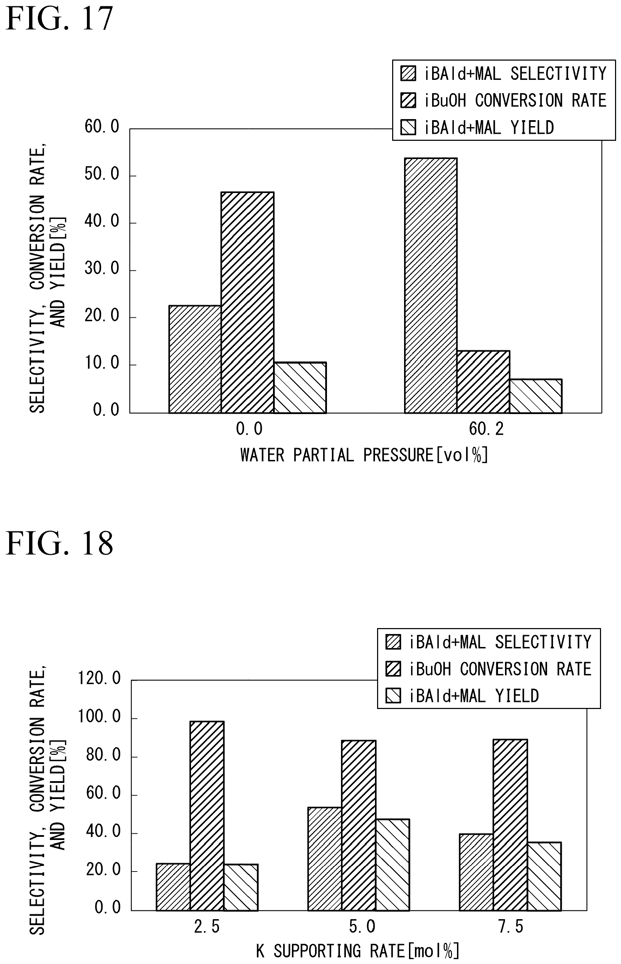

Table 17 shows the reaction conditions and evaluation results in Examples 2 and 57. In addition, Fig. 17 shows the isobutanol conversion rate, and the selectivities and yields of isobutyl aldehyde and methacrolein (MAL) by the catalyst of each example.

[Table 17]

| | | Example 57 | Example 2 |
|---|---|---|---|
| Catalyst name | | K (5)/Al$_2$O$_3$ (6.7)-FeOx (88.3) | K (5)/Al$_2$O$_3$ (6.7)-FeOx (88.3) |
| Catalyst (abbreviation) | | B-2 | B-2 |
| Average electronegativity | | 2.56 | 2.56 |
| Reaction temperature [°C] | | 450 | 450 |
| Catalyst mass [g] | | 1.1 | 1.07 |
| W/F [h$^{-1}$] | | 0.70 | 0.70 |
| Conditions for supply amount of raw material | | b | a |
| Supply amount of raw material | Isobutanol [mL/hour] | 1.9 | 1.9 |
| | Water [mL/hour] | 0.0 | 2.0 |
| | Nitrogen [mL (standard state)/hour] | 3660 | 1140 |

(continued)

|  |  | Example 57 | Example 2 |
|---|---|---|---|
| Composition of raw material gas [% by volume] | Isobutanol | 11.4 | 11.4 |
|  | Water | 0.0 | 60.2 |
|  | Nitrogen | 88.6 | 28.4 |
| Conversion rate [%] | | 46.5 | 12.9 |
| Selectivity [%] | Isobutyl aldehyde + methacrolein | 22.5 | 53.76 |
|  | Isobutyl aldehyde | 21.9 | 53.76 |
|  | Methacrolein | 0.6 | 0.00 |
|  | Propionaldehyde | 0.2 | 0.00 |
|  | Propylene | 7.3 | 0.00 |
|  | C4 butenes | 12.5 | 0.00 |
|  | COx | 4.7 | 26.44 |
|  | Isobutyric acid | 1.2 | 0.00 |
|  | Ketones | 10.9 | 0.00 |
|  | Cyclic compounds | 6.6 | 0.00 |
|  | Coke | 13.7 | 0.00 |
|  | Unknown material | 20.4 | 19.80 |
| Yield [%] | Isobutyl aldehyde + methacrolein | 10.5 | 6.9 |

As shown in Table 17 and Fig. 17, in Examples 2 and 57 using the catalyst B-2, the selectivities of isobutyl aldehyde and methacrolein were high at any water concentration.

[Example 58]

$\gamma$-alumina (manufactured by Strem Chemicals, Inc.) was calcined in the air at 550°C for 12 hours using an electric furnace as a pretreatment. 0.30 g of potassium nitrate was dissolved in 4.0 mL of distilled water (manufactured by FUJIFILM Wako Pure Chemical Corporation) to prepare an aqueous potassium nitrate solution. The aqueous potassium nitrate solution was uniformly added dropwise to 6.0 g of the pretreated alumina aliquoted into a mortar, and stirring was carried out for 15 minutes. After drying at 25°C for 24 hours, drying was carried out at 40°C for 2 hours and 60°C for 2 hours using a vacuum dryer, and drying was further carried out at 110°C for 24 hours using a dryer. After the drying treatment, grinding was carried out in a mortar, followed by calcining in the air in a calcining furnace at 550°C for 12 hours. After the calcining, grinding was carried out again in a mortar, and pressing was carried out at 2 tons for 15 minutes using a tablet molder to form a tablet, thereby preparing K (2.5)/Al$_2$O$_3$ (catalyst (abbreviation): B-16, acid amount: 0.167 mmol/g, base amount: 0.230 mmol/g, BET specific surface area: 183 m$^2$/g, average electronegativity: 2.51) having a particle diameter of 300 to 850 $\mu$m by using a sieve.

0.5 g of the catalyst B-16 (acid amount: 0.167 mmol/g, base amount: 0.230 mmol/g, BET specific surface area: 183 m$^2$/g) was used, isobutanol was set to 0.89 mL/hour, and isobutanol was supplied into a vaporizer set at 150°C and vaporized. The catalyst performance was evaluated in the same manner as in Example 5 except that the flow rate of nitrogen gas was set to 1,740 mL (standard state)/hour.

[Example 59]

The catalyst performance was evaluated in the same manner as in Example 58 except that 0.5 g of the catalyst B-10 (acid amount: 0.037 mmol/g, base amount: 0.638 mmol/g) was used.

Table 18 shows the reaction conditions and evaluation results in Examples 22, 58, and 59. In addition, Fig. 18 shows the isobutanol conversion rate, and the selectivities and yields of isobutyl aldehyde and methacrolein (MAL) by the catalyst of each example.

[Table 18]

|  | | Example 58 | Example 22 | Example 59 |
|---|---|---|---|---|
| Catalyst name | | K (2.5)/Al$_2$O$_3$ | K (5)/Al$_2$O$_3$ | K (7.5)/Al$_2$O$_3$ |
| Catalyst (abbreviation) | | B-16 | B-3 | B-10 |
| Potassium supporting rate [% by mole] | | 2.5 | 5.0 | 7.5 |
| Acid amount [mmol/g] | | 0.167 | 0.079 | 0.037 |
| Base amount [mmol/g] | | 0.230 | 0.439 | 0.638 |
| Average electronegativity | | 2.51 | 2.48 | 2.47 |
| Reaction temperature [°C] | | 500 | 500 | 500 |
| Catalyst mass [g] | | 0.5 | 1.1 | 0.5 |
| W/F [h$^{-1}$] | | 0.70 | 0.67 | 0.69 |
| Conditions for supply amount of raw material | | b | b | b |
| Supply amount of raw material | Isobutanol [ml/hour] | 0.9 | 1.9 | 1.9 |
|  | Water [ml/hour] | 0.0 | 0.0 | 0.0 |
|  | Nitrogen [ml (standard state)/hour] | 1740 | 3660 | 3660 |
| Composition of raw material gas [% by volume] | Isobutanol | 11.4 | 11.4 | 11.4 |
|  | Water | 0.0 | 0.0 | 0.0 |
|  | Nitrogen | 88.6 | 88.6 | 88.6 |
| Conversion rate [%] | | 98.4 | 88.6 | 89.1 |
| Selectivity [%] | Isobutyl aldehyde + methacrolein | 24.3 | 53.5 | 39.6 |
|  | Isobutyl aldehyde | 23.8 | 53.1 | 39.1 |
|  | Methacrolein | 0.5 | 0.4 | 0.4 |
|  | Propionaldehyde | 0.2 | 0.8 | 0.5 |
|  | Propylene | 2.0 | 2.7 | 3.3 |
|  | C4 butenes | 53.7 | 15.2 | 7.1 |
|  | COx | 0.7 | 2.1 | 4.7 |
|  | Isobutyric acid | 0.4 | 0.3 | 0.2 |
|  | Ketones | 0.1 | 4.3 | 15.5 |
|  | Cyclic compounds | 0.1 | 0.6 | 1.5 |
|  | Coke | 0.6 | 0.8 | 1.4 |
|  | Unknown material | 17.9 | 19.7 | 26.2 |
| Yield [%] | Isobutyl aldehyde + methacrolein | 23.9 | 47.4 | 35.2 |

As shown in Table 18 and Fig. 18, the selectivities of isobutyl aldehyde and methacrolein were high even when various catalysts having acid amounts and base amounts different from each other.

[Example 61]

The catalyst performance was evaluated in the same manner as in Example 1 except for the following points. The inside of a reaction tube was filled with 1.1 g of the catalyst B-1 to form a catalyst layer. The flow rates of isobutanol and water were each set to 1.9 mL/hour and 0.50 mL/hour, and isobutanol and water were supplied into a vaporizer set at 150°C and vaporized. The flow rate of nitrogen gas was 1,620 mL (standard state)/hour. In addition, the reaction temperature was set to 450°C.

The concentrations of isobutanol and water in the raw material gas supplied to the catalyst layer were 17.0% by volume and 23.0% by volume.

[Example 62]

The catalyst performance was evaluated in the same manner as in Example 1 except for the following points. The inside of a reaction tube was filled with 1.1 g of the catalyst B-1 to form a catalyst layer. The flow rates of isobutanol and water were each set to 1.9 mL/hour and 0.50 mL/hour, and isobutanol and water were supplied into a vaporizer set at 150°C and vaporized. Air and nitrogen gas were used as diluent gases, flow rates thereof were set to 900 mL (standard state)/hour and 780 mL (standard state)/hour, and air and nitrogen gas were supplied into a vaporizer and then supplied into a reactor together with the vaporized isobutanol. In addition, the reaction temperature was set to 450°C. The concentrations of isobutanol, water, and oxygen in the raw material gas supplied to the catalyst layer were 16.7% by volume, 22.6% by volume, and 6.8% by volume.

[Example 63]

The inside of a reaction tube was filled with 1.1 g of the catalyst B-1 to form a catalyst layer. The flow rates of isobutanol and water were each set to 1.9 mL/hour and 0.50 mL/hour, and isobutanol and water were supplied into a vaporizer set at 150°C and vaporized. Air was used as a diluent gas, the flow rate thereof was set to 1,680 mL (standard state)/hour, and the air was supplied into a vaporizer and then supplied into a reactor together with the vaporized isobutanol. In addition, the reaction temperature was set to 450°C. The concentrations of isobutanol, water, and oxygen in the raw material gas supplied to the catalyst layer were 16.8% by volume, 22.7% by volume, and 12.8% by volume.

Table 19 shows the reaction conditions and evaluation results in Examples 61 to 63.

[Table 19]

| | | Example 61 | Example 62 | Example 63 |
|---|---|---|---|---|
| Catalyst name | | K (5)/ZrO$_2$ (6.7)-FeOx (88.3) | K (5)/ZrO$_2$ (6.7)-FeOx (88.3) | K (5)/ZrO$_2$ (6.7)-FeOx (88.3) |
| Catalyst | | B-1 | B-1 | B-1 |
| Average electronegativity | | 2.55 | 2.55 | 2.55 |
| Reaction temperature [°C] | | 450 | 450 | 450 |
| Catalyst mass [g] | | 1.1 | 1.1 | 1.1 |
| W/F [h$^{-1}$] | | 0.68 | 0.70 | 0.70 |
| Conditions for supply amount of raw material | | g | h | i |
| Supply amount of raw material | Isobutanol [mL/hour] | 1.9 | 1.9 | 1.9 |
| | Water [mL/hour] | 0.5 | 0.5 | 0.5 |
| | Nitrogen [mL (standard state)/ hour] | 1620 | 780 | 0 |
| | Air [mL (standard state)/hour] | 0 | 900 | 1680 |

(continued)

| | | Example 61 | Example 62 | Example 63 |
|---|---|---|---|---|
| Composition of raw material gas [% by volume] | Isobutanol | 17.0 | 16.7 | 16.8 |
| | Water | 23.0 | 22.6 | 22.7 |
| | Oxygen | 0.0 | 6.8 | 12.8 |
| | Nitrogen | 60.0 | 53.9 | 47.7 |
| Conversion rate [%] | | 34.4 | 37.8 | 50.3 |
| Selectivity [%] | Isobutyl aldehyde + methacrolein | 23.0 | 31.7 | 27.3 |
| | Isobutyl aldehyde | 22.7 | 28.4 | 21.2 |
| | Methacrolein | 0.4 | 3.3 | 6.1 |
| | Propionaldehyde | 0.5 | 1.3 | 1.3 |
| | Propylene | 2.6 | 2.8 | 2.6 |
| | C4 butenes | 0.6 | 1.2 | 1.0 |
| | COx | 10.8 | 20.1 | 25.6 |
| | Isobutyric acid | 2.1 | 1.2 | 0.5 |
| | Ketones | 10.9 | 1.2 | 0.6 |
| | Cyclic compounds | 2.4 | 5.3 | 0.8 |
| | Coke | 9.1 | 6.0 | 4.3 |
| | Unknown material | 37.9 | 29.2 | 35.9 |
| Yield [%] | Isobutyl aldehyde + methacrolein | 7.9 | 12.0 | 13.7 |

As shown in Table 19, in Examples 61 to 63 using the catalyst B-1, the selectivities of isobutyl aldehyde and methacrolein were high even when the air was used as a diluent gas of the raw material gas.

[Example 64]

The catalyst performance was evaluated in the same manner as in Example 53 except for the following points. The inside of a reaction tube was filled with 1.1 g of the catalyst A-4 to form a catalyst layer. The flow rates of isobutanol and water were each set to 1.9 mL/hour and 0.50 mL/hour, and isobutanol and water were supplied into a vaporizer set at 150°C and vaporized. Nitrogen gas was used as a diluent gas, the flow rate thereof was set to 480 mL (standard state)/hour, and the air was supplied into a vaporizer and then supplied into a reactor together with the vaporized isobutanol. In addition, the reaction temperature was set to 450°C. The concentrations of isobutanol and water in the raw material gas supplied to the catalyst layer were 29.5% by volume and 40.0% by volume.

As shown in Table 20, in Examples 53 and 64 using the catalyst A-4, the selectivities of isobutyl aldehyde and methacrolein were high even when water was used as a diluent gas of the raw material gas.

[Table 20]

| | Example 53 | Example 64 |
|---|---|---|
| Catalyst name | ZnO | ZnO |
| Catalyst | A-4 | A-4 |
| Average electronegativity | 2.38 | 2.38 |
| Reaction temperature [°C] | 450 | 450 |
| Catalyst mass [g] | 1.1 | 1.1 |

(continued)

|  |  | Example 53 | Example 64 |
|---|---|---|---|
| W/F [h$^{-1}$] | | 0.68 | 0.68 |
| Conditions for supply amount of raw material | | b | j |
| Supply amount of raw material | Isobutanol [ml/hour] | 1.9 | 1.9 |
| | Water [ml/hour] | 0.0 | 0.5 |
| | Nitrogen [ml (standard state)/hour] | 1080 | 480 |
| Composition of raw material gas [% by volume] | Isobutanol | 29.6 | 29.5 |
| | Water | 0.0 | 40.0 |
| | Nitrogen | 70.4 | 30.5 |
| Conversion rate [%] | | 95.6 | 46.4 |
| Selectivity [%] | Isobutyl aldehyde + methacrolein | 69.3 | 81.0 |
| | Isobutyl aldehyde | 68.1 | 80.6 |
| | Methacrolein | 1.2 | 0.4 |
| | Propionaldehyde | 0.0 | 0.0 |
| | Propylene | 0.1 | 0.1 |
| | C4 butenes | 1.0 | 0.0 |
| | COx | 0.2 | 0.2 |
| | Isobutyric acid | 0.5 | 15.5 |
| | Ketones | 0.6 | 0.5 |
| | Cyclic compounds | 0.4 | 0.2 |
| | Coke | 0.0 | 0.0 |
| | Unknown material | 27.8 | 2.5 |
| Yield [%] | Isobutyl aldehyde + methacrolein | 66.3 | 37.6 |

[Example 65]

19.7 g of ethanol (manufactured by FUJIFILM Wako Pure Chemical Corporation) and a few drops of 0.1 mol/l hydrochloric acid (FUJIFILM Wako Pure Chemical Corporation) were added to 5.8 g of tetraethoxysilane (manufactured by FUJIFILM Wako Pure Chemical Corporation), and tetraethoxysilane was hydrolyzed while stirring at 75°C for 2 hours. 6.5 g of zinc nitrate hexahydrate (manufactured by FUJIFILM Wako Pure Chemical Corporation) was added thereto, stirring was carried out for 1 hour, a few drops of ammonia water (manufactured by FUJIFILM Wako Pure Chemical Corporation) was subsequently added thereto, and stirring was continued for 3 hours while heating at 75°C to obtain a gel. The obtained gel was dried in a dryer at 110°C for 12 hours and then calcined at 500°C for 12 hours. After the calcining, grinding was carried out in a mortar, and pressing was carried out at 2.0 tons for 15 minutes using a tablet molder to form a tablet, thereby obtaining ZnO (75)-SiO$_2$ (25) (catalyst (abbreviation): A-8, average electronegativity: 2.48, acid amount: 0.094 mmol/g, base amount: 0.002 mmol/g) having a particle diameter of 300 to 850 $\mu$m by using a sieve. Here, the number in parentheses indicates the content (% by mole) of the metal element.

The catalyst performance was evaluated in the same manner as in Example 52 except that 1.1 g of the catalyst A-8 was used.

[Example 66]

13.31 g of titanium sulfate and 14.85 g of zinc nitrate hexahydrate (manufactured by FUJIFILM Wako Pure Chemical Corporation) were added to 170 g of distilled water (manufactured by FUJIFILM Wako Pure Chemical Corporation) and dissolve at 25°C (prepared so that the element concentrations of titanium and alumina were 0.4 mol/l). After stirring this solution for 1 hour, ammonia water (manufactured by FUJIFILM Wako Pure Chemical Corporation) was added dropwise thereto until the pH reached 8.0, and further stirring was carried out at 25°C for 2 hours.

The obtained precipitate was separated by a centrifuge and recovered. Distilled water was added to the recovered precipitate to make it into a slurry shape and separated by a centrifuge. This operation was repeated twice.

The precipitate was collected and dried in a dryer at 110°C for 12 hours. The obtained solid was calcined in the air at 550°C for 8 hours. After the calcining, grinding was carried out in a mortar, and pressing was carried out at 2.0 tons for 15 minutes using a tablet molder to form a tablet, thereby obtaining ZnO (75)-TiO$_2$ (25) (catalyst (abbreviation): A-9, average electronegativity: 2.44, acid amount: 0.097 mmol/g, base amount: 0.000 mmol/g, BET specific surface area: 18 m$^2$/g) having a particle diameter of 300 to 850 μm by using a sieve. Here, the number in parentheses indicates the content (% by mole) of the metal element.

The catalyst performance was evaluated in the same manner as in Example 52 except that 1.1 g of the catalyst A-9 was used.

[Example 67]

12.5 g of aluminum nitrate nonahydrate (manufactured by FUJIFILM Wako Pure Chemical Corporation) and 29.7 g of zinc nitrate hexahydrate (manufactured by FUJIFILM Wako Pure Chemical Corporation) were added to 330 g of distilled water (manufactured by FUJIFILM Wako Pure Chemical Corporation) and dissolved at 25°C. After stirring this solution for 1 hour, ammonia water (manufactured by FUJIFILM Wako Pure Chemical Corporation) was added dropwise thereto until the pH reached 8.0, and further stirring was carried out at 25°C for 2 hours. The obtained precipitate was separated using a centrifuge and washed with distilled water. This operation was repeated twice. The precipitate was dried in a dryer at 110°C for 12 hours. The obtained solid was calcined in the air at 550°C for 8 hours. After the calcining, grinding was carried out in a mortar, and pressing was carried out at 2.0 tons for 15 minutes using a tablet molder to form a tablet, thereby obtaining ZnO (75)-Al$_2$O$_3$ (25) (catalyst (abbreviation): A-10, average electronegativity: 2.41, acid amount: 0.04 mmol/g, base amount: 0.004 mmol/g, BET specific surface area: 32 m$^2$/g) having a diameter of 300 to 850 μm by using a sieve. Here, the number in parentheses indicates the content (% by mole) of each component.

The catalyst performance was evaluated in the same manner as in Example 52 except that 1.1 g of the catalyst A-10 was used.

[Table 21]

| | | Example 52 | Example 65 | Example 66 | Example 67 |
|---|---|---|---|---|---|
| Catalyst name | | ZnO | ZnO (75)-SiO$_2$ (25) | ZnO (75)-TiO$_2$ (25) | ZnO (75)-Al$_2$O$_3$ (25) |
| Catalyst | | A-4 | A-8 | A-9 | A-10 |
| Acid amount [mmol/g] | | 0.003 | 0.094 | 0.097 | 0.04 |
| Base amount [mmol/g] | | 0.003 | 0.002 | 0 | 0.04 |
| Average electronegativity | | 2.38 | 2.48 | 2.44 | 2.41 |
| Reaction temperature [°C] | | 400 | 400 | 400 | 400 |
| Catalyst mass [g] | | 1.1 | 1.1 | 1.1 | 1.1 |
| W/F [h$^{-1}$] | | 0.70 | 0.70 | 0.70 | 0.70 |
| Conditions for supply amount of raw material | | d | d | d | d |
| Supply amount of raw material | Isobutanol [ml/hour] | 1.9 | 1.9 | 1.9 | 1.9 |
| | Water [ml/hour] | 0.0 | 0.0 | 0.0 | 0.0 |
| | Nitrogen [ml (standard state)/hour] | 1080 | 1080 | 1080 | 1080 |
| Composition of raw material gas [% by volume] | Isobutanol | 29.6 | 29.6 | 29.6 | 29.6 |
| | Water | 0.0 | 0.0 | 0.0 | 0.0 |
| | Nitrogen | 70.4 | 70.4 | 70.4 | 70.4 |
| Conversion rate [%] | | 43.9 | 98.6 | 95.7 | 84.8 |

(continued)

|  |  | Example 52 | Example 65 | Example 66 | Example 67 |
|---|---|---|---|---|---|
| Selectivity [%] | Isobutyl aldehyde + methacrolein | 94.6 | 30.1 | 46.5 | 88.5 |
|  | Isobutyl aldehyde | 94.5 | 30.0 | 46.2 | 88.2 |
|  | Methacrolein | 0.1 | 0.2 | 0.3 | 0.2 |
|  | Propionaldehyde | 0.0 | 0.0 | 0.0 | 0.0 |
|  | Propylene | 0.0 | 0.0 | 0.0 | 0.0 |
|  | C4 butenes | 1.5 | 40.6 | 16.7 | 0.3 |
|  | COx | 0.0 | 0.0 | 0.0 | 0.1 |
|  | Isobutyric acid | 1.2 | 3.3 | 1.8 | 1.9 |
|  | Ketones | 0.2 | 0.0 | 0.4 | 0.5 |
|  | Cyclic compounds | 0.3 | 0.0 | 5.4 | 0.2 |
|  | Coke | 0.0 | 0.2 | 0.0 | 0.0 |
|  | Unknown material | 2.2 | 25.6 | 29.1 | 8.5 |
| Yield [%] | Isobutyl aldehyde + methacrolein | 41.5 | 29.7 | 44.5 | 75.0 |

As shown in Table 21, in Examples 52 and 65 to 67 using the catalyst A-4 and 8 to 10, the selectivities of isobutyl aldehyde and methacrolein were high.

[Example 68]

28.09 g of aluminum nitrate nonahydrate (manufactured by FUJIFILM Wako Pure Chemical Corporation) and 7.43 g of zinc nitrate hexahydrate (manufactured by FUJIFILM Wako Pure Chemical Corporation) were added to 250 g of distilled water (manufactured by FUJIFILM Wako Pure Chemical Corporation) and dissolved at 25°C. After stirring this solution for 1 hour, ammonia water (manufactured by FUJIFILM Wako Pure Chemical Corporation) was added dropwise thereto until the pH reached 8.0, and further stirring was carried out at 25°C for 2 hours. The obtained precipitate was separated using a centrifuge and washed with distilled water. This operation was repeated twice. The precipitate was dried in a dryer at 110°C for 12 hours. The obtained solid was calcined in the air at 550°C for 8 hours. After the calcining, grinding was carried out in a mortar, and pressing was carried out at 2.0 tons for 15 minutes using a tablet molder to form a tablet, thereby obtaining ZnO (25)-$Al_2O_3$ (75) (catalyst (abbreviation): A-11, average electronegativity: 2.51, acid amount: 0.284 mmol/g, base amount: 0.119 mmol/g) having a particle diameter of 300 to 850 $\mu$m by using a sieve. Here, the number in parentheses indicates the content (% by mole) of each component.
The catalyst performance was evaluated in the same manner as in Example 52 except that 1.1 g of the catalyst A-11 was used.

[Example 69]

37.83 g of aluminum nitrate nonahydrate (manufactured by FUJIFILM Wako Pure Chemical Corporation) and 30.0 g of zinc nitrate hexahydrate (manufactured by FUJIFILM Wako Pure Chemical Corporation) were added to 630 g of distilled water (manufactured by FUJIFILM Wako Pure Chemical Corporation) and dissolved at 25°C. After stirring this solution for 1 hour, ammonia water (manufactured by FUJIFILM Wako Pure Chemical Corporation) was added dropwise thereto until the pH reached 8.0, and further stirring was carried out at 25°C for 2 hours. The obtained precipitate was separated using a centrifuge and washed with distilled water. This operation was repeated twice. The precipitate was dried in a dryer at 110°C for 12 hours. The obtained solid was calcined in the air at 550°C for 8 hours. After the calcining, grinding was carried out in a mortar, and pressing was carried out at 2.0 tons for 15 minutes using a tablet molder to form a tablet, thereby obtaining ZnO (40)-$Al_2O_3$ (60) (catalyst (abbreviation): A-12, average electronegativity: 2.48, acid amount: 0.131 mmol/g, base amount: 0.187 mmol/g) having a particle diameter of 300 to 850 $\mu$m by using a sieve. Here, the number in parentheses indicates the content (% by mole) of each component.

The catalyst performance was evaluated in the same manner as in Example 52 except that 1.1 g of the catalyst A-12 was used.

[Example 70]

56.74 g of aluminum nitrate nonahydrate (manufactured by FUJIFILM Wako Pure Chemical Corporation) and 30.0 g of zinc nitrate hexahydrate (manufactured by FUJIFILM Wako Pure Chemical Corporation) were added to 500 g of distilled water (manufactured by FUJIFILM Wako Pure Chemical Corporation) and dissolved at 25°C. After stirring this solution for 1 hour, ammonia water (manufactured by FUJIFILM Wako Pure Chemical Corporation) was added dropwise thereto until the pH reached 8.0, and further stirring was carried out at 25°C for 2 hours. The obtained precipitate was separated using a centrifuge and washed with distilled water. This operation was repeated twice. The precipitate was dried in a dryer at 110°C for 12 hours. The obtained solid was calcined in the air at 550°C for 8 hours. After the calcining, grinding was carried out in a mortar, and pressing was carried out at 2.0 tons for 15 minutes using a tablet molder to form a tablet, thereby obtaining ZnO (50)-$Al_2O_3$ (50) (catalyst (abbreviation): A-13, average electronegativity: 2.48, acid amount: 0.103 mmol/g, base amount: 0.107 mmol/g, BET specific surface area: 53 $m^2$/g) having a particle diameter of 300 to 850 $\mu$m by using a sieve. Here, the number in parentheses indicates the content (% by mole) of each component.

The catalyst performance was evaluated in the same manner as in Example 52 except that 1.1 g of the catalyst A-13 was used.

[Example 71]

12.61 g of aluminum nitrate nonahydrate (manufactured by FUJIFILM Wako Pure Chemical Corporation) and 20.0 g of zinc nitrate hexahydrate (manufactured by FUJIFILM Wako Pure Chemical Corporation) were added to 250 g of distilled water (manufactured by FUJIFILM Wako Pure Chemical Corporation) and dissolved at 25°C. After stirring this solution for 1 hour, ammonia water (manufactured by FUJIFILM Wako Pure Chemical Corporation) was added dropwise thereto until the pH reached 8.0, and further stirring was carried out at 25°C for 2 hours. The obtained precipitate was separated using a centrifuge and washed with distilled water. This operation was repeated twice. The precipitate was dried in a dryer at 110°C for 12 hours. The obtained solid was calcined in the air at 550°C for 8 hours. After the calcining, grinding was carried out in a mortar, and pressing was carried out at 2.0 tons for 15 minutes using a tablet molder to form a tablet, thereby obtaining ZnO (66)-$Al_2O_3$ (33) (catalyst (abbreviation): A-14, average electronegativity: 2.47, acid amount: 0.077 mmol/g, base amount: 0.046 mmol/g) having a diameter of 300 to 850 $\mu$m by using a sieve. Here, the number in parentheses indicates the content (% by mole) of each component.

The catalyst performance was evaluated in the same manner as in Example 52 except that 1.1 g of the catalyst A-14 was used.

[Example 79]

20.0 g of magnesium nitrate hexahydrate (manufactured by FUJIFILM Wako Pure Chemical Corporation) and 14.63 g of aluminum nitrate nonahydrate (manufactured by FUJIFILM Wako Pure Chemical Corporation) were added to 300 g of distilled water (manufactured by FUJIFILM Wako Pure Chemical Corporation) and dissolved at 25°C. After stirring this solution for 1 hour, ammonia water (manufactured by FUJIFILM Wako Pure Chemical Corporation) was added dropwise thereto until the pH became 9.0, and further stirring was carried out at 25°C for 2 hours. The obtained precipitate was separated using a centrifuge and washed with distilled water. This operation was repeated twice. The precipitate was dried in a dryer at 110°C for 12 hours. The obtained solid was calcined in the air at 550°C for 8 hours. After the calcining, grinding was carried out in a mortar, and pressing was carried out at 2.0 tons for 15 minutes using a tablet molder to form a tablet, thereby obtaining MgO (66)-$Al_2O_3$ (33) (catalyst (abbreviation): A-18, average electronegativity: 2.27, acid amount: 0.12 mmol/g, base amount: 0.13 mmol/g) having a particle diameter of 300 to 850 $\mu$m by using a sieve. Here, the number in parentheses indicates the content (% by mole) of each component.

[Table 22A]

| | Example 68 | Example 69 | Example 70 |
|---|---|---|---|
| Catalyst name | ZnO (25)-$Al_2O_3$ (75) | ZnO (40)-$Al_2O_3$ (60) | ZnO (50)-$Al_2O_3$ (50) |
| Catalyst | A-11 | A-12 | A-13 |
| Acid amount [mmol/g] | 0.284 | 0.131 | 0.103 |

(continued)

| | | Example 68 | Example 69 | Example 70 |
|---|---|---|---|---|
| Base amount [mmol/g] | | 0.119 | 0.187 | 0.107 |
| Average electronegativity | | 2.51 | 2.48 | 2.48 |
| Reaction temperature [°C] | | 400 | 400 | 400 |
| Catalyst mass [g] | | 1.1 | 1.1 | 1.1 |
| W/F [h$^{-1}$] | | 0.70 | 0.70 | 0.70 |
| Conditions for supply amount of raw material | | d | d | d |
| Supply amount of raw material | Isobutanol [ml/hour] | 1.9 | 1.9 | 1.9 |
| | Water [ml/hour] | 0.0 | 0.0 | 0.0 |
| | Nitrogen [ml (standard state)/hour] | 1080 | 1080 | 1080 |
| Composition of raw material gas [% by volume] | Isobutanol | 29.6 | 29.6 | 29.6 |
| | Water | 0.0 | 0.0 | 0.0 |
| | Nitrogen | 70.4 | 70.4 | 70.4 |
| Conversion rate [%] | | 56.5 | 91.4 | 91.0 |
| Selectivity [%] | Isobutyl aldehyde + methacrolein | 55.2 | 82.1 | 88.0 |
| | Isobutyl aldehyde | 55.2 | 80.8 | 87.0 |
| | Methacrolein | 0.0 | 1.3 | 1.0 |
| | Propionaldehyde | 0.0 | 0.1 | 0.0 |
| | Propylene | 0.0 | 0.2 | 0.1 |
| | C4 butenes | 26.6 | 0.8 | 0.3 |
| | COx | 0.0 | 1.3 | 0.5 |
| | Isobutyric acid | 3.4 | 0.3 | 0.7 |
| | Ketones | 0.2 | 6.3 | 2.3 |
| | Cyclic compounds | 0.0 | 1.5 | 0.5 |
| | Coke | 1.9 | 0.0 | 0.5 |
| | Unknown material | 12.6 | 7.4 | 7.1 |
| Yield [%] | Isobutyl aldehyde + methacrolein | 31.2 | 75.1 | 80.1 |

[Table 22B]

| | Example 71 | Example 67 | Example 52 | Example 79 |
|---|---|---|---|---|
| Catalyst name | ZnO (66)-Al$_2$O$_3$ (33) | ZnO (75)-Al$_2$O$_3$ (25) | ZnO | MgO (66)-Al$_2$O$_3$ (33) |
| Catalyst | A-14 | A-10 | A-4 | A-18 |
| Acid amount [mmol/g] | 0.077 | 0.04 | 0.003 | 0.12 |
| Base amount [mmol/g] | 0.046 | 0.04 | 0.003 | 0.13 |

(continued)

| | | Example 71 | Example 67 | Example 52 | Example 79 |
|---|---|---|---|---|---|
| Average electronegativity | | 2.47 | 2.43 | 2.38 | 2.27 |
| Reaction temperature [°C] | | 400 | 400 | 400 | 400 |
| Catalyst mass [g] | | 1.1 | 1.1 | 1.1 | 1.1 |
| W/F [h$^{-1}$] | | 0.70 | 0.70 | 0.70 | 0.70 |
| Conditions for supply amount of raw material | | d | d | d | d |
| Supply amount of raw material | Isobutanol [ml/hour] | 1.9 | 1.9 | 1.9 | 1.9 |
| | Water [ml/hour] | 0.0 | 0.0 | 0.0 | 0.0 |
| | Nitrogen [ml (standard state)/hour] | 1080 | 1080 | 1080 | 1080 |
| Composition of raw material gas [% by volume] | Isobutanol | 29.6 | 29.6 | 29.6 | 29.6 |
| | Water | 0.0 | 0.0 | 0.0 | 0.0 |
| | Nitrogen | 70.4 | 70.4 | 70.4 | 70.4 |
| Conversion rate [%] | | 86.8 | 84.8 | 43.9 | 39.5 |
| Selectivity [%] | Isobutyl aldehyde + methacrolein | 96.6 | 88.5 | 94.6 | 39.5 |
| | Isobutyl aldehyde | 96.3 | 88.2 | 94.5 | 39.5 |
| | Methacrolein | 0.2 | 0.2 | 0.1 | 0.0 |
| | Propionaldehyde | 0.0 | 0.0 | 0.0 | 0.0 |
| | Propylene | 0.0 | 0.0 | 0.0 | 0.0 |
| | C4 butenes | 0.2 | 0.3 | 1.5 | 14.9 |
| | COx | 0.1 | 0.1 | 0.0 | 0.0 |
| | Isobutyric acid | 1.0 | 1.9 | 1.2 | 0.2 |
| | Ketones | 0.2 | 0.5 | 0.2 | 0.8 |
| | Cyclic compounds | 0.2 | 0.2 | 0.3 | 0.0 |
| | Coke | 0.3 | 0.4 | 0.0 | 0.0 |
| | Unknown material | 1.4 | 8.1 | 2.2 | 44.4 |
| Yield [%] | Isobutyl aldehyde + methacrolein | 83.8 | 75.0 | 41.5 | 15.6 |

As shown in Tables 22A and 22B, in Examples 52, 67 to 71, and 79 using the catalysts A-4, 10, 11 to 14, and 18, the selectivities of isobutyl aldehyde and methacrolein were high.

[Examples 72 to 76]

The catalyst performance was evaluated in the same manner as in Example 52 except that the catalyst and the reaction temperature were changed as shown in Tables 23A and 23B.

[Table 23A]

| | Example 72 | Example 73 | Example 74 |
|---|---|---|---|
| Catalyst name | ZnO (25)-Al$_2$O$_3$ (75) | ZnO (33)-Al$_2$O$_3$ (66) | ZnO (40)-Al$_2$O$_3$ (60) |

(continued)

|  |  | Example 72 | Example 73 | Example 74 |
|---|---|---|---|---|
| Catalyst |  | A-11 | A-15 | A-12 |
| Acid amount [mmol/g] |  | 0.276 | 0.103 | 0.077 |
| Base amount [mmol/g] |  | 0.072 | 0.092 | 0.046 |
| Average electronegativity |  | 2.51 | 2.49 | 2.48 |
| Reaction temperature [°C] |  | 350 | 350 | 350 |
| Catalyst mass [g] |  | 1.1 | 1.1 | 1.1 |
| W/F [$h^{-1}$] |  | 0.70 | 0.70 | 0.70 |
| Conditions for supply amount of raw material |  | d | a | d |
| Supply amount of raw material | Isobutanol [ml/hour] | 1.9 | 1.9 | 1.9 |
|  | Water [ml/hour] | 0.0 | 0.0 | 0.0 |
|  | Nitrogen [ml (standard state)/hour] | 1080 | 1080 | 1080 |
| Composition of raw material gas [% by volume] | Isobutanol | 29.6 | 29.6 | 29.6 |
|  | Water | 0.0 | 0.0 | 0.0 |
|  | Nitrogen | 70.4 | 70.4 | 70.4 |
| Conversion rate [%] |  | 28.4 | 42.5 | 63.2 |
| Selectivity [%] | Isobutyl aldehyde + methacrolein | 51.7 | 86.8 | 84.1 |
|  | Isobutyl aldehyde | 51.7 | 86.8 | 84.1 |
|  | Methacrolein | 0.0 | 0.0 | 0.0 |
|  | Propionaldehyde | 0.0 | 0.0 | 0.0 |
|  | Propylene | 0.0 | 0.0 | 0.0 |
|  | C4 butenes | 38.7 | 0.2 | 0.1 |
|  | COx | 0.0 | 0.0 | 0.1 |
|  | Isobutyric acid | 0.4 | 1.5 | 2.6 |
|  | Ketones | 1.1 | 0.1 | 0.4 |
|  | Cyclic compounds | 0.1 | 0.4 | 0.1 |
|  | Coke | 3.5 | 1.3 | 1.1 |
|  | Unknown material | 4.6 | 9.6 | 11.5 |
| Yield [%] | Isobutyl aldehyde + methacrolein | 14.7 | 36.9 | 53.2 |

[Table 23B]

|  | Example 75 | Example 76 |
|---|---|---|
| Catalyst name | ZnO (50)-$Al_2O_3$ (50) | ZnO (75)-$Al_2O_3$ (25) |
| Catalyst | A-13 | A-10 |
| Acid amount [mmol/g] | 0.077 | 0.04 |
| Base amount [mmol/g] | 0.046 | 0.029 |

(continued)

|  | | Example 75 | Example 76 |
|---|---|---|---|
| Average electronegativity | | 2.47 | 2.43 |
| Reaction temperature [°C] | | 350 | 350 |
| Catalyst mass [g] | | 1.1 | 1.1 |
| W/F [h$^{-1}$] | | 0.70 | 0.70 |
| Conditions for supply amount of raw material | | d | d |
| Supply amount of raw material | Isobutanol [ml/hour] | 1.9 | 1.9 |
| | Water [ml/hour] | 0.0 | 0.0 |
| | Nitrogen [m] (standard state)/hour] | 1080 | 1080 |
| Composition of raw material gas [% by volume] | Isobutanol | 29.6 | 29.6 |
| | Water | 0.0 | 0.0 |
| | Nitrogen | 70.4 | 70.4 |
| Conversion rate [%] | | 48.0 | 40.4 |
| Selectivity [%] | Isobutyl aldehyde + methacrolein | 93.9 | 96.1 |
| | Isobutyl aldehyde | 93.9 | 96.1 |
| | Methacrolein | 0.0 | 0.0 |
| | Propionaldehyde | 0.0 | 0.0 |
| | Propylene | 0.0 | 0.0 |
| | C4 butenes | 0.2 | 0.1 |
| | COx | 0.1 | 0.0 |
| | Isobutyric acid | 3.6 | 1.8 |
| | Ketones | 0.2 | 0.2 |
| | Cyclic compounds | 0.3 | 0.0 |
| | Coke | 1.2 | 1.0 |
| | Unknown material | 0.5 | 0.8 |
| Yield [%] | Isobutyl aldehyde + methacrolein | 45.1 | 38.9 |

As shown in Tables 23A and 23B, in Examples 72 to 76 using the catalysts A-10 and 11 to 14, the selectivities of isobutyl aldehyde and methacrolein were high.

[Example 77]

The catalyst performance was evaluated in the same manner as in Example 52 except that the catalyst A-12 was used and the reaction temperature was set to 300°C.

[Table 24]

|  | Example 77 | Example 74 | Example 69 |
|---|---|---|---|
| Catalyst name | ZnO (40)-Al$_2$O$_3$ (60) | ZnO (40)-Al$_2$O$_3$ (60) | ZnO (40)-Al$_2$O$_3$ (60) |
| Catalyst | A-12 | A-12 | A-12 |

(continued)

| | | Example 77 | Example 74 | Example 69 |
|---|---|---|---|---|
| Acid amount [mmol/g] | | 0.077 | 0.077 | 0.077 |
| Base amount [mmol/g] | | 0.046 | 0.046 | 0.046 |
| Average electronegativity | | 2.48 | 2.48 | 2.48 |
| Reaction temperature [°C] | | 300 | 350 | 400 |
| Catalyst mass [g] | | 1.1 | 1.1 | 1.1 |
| W/F [h$^{-1}$] | | 0.70 | 0.70 | 0.70 |
| Conditions for supply amount of raw material | | d | d | d |
| Supply amount of raw material | Isobutanol [ml/hour] | 1.9 | 1.9 | 1.9 |
| | Water [ml/hour] | 0.0 | 0.0 | 0.0 |
| | Nitrogen [ml (standard state)/hour] | 1080 | 1080 | 1080 |
| Composition of raw material gas [% by volume] | Isobutanol | 29.6 | 29.6 | 29.6 |
| | Water | 0.0 | 0.0 | 0.0 |
| | Nitrogen | 70.4 | 70.4 | 70.4 |
| Conversion rate [%] | | 9.9 | 52.3 | 91.4 |
| Selectivity [%] | Isobutyl aldehyde + methacrolein | 77.3 | 94.1 | 82.1 |
| | Isobutyl aldehyde | 77.3 | 94.0 | 80.8 |
| | Methacrolein | 0.0 | 0.1 | 1.3 |
| | Propionaldehyde | 0.0 | 0.0 | 0.1 |
| | Propylene | 0.0 | 0.0 | 0.2 |
| | C4 butenes | 0.0 | 0.1 | 0.8 |
| | COx | 0.1 | 0.1 | 1.3 |
| | Isobutyric acid | 0.7 | 1.2 | 0.3 |
| | Ketones | 0.2 | 0.3 | 6.3 |
| | Cyclic compounds | 0.0 | 0.2 | 1.5 |
| | Coke | 5.8 | 1.3 | 0.0 |
| | Unknown material | 15.9 | 2.7 | 7.4 |
| Yield [%] | Isobutyl aldehyde + methacrolein | 7.7 | 49.2 | 75.1 |

As shown in Table 24, even in the case of Examples 69, 74, and 77 in which the reaction was carried out at a reaction temperature of 300 to 400°C using the catalyst A-12, the selectivities of isobutyl aldehyde and methacrolein were high.

[Example 78]

56.74 g of aluminum nitrate nonahydrate (manufactured by FUJIFILM Wako Pure Chemical Corporation) and 30.0 g of zinc nitrate hexahydrate (manufactured by FUJIFILM Wako Pure Chemical Corporation) were added to 500 g of distilled water (manufactured by FUJIFILM Wako Pure Chemical Corporation) and dissolved at 25°C. After stirring this solution for 1 hour, ammonia water (manufactured by FUJIFILM Wako Pure Chemical Corporation) was added dropwise thereto until the pH reached 8.0, and further stirring was carried out at 25°C for 2 hours. The obtained precipitate was separated using a centrifuge and washed with distilled water. This operation was repeated twice. The precipitate was dried in a dryer at 110°C for 12 hours. The obtained solid was calcined in the air at 900°C for 8 hours. After the calcining, grinding was carried out in a mortar, and pressing was carried out at 2.0 tons for 15 minutes using a tablet molder to form a tablet, thereby obtaining ZnO (50)-Al$_2$O$_3$ (50) (catalyst (abbreviation): A-16, average electronegativity: 2.48, acid amount: 0.103 mmol/g, base amount: 0.107 mmol/g) having a particle diameter of 300 to 850 μm by using a sieve. Here, the number in parentheses indicates the content (% by mole) of each component.

The catalyst performance was evaluated in the same manner as in Example 75 except that 1.1 g of the catalyst A-16 was used.

[Table 25]

| | | Example 75 | Example 78 |
|---|---|---|---|
| Catalyst name | | ZnO (50)-Al$_2$O$_3$ (50) calcined at 550°C | ZnO (50)-Al$_2$O$_3$ (50) calcined at 900°C |
| Catalyst | | A-13 | A-16 |
| Reaction temperature [°C] | | 350 | 350 |
| Catalyst mass [g] | | 1.1 | 1.1 |
| W/F [h$^{-1}$] | | 0.70 | 0.70 |
| Conditions for supply amount of raw material | | d | d |
| Supply amount of raw material | Isobutanol [ml/hour] | 1.9 | 1.9 |
| | Water [ml/hour] | 0.0 | 0.0 |
| | Nitrogen [ml (standard state)/hour] | 1080 | 1080 |
| Composition of raw material gas [% by volume] | Isobutanol | 29.6 | 29.6 |
| | Water | 0.0 | 0.0 |
| | Nitrogen | 70.4 | 70.4 |
| Conversion rate [%] | | 44.7 | 21.3 |
| Selectivity [%] | Isobutyl aldehyde + methacrolein | 95.8 | 89.0 |
| | Isobutyl aldehyde | 95.7 | 89.0 |
| | Methacrolein | 0.1 | 0.0 |
| | Propionaldehyde | 0.0 | 0.0 |
| | Propylene | 0.0 | 0.0 |
| | C4 butenes | 0.1 | 0.7 |
| | COx | 0.0 | 0.0 |
| | Isobutyric acid | 1.7 | 1.0 |
| | Ketones | 0.3 | 0.1 |
| | Cyclic compounds | 0.5 | 1.2 |
| | Coke | 1.3 | 0.0 |
| | Unknown material | 0.3 | 8.0 |
| Yield [%] | Isobutyl aldehyde + methacrolein | 42.8 | 18.9 |

As shown in Table 25, even when the catalyst A-16 was used and the reaction temperature was changed from 300°C to 400°C, the selectivities of isobutyl aldehyde and methacrolein were high.

[Industrial Applicability]

According to the present invention, it is possible to provide a catalyst for dehydration, by which a reduction treatment with hydrogen is not required, and isobutyl aldehyde and methacrolein can be produced from isobutanol with a high selectivity while suppressing the oxidation and dimerization of isobutyl aldehyde and to provide a method for producing a dehydrogenated compound using a catalyst for dehydration.

**Claims**

1. A catalyst having an average electronegativity of 2.1 or more and 2.8 or less.

2. The catalyst according to Claim 1,
   wherein the catalyst contains a metal oxide having an average electronegativity of 2.1 or more and 2.8 or less.

3. The catalyst according to Claim 2,
   wherein the metal oxide includes an oxide of one or more metals selected from the group consisting of Al, Si, Zn, Ag, Ce, La, and Mg.

4. The catalyst according to Claim 2,
   wherein the metal oxide includes an oxide of one or more metals selected from the group consisting of Zn, Al, Fe, Si, Zr, and Ti.

5. The catalyst according to any one of Claims 1 to 4,
   wherein the catalyst contains one or more alkali metals.

6. The catalyst according to any one of Claims 1 to 5,
   wherein an acid amount with respect to 1 g of the catalyst is 0.3 mmol/g or less.

7. The catalyst according to any one of Claims 1 to 6,
   wherein the catalyst is a catalyst that is used for producing, from isobutanol, at least one dehydrogenated compound selected from the group consisting of isobutyl aldehyde and methacrolein.

8. A method for producing the catalyst according to any one of Claims 1 to 7, the method comprising:
   calcining a metal oxide.

9. A method for producing a dehydrogenated compound, which is a method for producing a dehydrogenated compound from isobutanol, the method comprising:
   producing, from isobutanol, at least one dehydrogenated compound selected from the group consisting of isobutyl aldehyde and methacrolein by using the catalyst according to any one of Claims 1 to 7.

10. A method for producing methacrylic acid, the method comprising:
    producing methacrylic acid from at least one selected from the group consisting of the isobutyl aldehyde and methacrolein obtained according to the method for producing a dehydrogenated compound according to Claim 9.

11. A method for producing a methacrylic acid ester, the method comprising:
    producing methacrylic acid according to the method for producing methacrylic acid according to Claim 10 and producing a methacrylic acid ester from the obtained methacrylic acid and an alcohol having 1 to 20 carbon atoms.

FIG. 1

FIG. 2

## FIG. 3

## FIG. 4

## FIG. 5

## FIG. 6

FIG. 7

FIG. 8A

FIG. 8B

FIG. 8C

## FIG. 9

## FIG. 10

## FIG. 11

## FIG. 12

## FIG. 13

## FIG. 14

FIG. 15

FIG. 16

FIG. 17

FIG. 18

FIG. 19

FIG. 20

## FIG. 21

## FIG. 22

FIG. 23

FIG. 24

FIG. 25

**INTERNATIONAL SEARCH REPORT**

International application No.

PCT/JP2021/013989

| A. | CLASSIFICATION OF SUBJECT MATTER |
|---|---|

Int.Cl. B01J23/78(2006.01)i, B01J21/10(2006.01)i, B01J23/02(2006.01)i, B01J23/04(2006.01)i, B01J23/06(2006.01)i, B01J23/10(2006.01)i, B01J29/70(2006.01)i, C07C45/29(2006.01)i, C07C47/02(2006.01)i, C07C47/22(2006.01)i, C07B61/00(2006.01)n
FI: B01J23/78Z, B01J21/10Z, B01J23/02Z, B01J23/04Z, B01J23/06Z, B01J23/10Z, B01J29/70Z, C07C45/29, C07C47/02, C07C47/22, C07C47/22Z, C07B61/00300

According to International Patent Classification (IPC) or to both national classification and IPC

| B. | FIELDS SEARCHED |
|---|---|

Minimum documentation searched (classification system followed by classification symbols)
Int.Cl. B01J23/78, B01J21/10, B01J23/02, B01J23/04, B01J23/06, B01J23/10, B01J29/70, C07C45/29, C07C47/02, C07C47/22, C07B61/00

Documentation searched other than minimum documentation to the extent that such documents are included in the fields searched
Published examined utility model applications of Japan        1922-1996
Published unexamined utility model applications of Japan      1971-2021
Registered utility model specifications of Japan              1996-2021
Published registered utility model applications of Japan      1994-2021

Electronic data base consulted during the international search (name of data base and, where practicable, search terms used)
JSTPlus(JDreamIII), JST7580(JDreamIII), JSTChina(JDreamIII)

| C. | DOCUMENTS CONSIDERED TO BE RELEVANT | |
|---|---|---|
| Category* | Citation of document, with indication, where appropriate, of the relevant passages | Relevant to claim No. |
| X | JP 2016-520097 A (THE CHEMOURS COMPANY FC, LLC) 11 July 2016 (2016-07-11), claims, examples | 1-6, 8 |
| X | CN 103506130 A (YELLOW RIVER DELTA JINGBO RESEARCH INSTITUTE OF CHEMICAL INDUSTRY CO., LTD.) 15 January 2014 (2014-01-15), claims, examples | 1-6, 8 |
| X | NAGAO, Y. et al., Rh/ZrP2O7 as an efficient automotive catalyst for NOx reduction under slightly lean conditions, ACS Catalysis, 2015, 5, 1986-1994., DOI:10.1021/cs5020157, experimental section, results and discussion (particularly, Possible reason for the higher deNOx activity in slightly lean regions) | 1-4, 6, 8 |

☒ Further documents are listed in the continuation of Box C.        ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 May 2021 | 01 June 2021 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2021/013989

C (Continuation). DOCUMENTS CONSIDERED TO BE RELEVANT

| Category* | Citation of document, with indication, where appropriate, of the relevant passages | Relevant to claim No. |
|---|---|---|
| X | WO 2017/102811 A1 (TOTAL RESEARCH & TECHNOLOGY FELUY) 22 June 2017 (2017-06-22), page 17, lines 17-20, examples | 1-9 |
| Y | | 10, 11 |
| Y | JP 2014-161776 A (ASAHI KASEI CHEMICALS CORPORATION) 08 September 2014 (2014-09-08), paragraphs [0002]-[0004] | 10, 11 |

Form PCT/ISA/210 (second sheet) (January 2015)

| INTERNATIONAL SEARCH REPORT | International application No. |
|---|---|
| Information on patent family members | PCT/JP2021/013989 |

| | | |
|---|---|---|
| JP 2016-520097 A | 11 July 2016 | US 2014/0350310 A1<br>claims, examples<br>WO 2014/190126 A2<br>CN 105431400 A |
| CN 103506130 A | 15 January 2014 | (Family: none) |
| WO 2017/102811 A1 | 22 June 2017 | US 2020/0002244 A1<br>EP 3390336 A1 |
| JP 2014-161776 A | 08 September 2014 | (Family: none) |

Form PCT/ISA/210 (patent family annex) (January 2015)

**EP 4 129 471 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020065205 A
- JP 2020150757 A
- JP 2011505490 W
- JP 2010104938 A
- JP 2016079143 A

**Non-patent literature cited in the description**

- *Journal of Catalysis,* 2000, vol. 195, 360-375
- *Journal of Molecular Catalysis A: Chemical,* 2000, vol. 152, 141-155
- *Journal of the Serbian Chemical Society,* 2008, vol. 73, 997-1009